# EUROPEAN PATENT APPLICATION

(11) **EP 4 682 142 A1**
(43) Date of publication of application: **21.01.2026**
(21) Application number: 24774037.6
(22) Date of filing: 15.03.2024
(51) Int. Cl.: C07D 401/12, C07D 207/416, C07D 405/12, C07D 409/12, C07D 403/12, C07D 413/12, C07D 417/12, C07D 417/14, C07D 471/04, C07D 487/04, C07D 498/14, C07D 413/14, C07D 498/04, C07D 491/107, C07D 498/08, A61K 31/4439, A61K 31/4025, A61K 31/4178, A61K 31/422, A61K 31/427, A61K 31/4155

(54) **1H-PYRROLE-2-AMIDE DERIVATIVE AND USE THEREOF**

(30) Priority: 17.03.2023 CN 202310264593
(71) Applicant: Chongqing Pharscin Innobio Co., Ltd., Chongqing 401121 (CN)
(72) Inventor: YANG, Shengyong, Chengdu, Sichuan 610065 (CN); LI, Linli, Chengdu, Sichuan 610065 (CN)
(74) Representative: Elzaburu S.L.P.
(86) International application number: PCT/CN2024/081963
(87) International publication number: WO 2024/193472

(57) **Abstract**

The present invention relates to the field of chemical medicines. Disclosed are a 1H-pyrrole-2-amide derivative and a use thereof. In order to obtain a specific inhibitor for an m6A-modified RNA reader protein YTHDC1 of AML, the present invention provides a 1H-pyrrole-2-amide derivative as shown in formula I, wherein said derivative has high inhibitory activity against YTHDC1. In-vitro experiments prove that said derivative can effectively inhibit the proliferation of acute myeloid leukemia cells, significantly arrest a cell cycle of the acute myeloid leukemia cells in the G0/G1 phase, and induce differentiation and apoptosis of the acute myeloid leukemia cells. A compound and a salt thereof or a pharmaceutical composition of the 1H-pyrrole-2-amide derivative of the present invention provide new options for antitumor drug development targeting YTHDC1 in the art, and have good application prospects.

## Description

### Technical Field

The present invention relates to a 1H-pyrrole-2-amide derivative and a use thereof, and belongs to the field of chemical medicine.

### Background

N6-methyladenosine (m⁶A) modification is the most common chemical modification in eukaryotic mRNA. The m⁶A methylation modification mainly regulates the stability, transcription, processing, and translation of mRNA, and the splicing of precursor RNA. The m⁶A modification of RNA is a dynamic and reversible process, where the 'writer' methyltransferase METTL3-14 is responsible for transferring methyl groups to RNA, the 'eraser' demethylases FTO and ALKBH5 are responsible for removing methyl modifications from RNA, and the 'reader' YTH domain family (YTHDF1-3, YTHDC1-2) specifically recognizes the m⁶A methylation modification. A growing body of research indicates that the m⁶A modification of RNA has a significant impact on the production and metabolism of RNA and is closely related to the pathogenesis of various diseases, such as neoplasms, neurological diseases, viral infections, and immune system-related diseases. To date, with the exception of METTL3 and FTO, the biological functions and mechanisms of action of the other key proteins involved in dynamic m⁶A modification have not yet been elucidated, and there is a particular lack of reports on highly active and selective small-molecule inhibitors.

YTHDC1 (also known as YT521-B) was initially identified as an RNA splicing-related protein because it contains a glutamic acid/arginine-rich carboxy-terminal domain characteristic of splicing factors. The YTH domain in YTHDC1 specifically recognizes m⁶A modifications and preferentially recognizes the G(m⁶A)C sequence through a hydrophobic aromatic cage pocket formed by W377, W428, L430, and L439 residues thereof. YTHDC1 binds to m⁶A-modified pre-RNA to recruit the splicing factor serine and arginine rich splicing factor 3 (SRSF3) and block the binding of serine and arginine rich splicing factor 10 (SRSF10) to nuclear speckles, thereby promoting exon inclusion and the export of mRNA from the nucleus to the cytoplasm. YTHDC1 is the only 'reader' in the YTH domain family that can directly bind to m⁶A-modified RNA in the nucleus, and plays an important role in the process of dynamic m⁶A modification. The latest research results show that YTHDC1 is closely related to the occurrence and development of various diseases, such as acute myeloid leukemia (AML), hepatic cancer, pancreatic carcinoma, breast cancer, glioma, and hepatitis B. YTHDC1 is considered a potential and important therapeutic target for these diseases and has begun to attract significant attention from major pharmaceutical companies and drug research and development institutions in recent years. However, to date, there have been no reports, either domestically or internationally, of specific small-molecule inhibitors targeting YTHDC1.

Therefore, developing highly active and specific small-molecule inhibitors of YTHDC1 will not only provide new means for the treatment of various related diseases such as neoplasms and hepatitis B, but will also facilitate the exploration of the regulatory mechanisms and roles of YTHDC1 in physiological and pathological processes.

### Summary

The present invention provides a 1*H*-pyrrole-2-amide derivative that has high inhibitory activity against YTHDC1, offering a new means for the treatment of various YTHDC1-related diseases such as tumors and hepatitis B.

The present invention provides a compound represented by Formula I, or a hydrate thereof, or a pharmaceutically acceptable salt thereof, the structure of which is shown below: 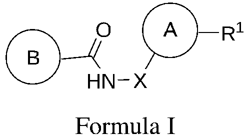
wherein, ring A is selected from a 6- to 10-membered aromatic ring and a 5- to 10-membered heteroaromatic ring;
in ring A, the 5- to 10-membered heteroaromatic ring contains 1 to 3 heteroatoms, wherein the heteroatoms are selected from N, S, and O;
R¹ is selected from H, halogen, cyano, halogen-substituted or unsubstituted C1-C8 alkyl, halogen-substituted or unsubstituted C1-C8 alkoxy, halogen-substituted or unsubstituted C2-C8 alkanoyl, halogen-substituted or unsubstituted C2-C8 alkoxycarbonyl, halogen-substituted or unsubstituted 3- to 8-membered cycloalkyl, substituted or unsubstituted 6- to 10-membered aryl, and substituted or unsubstituted 5- to 10-membered heteroaryl;
in R¹, the 5- to 10-membered heteroaryl contains 1 to 3 heteroatoms, wherein the heteroatoms are selected from N, S, and O;
in R¹, the substituents of the substituted or unsubstituted 6- to 10-membered aryl and the substituted or unsubstituted 5- to 10-membered heteroaryl are selected from at least one of cyano, halogen-substituted or unsubstituted C1-C6 alkyl, and -NR¹³R¹⁴;
R¹³ and R¹⁴ are independently selected from H and halogen-substituted or unsubstituted C1-C4 alkyl, or R¹³ and R¹⁴, together with the N atom in -NR¹³R¹⁴, form a halogen-substituted or unsubstituted 3- to 8-membered heterocycloalkyl, wherein the 3- to 8-membered heterocycloalkyl, in addition to the aforementioned N atom, further contains 0 to 2 heteroatoms selected from N, S, and O;
ring B is selected from 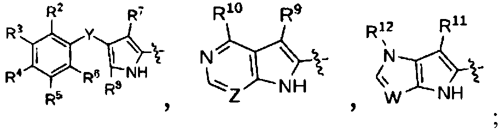
R² to R⁶ are independently selected from H, halogen, halogen-substituted or unsubstituted C1-C8 alkyl, and halogen-substituted or unsubstituted C1-C8 alkoxy;
R⁷ to R⁹ and R¹¹ are independently selected from H, halogen, and halogen-substituted or unsubstituted C1-C6 alkyl;
R¹⁰ and R¹² are independently selected from substituted or unsubstituted 6- to 10-membered aryl and substituted or unsubstituted 5- to 10-membered heteroaryl;
in R¹⁰ and R¹², the 5- to 10-membered heteroaryl contains 1 to 3 heteroatoms, wherein the heteroatoms are selected from N, S, and O;
in R¹⁰ and R¹², the substituents of the substituted 6- to 10-membered aryl and the substituted 5- to 10-membered heteroaryl are selected from cyano, halogen, substituted or unsubstituted C1-C6 alkyl, substituted or unsubstituted C1-C6 alkoxy, -NR¹⁵R¹⁶, and -SO₂R¹⁷;
in R¹⁰ and R¹², the substituents of the substituted C1-C6 alkyl and the substituted C1-C6 alkoxy are selected from hydroxyl and halogen;
R¹⁵, R¹⁶, and R¹⁷ are independently selected from H and halogen-substituted or unsubstituted C1-C4 alkyl;
X is selected from a bond and -CH(R¹⁸)(CH₂)ₙ-; wherein n is an integer from 0 to 3;
R¹⁸ is selected from H and C1-C4 alkyl;
Y is selected from 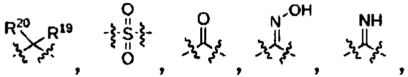 -NH- (the left end is connected to the benzene ring, and the right end is connected to the pyrrole ring);
R¹⁹ and R²⁰ are independently selected from H, hydroxyl, cyano, halogen, and halogen-substituted or unsubstituted C1-C4 alkyl;
Z and W are independently selected from N and CR²¹;
R²¹ is selected from H and halogen.

In some embodiments of the present invention, ring A is selected from a 6- to 10-membered aromatic ring and a 5- to 6-membered heteroaromatic ring; in ring A, the 5- to 6-membered heteroaromatic ring contains 1 to 2 heteroatoms selected from N, S, and O.

In some preferred embodiments of the present invention, ring A is selected from a benzene ring and a pyridine ring.

In some most preferred embodiments of the present invention, ring A is selected from

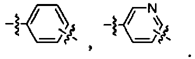

In some embodiments of the present invention, R¹ is selected from H, halogen, cyano, halogen-substituted or unsubstituted C1-C6 alkyl, halogen-substituted or unsubstituted C1-C6 alkoxy, halogen-substituted or unsubstituted C2-C6 alkanoyl, halogen-substituted or unsubstituted C2-C6 alkoxycarbonyl, halogen-substituted or unsubstituted 3- to 6-membered cycloalkyl, substituted or unsubstituted phenyl, and substituted or unsubstituted 5- to 6-membered heteroaryl; in R¹, the 5- to 6-membered heteroaryl contains 1 to 2 heteroatoms selected from N, S, and O.

In some preferred embodiments of the present invention, R¹ is selected from H, halogen, cyano, halogen-substituted or unsubstituted C1-C4 alkyl, halogen-substituted or unsubstituted C1-C4 alkoxy, halogen-substituted or unsubstituted C2-C4 alkanoyl, halogen-substituted or unsubstituted C2-C5 alkoxycarbonyl, halogen-substituted or unsubstituted 3- to 6-membered cycloalkyl, substituted or unsubstituted phenyl, and substituted or unsubstituted 5- to 6-membered heteroaryl; in R¹, the 5- to 6-membered heteroaryl contains 1 to 2 heteroatoms selected from N, S, and O.

In some further preferred embodiments of the present invention, R¹ is selected from H, F, Cl, Br, cyano, fluorine-substituted or unsubstituted C1-C4 alkyl, fluorine-substituted or unsubstituted C1-C4 alkoxy, fluorine-substituted or unsubstituted C2-C4 alkanoyl, fluorine-substituted or unsubstituted C2-C5 alkoxycarbonyl, fluorine-substituted or unsubstituted 3- to 6-membered cycloalkyl, substituted or unsubstituted phenyl, and substituted or unsubstituted 5- to 6-membered heteroaryl; in R¹, the 5- to 6-membered heteroaryl contains 1 to 2 heteroatoms selected from N, S, and O.

In some still further preferred embodiments of the present invention, R¹ is selected from H, F, Cl, Br, cyano, fluorine-substituted or unsubstituted C1-C4 alkyl, fluorine-substituted or unsubstituted C1-C4 alkoxy, fluorine-substituted or unsubstituted C2-C4 alkanoyl, fluorine-substituted or unsubstituted C2-C5 alkoxycarbonyl, fluorine-substituted or unsubstituted 3- to 6-membered cycloalkyl, substituted or unsubstituted phenyl, and substituted or unsubstituted 5- to 6-membered heteroaryl; in R¹, the 5- to 6-membered heteroaryl is selected from pyridyl, thienyl, furyl, pyrrolyl, thiazolyl, isothiazolyl, oxazolyl, isoxazolyl, imidazolyl, and pyrazolyl.

In some most preferred embodiments of the present invention, R¹ is selected from H, F, Cl, Br, methyl, trifluoromethyl, methoxy, trifluoromethoxy, acetyl, ethoxycarbonyl, substituted or unsubstituted phenyl, and substituted or unsubstituted 5- to 6-membered heteroaryl; in R¹, the 5- to 6-membered heteroaryl is selected from

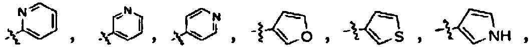

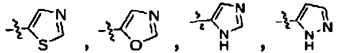

In some embodiments of the present invention, in R¹, the substituents of the substituted or unsubstituted aryl and the substituted or unsubstituted heteroaryl are selected from at least one of cyano, halogen-substituted or unsubstituted C1-C4 alkyl, and -NR¹³R¹⁴; R¹³ and R¹⁴ are independently selected from halogen-substituted or unsubstituted C1-C4 alkyl, or R¹³ and R¹⁴, together with the N atom in -NR¹³R¹⁴, form a halogen-substituted or unsubstituted 3- to 6-membered heterocycloalkyl, wherein the 3- to 6-membered heterocycloalkyl, in addition to the aforementioned N atom, further contains 0 to 1 heteroatom selected from N, S, and O.

In some preferred embodiments of the present invention, in R¹, the substituents of the substituted or unsubstituted aryl and the substituted or unsubstituted heteroaryl are selected from at least one of cyano, fluorine-substituted or unsubstituted C1-C4 alkyl, and -NR¹³R¹⁴; R¹³ and R¹⁴ are independently selected from fluorine-substituted or unsubstituted C1-C4 alkyl, or R¹³ and R¹⁴, together with the N atom in -NR¹³R¹⁴, form a fluorine-substituted or unsubstituted 3- to 6-membered heterocycloalkyl, wherein the 3- to 6-membered heterocycloalkyl, in addition to the aforementioned N atom, further contains 0 to 1 heteroatom selected from S and O.

In some further preferred embodiments of the present invention, in R¹, the substituents of the substituted or unsubstituted aryl and the substituted or unsubstituted heteroaryl are selected from at least one of cyano, methyl, trifluoromethyl, and -NR¹³R¹⁴; R¹³ and R¹⁴ are independently selected from methyl and trifluoromethyl, or R¹³ and R¹⁴, together with the N atom in -NR¹³R¹⁴, form a fluorine-substituted or unsubstituted 3- to 6-membered heterocycloalkyl, wherein the 3- to 6-membered heterocycloalkyl is selected from

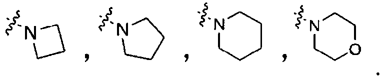

In some most preferred embodiments of the present invention, in R¹, the substituents of the substituted or unsubstituted aryl and the substituted or unsubstituted heteroaryl are selected from cyano, methyl, trifluoromethyl, and

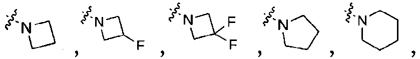

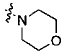

In some embodiments of the present invention, the structural unit 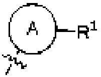 is selected from:

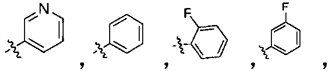

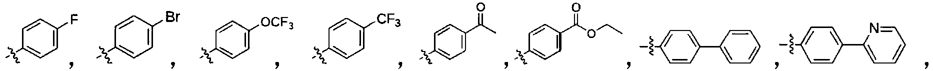

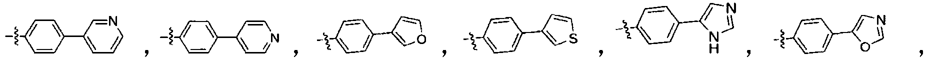

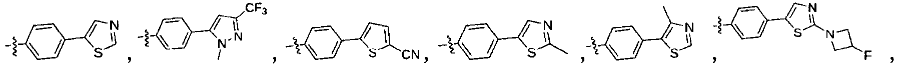

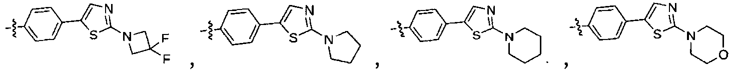

In some embodiments of the present invention, R² to R⁶ are independently selected from H, halogen, halogen-substituted or unsubstituted C1-C6 alkyl, and halogen-substituted or unsubstituted C1-C6 alkoxy.

In some preferred embodiments of the present invention, R² to R⁶ are independently selected from H, halogen, halogen-substituted or unsubstituted C1-C4 alkyl, and halogen-substituted or unsubstituted C1-C4 alkoxy.

In some further preferred embodiments of the present invention, R² to R⁶ are independently selected from H, F, Cl, Br, fluorine-substituted or unsubstituted C1-C4 alkyl, and fluorine-substituted or unsubstituted C1-C4 alkoxy.

In some still further preferred embodiments of the present invention, R² to R⁶ are independently selected from H, F, Cl, Br, fluorine-substituted or unsubstituted C1-C4 alkyl, and fluorine-substituted or unsubstituted C1-C4 alkoxy, and R² and R⁶ are not both H at the same time.

In some most preferred embodiments of the present invention, R² is selected from H, F, Cl, Br, methyl, trifluoromethyl, ethyl, tert-butyl, methoxy, trifluoromethoxy, and ethoxy, R³ is selected from H, F, Cl, and Br, R⁴ is selected from H, F, Cl, Br, methyl, and trifluoromethyl, R^{S} is selected from H, F, Cl, and Br, R⁶ is selected from H, F, Cl, Br, methyl, trifluoromethyl, ethyl, tert-butyl, methoxy, trifluoromethoxy, and ethoxy, and R² and R⁶ are not both H at the same time.

In some embodiments of the present invention, the structural unit 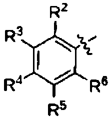 is selected from:

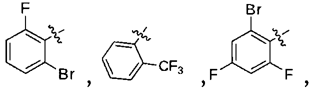

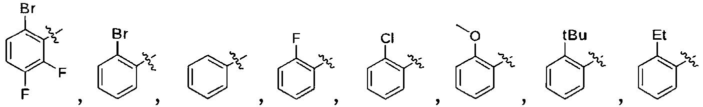

In some embodiments of the present invention, Y is selected from 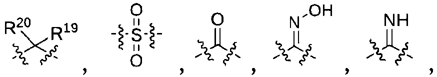 -NH- ; R¹⁹ and R²⁰ are independently selected from H, hydroxyl, cyano, F, and F-substituted or unsubstituted C1-C4 alkyl.

In some preferred embodiments of the present invention, Y is selected from 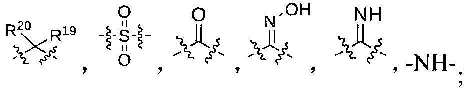 R¹⁹ and R²⁰ are independently selected from H, hydroxyl, cyano, F, methyl, and trifluoromethyl.

In some most preferred embodiments of the present invention, Y is selected from

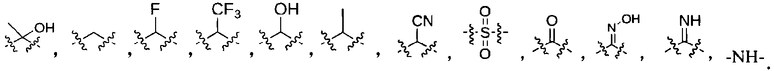

In some embodiments of the present invention, R⁷ to R⁹ and R¹¹ are independently selected from H, halogen, and halogen-substituted or unsubstituted C1-C4 alkyl.

In some preferred embodiments of the present invention, R⁷ to R⁹ and R¹¹ are independently selected from H, F, and F-substituted or unsubstituted C1-C4 alkyl.

In some most preferred embodiments of the present invention, R⁷ to R⁹ and R¹¹ are independently selected from H, F, methyl, and trifluoromethyl.

In some embodiments of the present invention, R¹⁰ and R¹² are independently selected from substituted or unsubstituted phenyl and substituted or unsubstituted 5- to 6-membered heteroaryl; in R¹⁰ and R¹², the 5- to 6-membered heteroaryl contains 1 to 2 heteroatoms selected from N, S, and O.

In some preferred embodiments of the present invention, R¹⁰ and R¹² are independently selected from substituted or unsubstituted phenyl and substituted or unsubstituted 5- to 6-membered heteroaryl; in R¹⁰ and R¹², the 5- to 6-membered heteroaryl is selected from pyridyl, thienyl, furyl, pyrrolyl, thiazolyl, isothiazolyl, oxazolyl, isoxazolyl, imidazolyl, and pyrazolyl.

In some most preferred embodiments of the present invention, R¹⁰ and R¹² are independently selected from substituted or unsubstituted phenyl and substituted or unsubstituted 5- to 6-membered heteroaryl; in R¹⁰ and R¹², the 5- to 6-membered heteroaryl is selected from

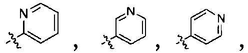

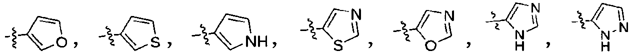

In some embodiments of the present invention, in R¹⁰ and R¹², the substituents of the substituted aryl and the substituted heteroaryl are selected from cyano, halogen, substituted or unsubstituted C1-C4 alkyl, substituted or unsubstituted C1-C4 alkoxy, -NR¹⁵R¹⁶, and -SO₂R¹⁷· in R¹⁰ and R¹², the substituents of the substituted C1-C4 alkyl and the substituted C1-C4 alkoxy are selected from hydroxyl and halogen; R¹⁵, R¹⁶, and R¹⁷ are independently selected from H and halogen-substituted or unsubstituted C1-C4 alkyl.

In some preferred embodiments of the present invention, in R¹⁰ and R¹², the substituents of the substituted aryl and the substituted heteroaryl are selected from cyano, F, Cl, Br, substituted or unsubstituted C1-C4 alkyl, substituted or unsubstituted C1-C4 alkoxy, -NR¹⁵R¹⁶, and -SO₂R¹⁷; in R¹⁰ and R¹², the substituents of the substituted C1-C4 alkyl and the substituted C1-C4 alkoxy are selected from hydroxyl and F; R¹⁵, R¹⁶, and R¹⁷ are independently selected from H and fluorine-substituted or unsubstituted C1-C4 alkyl.

In some most preferred embodiments of the present invention, in R¹⁰ and R¹², the substituents of the substituted aryl and the substituted heteroaryl are selected from cyano, F, Cl, Br, methyl, ethyl, n-propyl, isopropyl, hydroxymethyl, trifluoromethyl, methoxy, trifluoromethoxy, amino, -SO₂CF₃, and -SO₂CH₃.

In some embodiments of the present invention, X is selected from a bond and-CH(R¹⁸)(CH₂)ₙ-; n is an integer selected from 0 to 1; R¹⁸ is selected from H and C1-C4 alkyl.

In some preferred embodiments of the present invention, X is selected from a bond,-CH(CH₃)-, and -CH₂-.

In some embodiments of the present invention, Z and W are independently selected from N and CR²¹; R²¹ is selected from H and F.

In some preferred embodiments of the present invention, Z is selected from N, CH, and CF, and W is selected from N.

In some embodiments of the present invention, 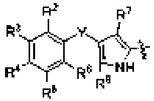 is selected from:

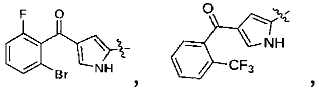

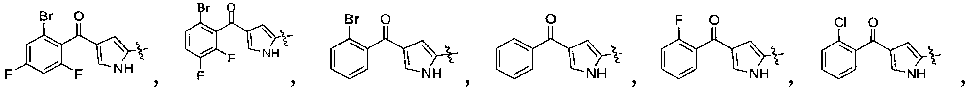

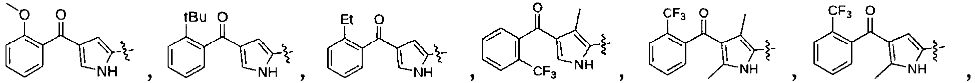

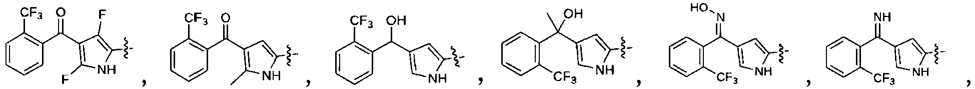

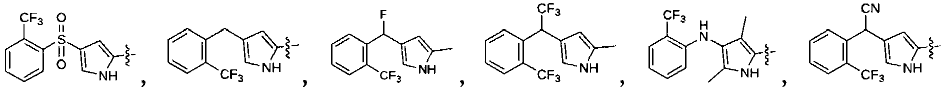

In some embodiments of the present invention, 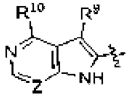 is selected from:

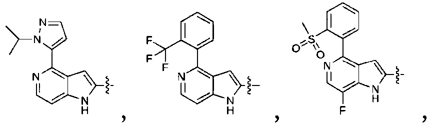

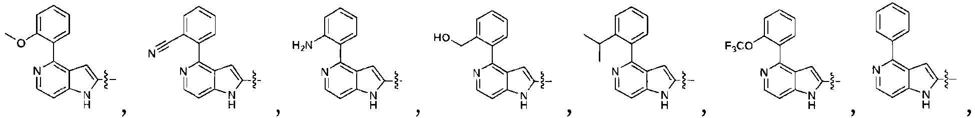

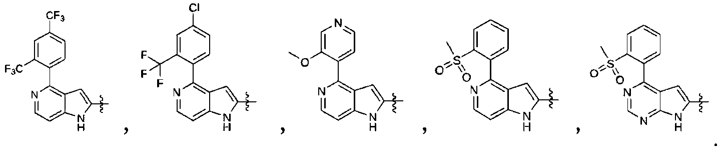

In some embodiments of the present invention, 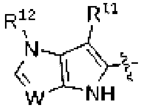 is selected from:

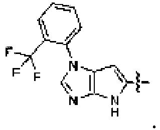

The present invention also provides some specific compounds represented by Formula I, characterized in that the structural formulas of which are as follows:

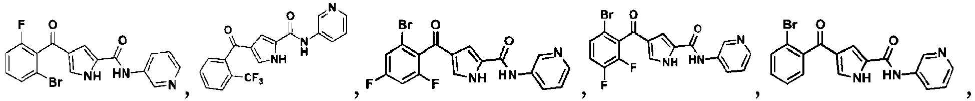

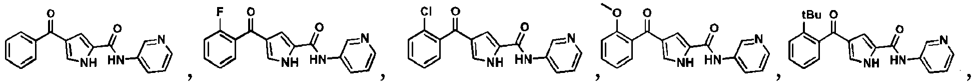

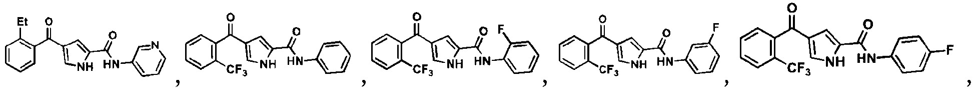

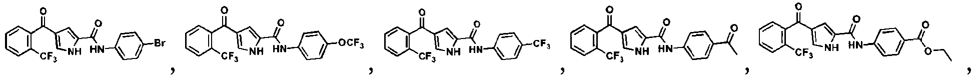

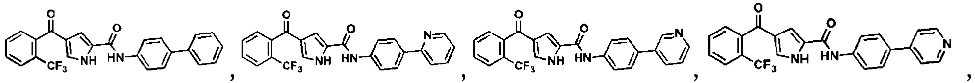

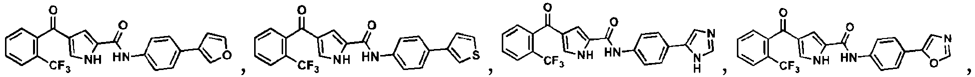

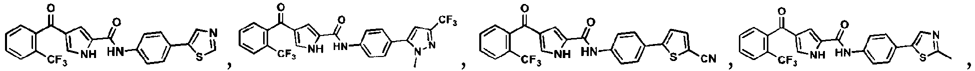

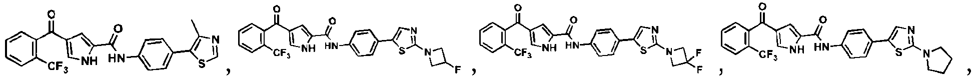

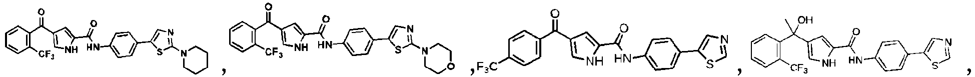

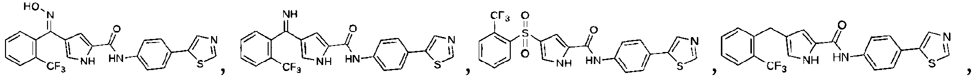

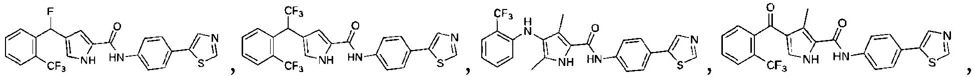

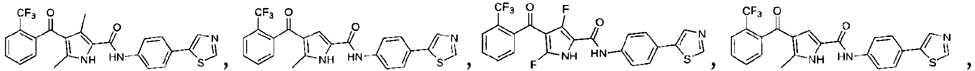

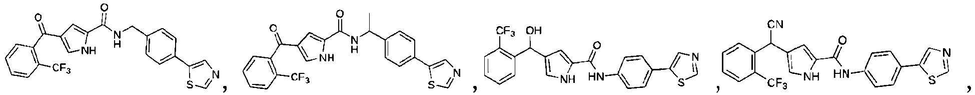

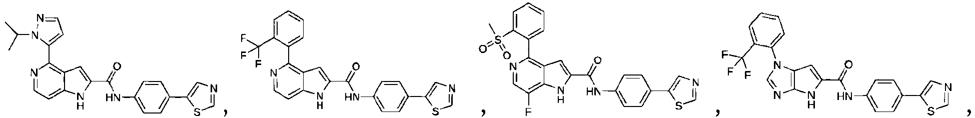

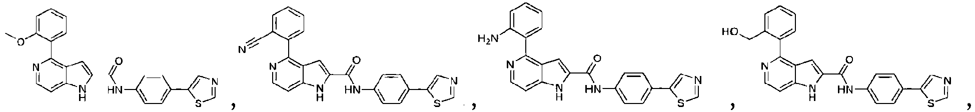

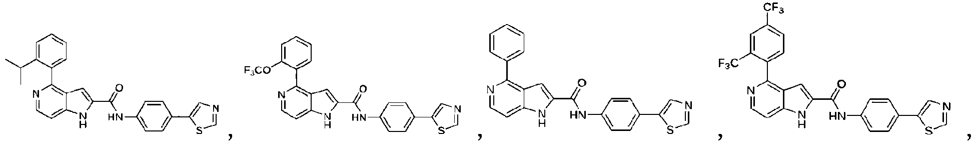

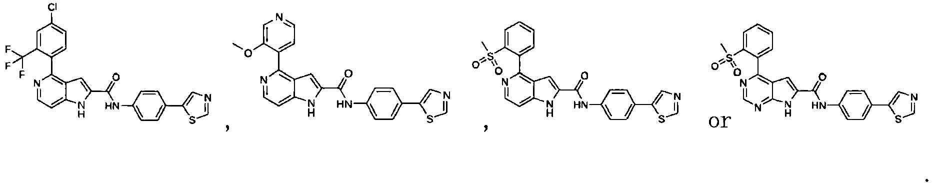

Based on the above Formula I compounds, the present invention also provides some compounds in which the structural unit 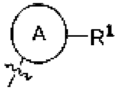 is selected from:

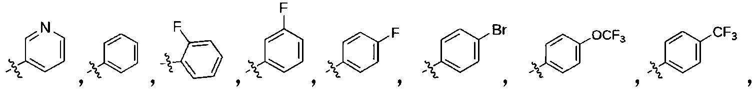

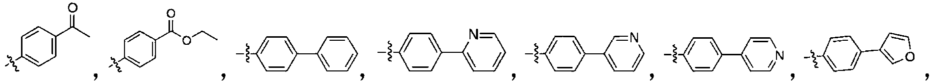

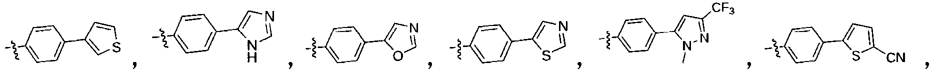

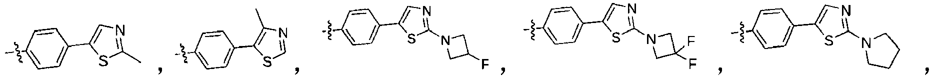

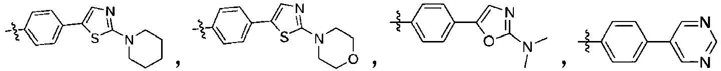

Based on the above Formula I compounds, the present invention also provides some compounds in which 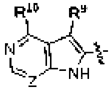 is selected from:

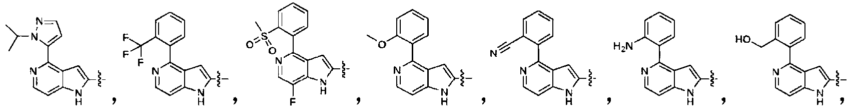

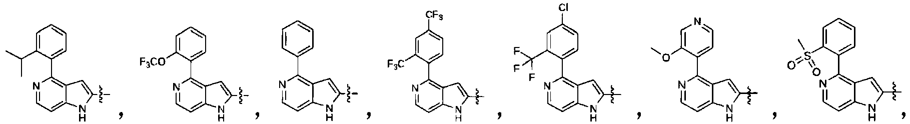

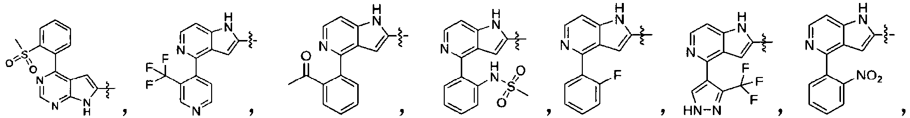

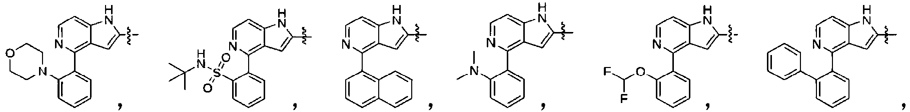

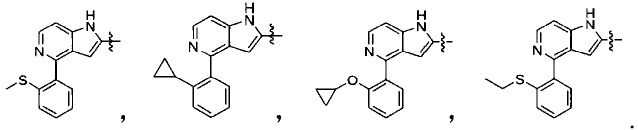

Based on the above Formula I compounds, the present invention also provides some specific compounds, the structural formulas of which are as follows: 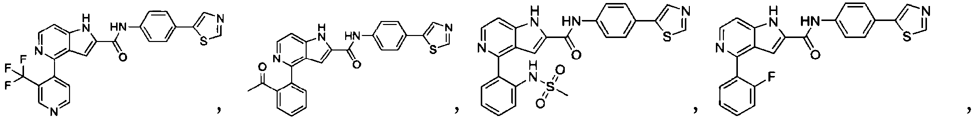 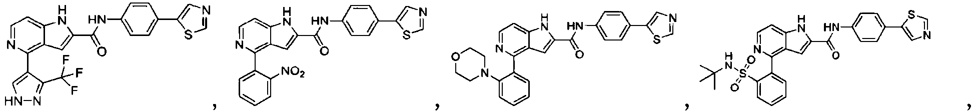 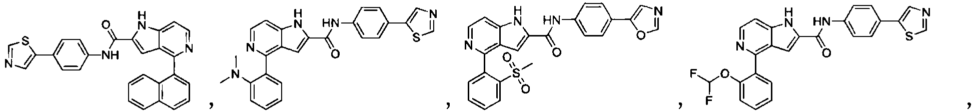 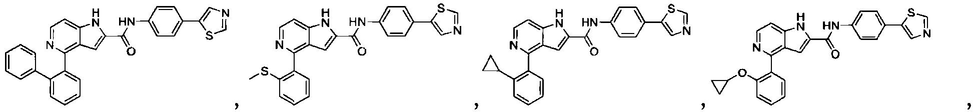 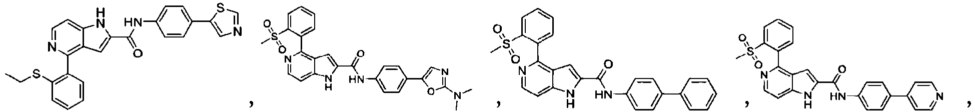 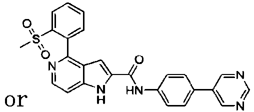 .

Based on the above Formula I compounds, the present invention also provides some specific compounds, the structural formulas of which are as follows:

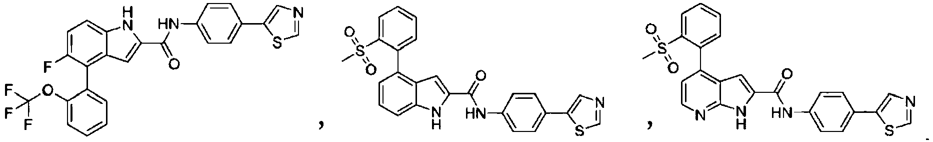

Based on the above Formula I compounds, the present invention further provides some compounds in which the structural unit 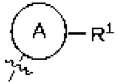 is selected from: 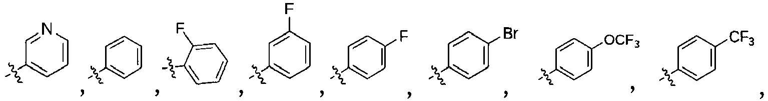 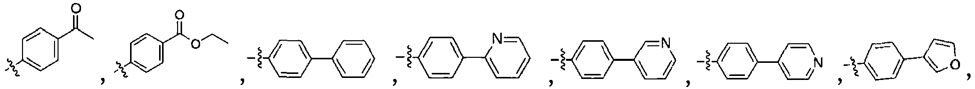 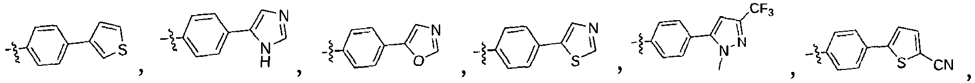 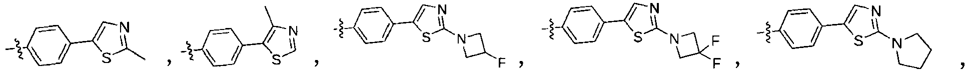 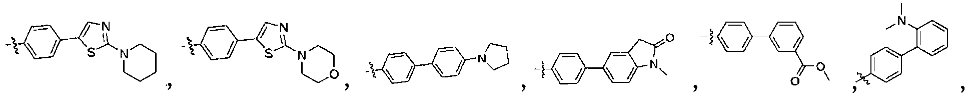 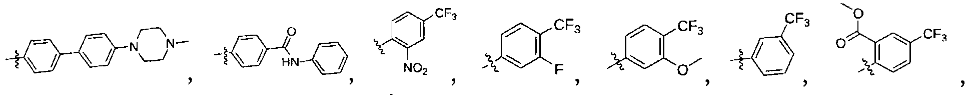 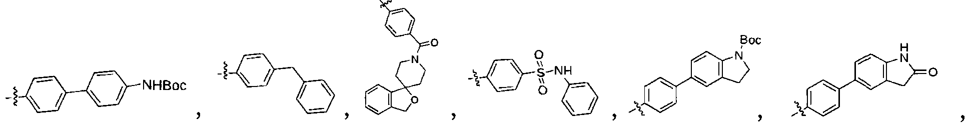 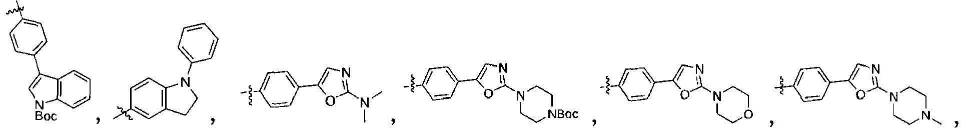 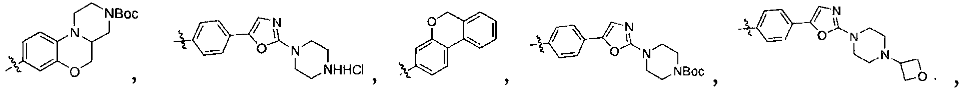 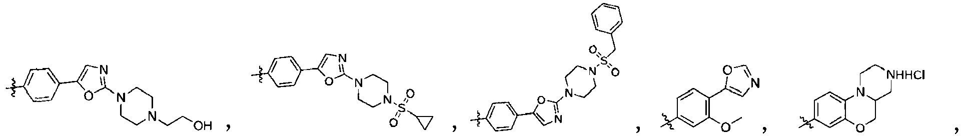 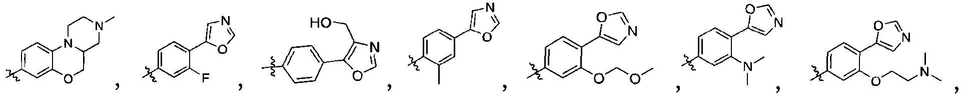 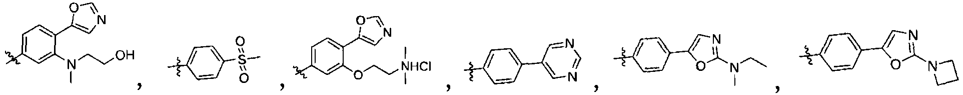 .

Based on the above Formula I compounds, the present invention further provides some compounds in which the structural unit 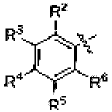 is selected from:

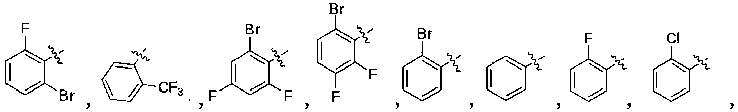

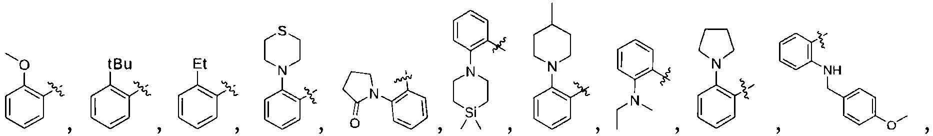

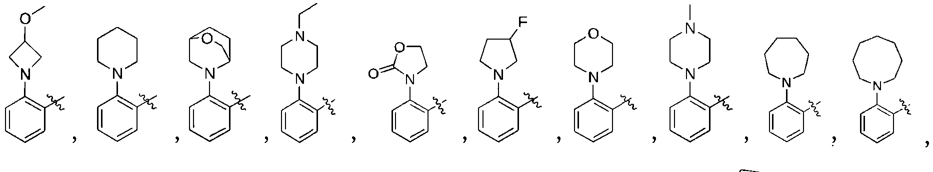

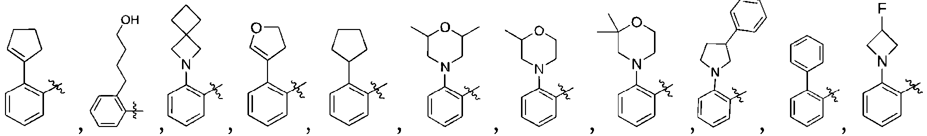

. Based on the above Formula I compounds, the present invention further provides some compounds in which 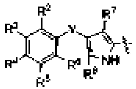 is selected from:

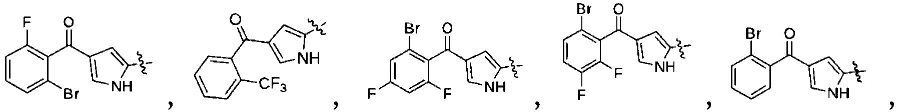

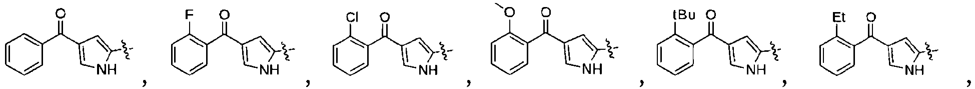

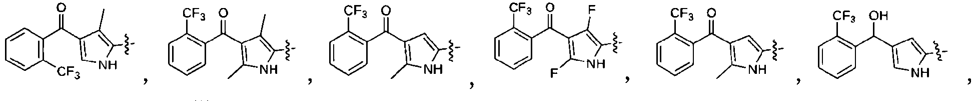

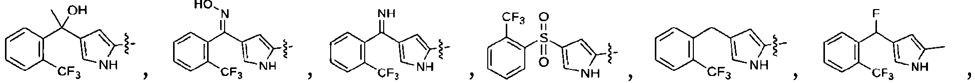

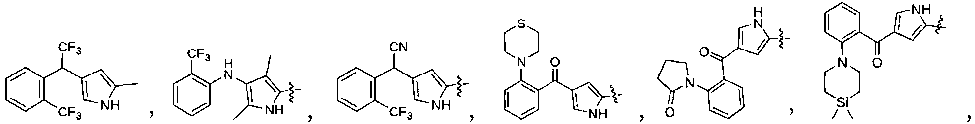

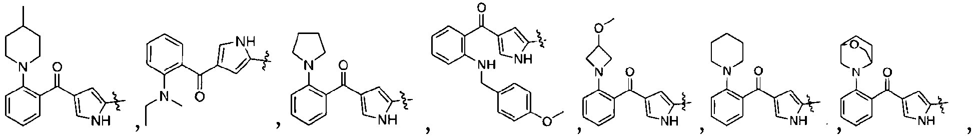

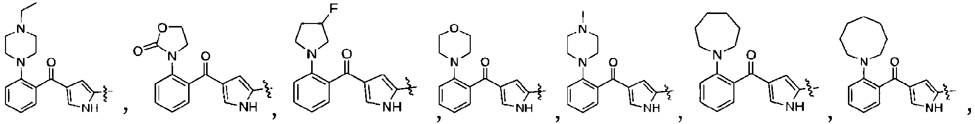

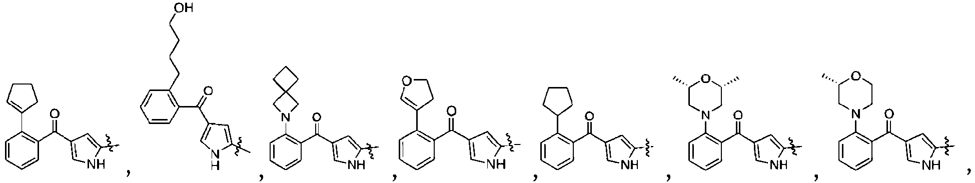

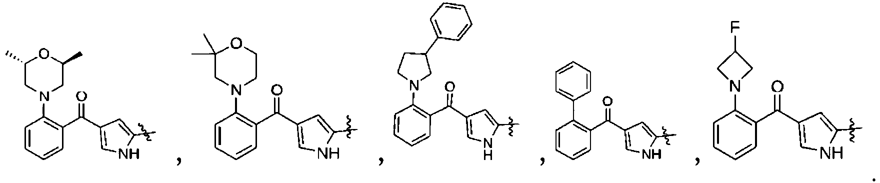

Based on the above Formula I compounds, the present invention further provides some specific compounds, the structural formulas of which are as follows: 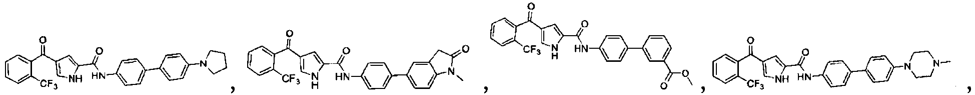 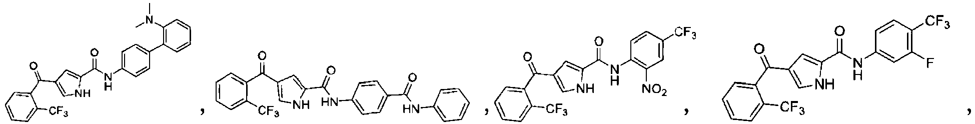 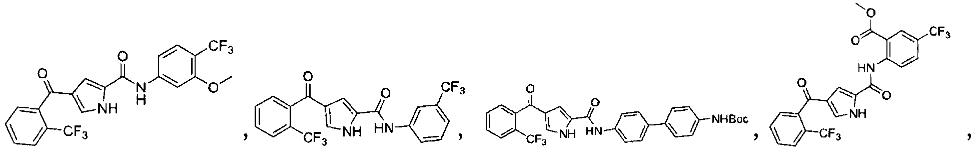 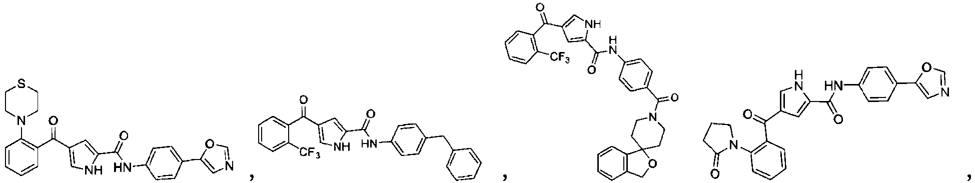 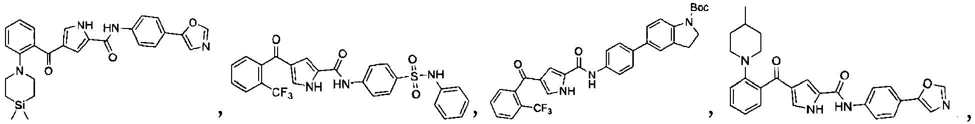 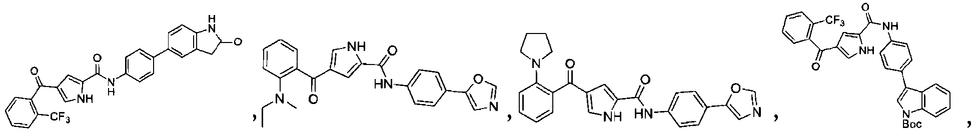 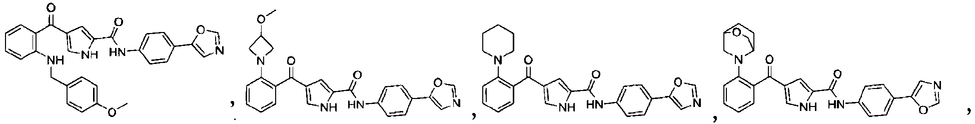 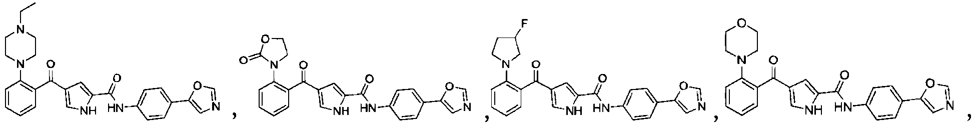 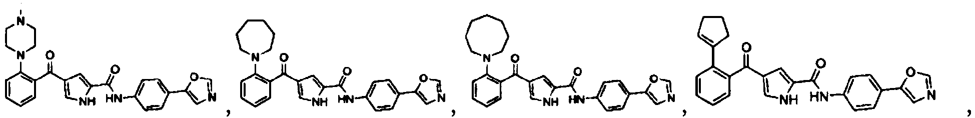 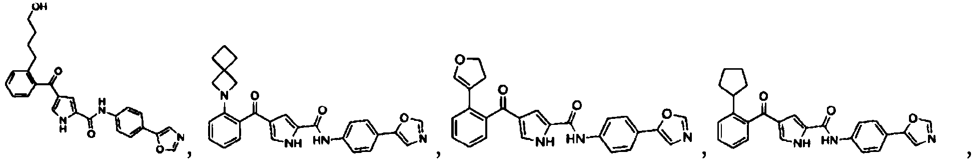 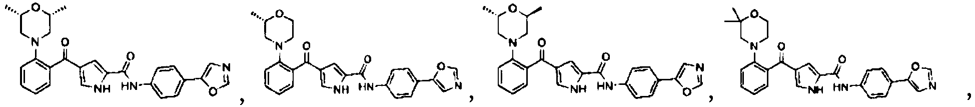 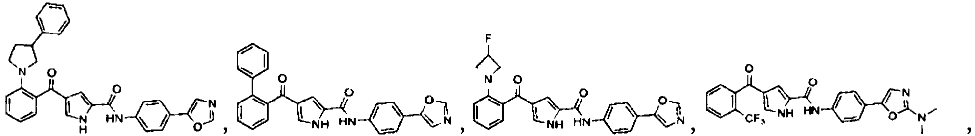 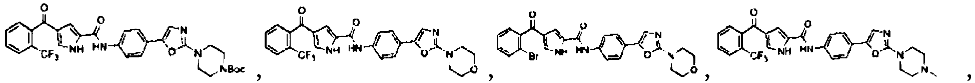 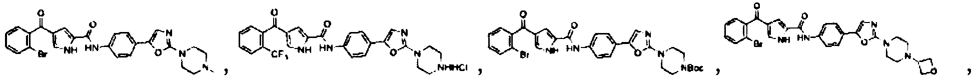 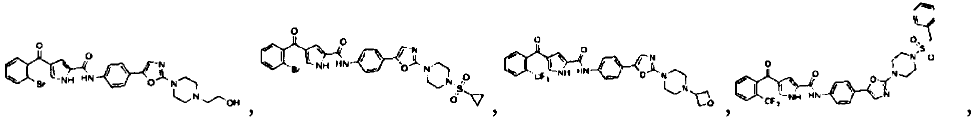 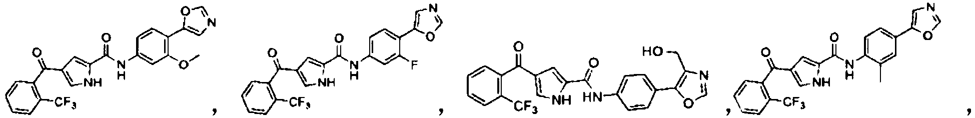 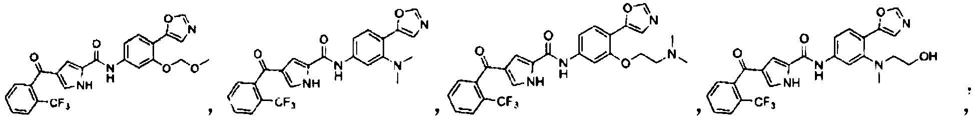 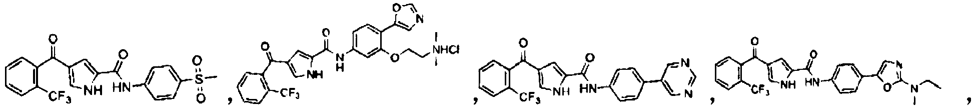 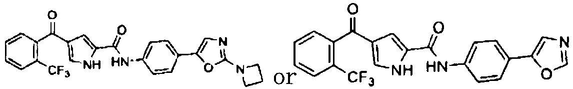 .

Based on the above Formula I compounds, the present invention further provides some specific compounds, the structural formulas of which are as follows: 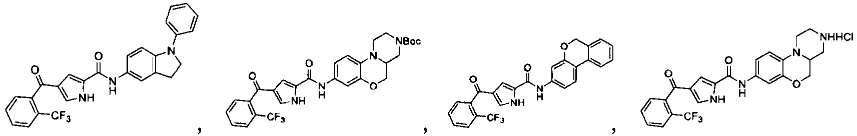 or 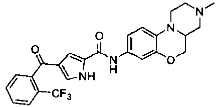 .

The present invention also provides a pharmaceutical composition, which is composed of the above-mentioned compound, hydrate thereof, or a pharmaceutically acceptable salt thereof as an active ingredient, with the addition of pharmaceutically acceptable auxiliary ingredients.

The present invention also provides the use of the above-mentioned compound, hydrate thereof, a pharmaceutically acceptable salt thereof, or the above-mentioned pharmaceutical composition in the preparation of a small-molecule inhibitor of YTHDC1.

The present invention also provides the use of the above-mentioned compound, hydrate thereof, a pharmaceutically acceptable salt thereof, or the above-mentioned pharmaceutical composition in the preparation of a medicament for treating and/or preventing YTHDC1-related diseases.

In some preferred embodiments of the present invention, the YTHDC1-related diseases include acute myeloid leukemia, t-cell lymphoma, hepatic cancer, pancreatic carcinoma, breast cancer, glioma, or hepatitis B.

Some more preferred embodiments include acute myeloid leukemia and t-cell lymphoma.

Definition of terms: The compounds and derivatives provided by the present invention can be named according to the International Union of Pure and Applied Chemistry (IUPAC) or Chemical Abstracts Service (CAS, Columbus, OH) nomenclature systems.

The term 'alkyl' refers to a straight-chain or branched-chain saturated hydrocarbon group. Examples of C1-C8 alkyl include, but are not limited to, methyl (C1), ethyl (C2), n-propyl (C3), isopropyl (C3), n-butyl (C4), tert-butyl (C4), sec-butyl (C4), isobutyl (C4), n-pentyl (C5), 3-pentyl (C5), pentyl (C5), neopentyl (C5), 3-methyl-2-butyl (C5), tert-amyl (C5), and n-hexyl (C6).

The term 'C2-C8 alkanoyl' refers to a group in which a straight-chain or branched-chain saturated hydrocarbon group is connected to an acyl group. Examples of C2-C8 alkanoyl include, but are not limited to, acetyl (C2), propionyl (C3), n-propanoyl (C4), isopropanoyl (C4), n-butanoyl (C5), and tert-butanoyl (C5). The term 'C2-C8 alkoxycarbonyl' refers to a group in which a straight-chain or branched-chain saturated hydrocarbon group is connected to an acyl group through an oxygen atom. Examples of C2-C8 alkoxycarbonyl include, but are not limited to, methoxycarbonyl (C2), ethoxycarbonyl (C3), n-propoxycarbonyl (C4), isopropoxycarbonyl (C4), n-butoxycarbonyl (C5), and tert-butoxycarbonyl (C5).

The term 'cycloalkyl' refers to a saturated cyclic hydrocarbon group that does not contain heteroatoms, which can be a monocyclic structure or a polycyclic structure (such as a bridged or spiro structure), for example: cyclopropyl (3-membered), cyclohexyl (6-membered).

The term 'aryl' refers to an all-carbon monocyclic or fused-ring group with a conjugated π-electron system, such as phenyl, naphthyl, anthracenyl, phenanthrenyl, and pyrenyl. The term 'heteroaryl' refers to an aryl group in which carbon atoms are replaced by heteroatoms, where the heteroatoms are selected from sulfur, oxygen, and/or nitrogen, for example, thiophene, furan, pyrrole, pyridine, quinoline, indole, etc.

The term 'halogen' refers to fluorine (F), chlorine (Cl), bromine (Br), and iodine (I).

The term 'pharmaceutically acceptable' means that a carrier, vehicle, diluent, excipient, and/or the salt formed is generally chemically or physically compatible with the other ingredients forming a pharmaceutical dosage form and is physiologically compatible with the recipient.

The term 'pharmaceutically acceptable salt' refers to organic and inorganic salts of the compounds of the present invention, preferably inorganic salts and salts formed with pharmaceutically acceptable non-toxic acids, including but not limited to, inorganic acid salts formed by reaction with an amino group, such as hydrochloride, hydrobromide, phosphate, sulfate, perchlorate, and nitrate; and organic acid salts such as acetate, oxalate, maleate, tartrate, citrate, succinate, malonate, hydrochloride, oleate, stearate, ascorbate, formate, borate, camphorsulfonate, methanesulfonate, ethanesulfonate, and malate.

The pharmaceutically acceptable auxiliary ingredients described in the present invention refer to substances contained in the dosage form other than the active ingredient, said auxiliary ingredients being, for example, cyclodextrin, arginine, or meglumine. The cyclodextrin is selected from α-cyclodextrin, β-cyclodextrin, γ-cyclodextrin, (C₁₋₄ alkyl)-α-cyclodextrin, (C₁₋₄ alkyl)-β-cyclodextrin, (C₁₋₄ alkyl)-γ-cyclodextrin, (hydroxy-C₁₋₄ alkyl)-α-cyclodextrin, (hydroxy- C₁₋₄ alkyl)-β-cyclodextrin, (hydroxy- C₁₋₄ alkyl)-γ-cyclodextrin, (carboxy- C₁₋₄ alkyl)-α-cyclodextrin, (carboxy- C₁₋₄ alkyl)-β-cyclodextrin, (carboxy- C₁₋₄ alkyl)-γ-cyclodextrin, saccharide ethers of α-cyclodextrin, saccharide ethers of β-cyclodextrin, saccharide ethers of γ-cyclodextrin, sulfobutyl ethers of α-cyclodextrin, sulfobutyl ethers of β-cyclodextrin, and sulfobutyl ethers of γ-cyclodextrin. The auxiliary ingredients also include medically acceptable carriers, adjuvants, or vehicles. Also usable in pharmaceutically acceptable pharmaceutical compositions are ion exchangers, alumina, aluminum stearate, lecithin; buffering substances include phosphate, glycine, arginine, sorbic acid, etc.

Beneficial Effects: The present invention discovers a 1H-pyrrole-2-amide derivative that has high inhibitory activity against YTHDC1, providing a new means for the treatment of various YTHDC1-related diseases such as neoplasms and hepatitis B. *In vitro* experiments have demonstrated that this class of compounds can effectively inhibit the proliferation of AML cells *in vitro,* significantly arrest the cell cycle of AML cells in the G0/G1 phase, and induce the differentiation and apoptosis of AML cells.

### Brief Description of the Drawings

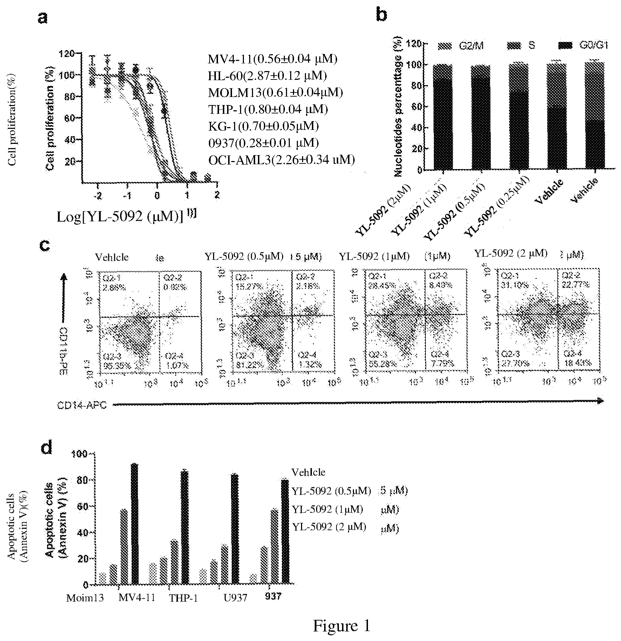

Figure 1 shows the results of *in vitro* cell experiments for Compound 29; wherein, a is a graph showing the inhibition of AML cell proliferation by Compound 29, b is a graph showing the induction of AML cell cycle arrest by Compound 29, c is a graph showing the induction of AML cell differentiation by Compound 29, and d is a graph showing the induction of AML cell apoptosis by Compound 29.

### Detailed Description of Embodiments

The schemes of the present invention will now be explained in conjunction with the embodiments. Those skilled in the art will understand that the following embodiments are for illustrative purposes only and should not be construed as limiting the scope of the present invention. In the embodiments, where specific techniques or conditions are not specified, the techniques or conditions described in the literature in the art or the product instructions will be followed. Reagents or instruments used for which the manufacturer is not specified are all conventional products that can be obtained by commercial purchase.

### Example 1

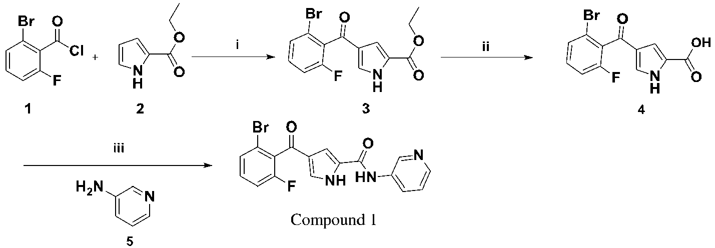

The preparation operating conditions shown in Example 1 above include: i, aluminum trichloride, super-dry dichloromethane, 0°C to room temperature, 16 hours; ii, sodium hydroxide, ethanol:water, 80°C, 1.5 hours; iii, 1-propylphosphonic anhydride, 4-dimethylaminopyridine, super-dry tetrahydrofuran, room temperature, 12 hours, 79%.

### Intermediate 3: Preparation of ethyl 4-(2-bromo-6-fluorophenyl)-1H-pyrrole-2-carboxylate

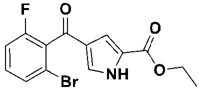

2-Bromo-6-fluorobenzoyl chloride (3.27 mL, 24 mmol) was added to ethyl 1H-pyrrole-2-carboxylate (1.5 g, 12 mmol), and a suspension of AlCl₃ (4 g, 30 mmol) in DCM (1 mL/mmol AlCl₃) was added at 0°C. After the mixture reached room temperature, it was stirred for 16 hours. The reaction is quenched with 1M aqueous HCl (20 mL) at 0°C. The product was extracted with dichloromethane (3 × 100 mL), washed with saturated NaHCO₃ solution (2 × 100 mL) and saturated sodium chloride solution (100 mL), dried over Na₂SO₄, and concentrated in vacuo to obtain a white solid, which was Compound 3. ¹H NMR(400MHz,DMSO-d₆)δ 12.90(s,1H),7.59(d, J =8.3 Hz,1H)7.52(s,1H),7.50(dd, J=6.3,8.3 Hz,1H),7.39-7.43(m,1H),7.00(s,1H),4.21(q, J=6.9 Hz,2H),1.25(t, J=7.0 Hz,3H), MS (ESI, positive ion) m/z:325.9[M + H]⁺ₒ

### Intermediate 4: Preparation of 4-(2-bromo-6-fluorophenyl)-1H-pyrrole-2-carboxylic acid

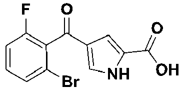

A 2N aqueous NaOH solution (10.0 mL, 20.0 mmol) was added to a solution of ethyl 4-(2-bromo-6-fluorobenzoyl)-1H-pyrrole-2-carboxylate (Intermediate 3, 1.00 g, 3.08 mmol) in ethanol (5.0 mL). The mixture was stirred at 80°C for 1.5 h and evaporated under reduced pressure. The residue was diluted with water and acidified with aqueous hydrochloric acid (3N). The aqueous mixture was extracted with ethyl acetate (2 × 50 mL), and the organic phase was washed with saturated brine (50 mL), dried over sodium sulfate, and filtered. The solvent was removed in vacuo, and the product was used in the next step without further purification.

### Compound 1: Preparation of 4-(2-bromo-6-fluorophenyl)-N-(pyridin-3-yl)-1H-pyrrole-2-carboxamide

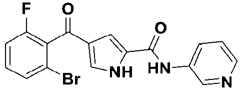

A solution of 4-(2-bromo-6-fluorobenzoyl)-1H-pyrrole-2-carboxylic acid (Intermediate 4, 0.50 g, 1.70 mmol), pyridin-3-amine (0.16 g, 1.70 mmol), 4-dimethylaminopyridine (0.42 g, 3.40 mmol), and 1-propylphosphonic anhydride (50 wt. % solution in ethyl acetate, 1.00 mL) in super-dry tetrahydrofuran (30 mL) was stirred at room temperature for 12 hours. It was then quenched with water and evaporated under reduced pressure. The residue was purified. Purification by chromatography (petroleum ether/ethyl acetate = 1:6) afforded a white solid, which was Compound 1. Yield 79%. ¹H NMR (400 MHz, DMSO-d₆) *δ* 12.71(s,1H),10.25(s,1H),8.90(s,1H),8.31(s,1H),8.14(d, *J*=6.8 Hz,1H),7.62(d, *J*=7.9 Hz,1H),7.56-7.42(m,3H),7.39(dt, *J* =8.3,4.0 Hz,1H).¹³C NMR (101 MHz, DMSO) *δ* 185.35,159.19,144.88,142.12,135.98,132.69,132.61,130.54,130.31,129.86,129.36,129.33,128. 63,127.48,125.43,124.04,119.58,119.52,115.95,115.74,112.13. HRMS (m/z): calculated for C₁₇H₁₁BrFN₃O₂⁺[M+H]⁺388.0019; found,388.0092.

### Example 2

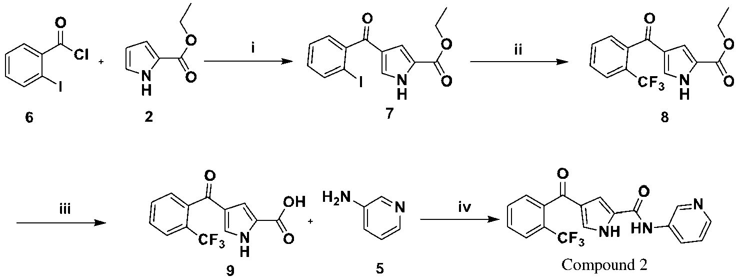

The preparation operating conditions shown in Example 2 above include: i, aluminum trichloride, super-dry dichloromethane, 0°C to room temperature, 10 hours; ii, methyl fluorosulfonyldifluoroacetate, copper(I) iodide, super-dry DMF, 80°C, 12 hours; iii, sodium hydroxide, ethanol:water, 15°C, 12 hours; iv, 1-propylphosphonic anhydride, 4-dimethylaminopyridine, super-dry tetrahydrofuran, room temperature, 12 hours, 85%.

### Intermediate 7: Preparation of ethyl 4-(2-iodophenyl)-1H-pyrrole-2-carboxylate

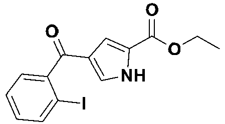

To anhydrous dichloromethane at 0°C was added 2-iodobenzoyl chloride (5.77 g, 21.72 mmol), followed by ethyl 1H-pyrrole-2-carboxylate (1.50 g, 10.86 mmol), and then 27.15 mmol of AlCl₃ to obtain a suspension. After the mixture reached room temperature, it was stirred for 10 hours. The reaction was quenched with 1M hydrochloric acid (20 mL) at 0°C. The reaction mixture was extracted with dichloromethane (3 × 100 mL) and washed with saturated NaHCO₃ solution (2 × 100 mL) and saturated brine (100 mL). The organic phase was dried over Na₂SO₄ and concentrated in vacuo to obtain a white solid. This was Intermediate 7. ¹H NMR(400MHz, DMSO-*d*₆) δ11.94(s,1H),8.05(d, *J*=1.5 Hz,1H),7.98(d, J=7.5 Hz,1H),7.71(dd, *J* =7.5,1.5 Hz,1H),7.58(t, *J*=4.8 Hz,1H),2.89(d, *J*=4.8 Hz,3H), MS (ESI, positive ion) m/z:369.8[M + H]⁺.

### Intermediate 8: Preparation of ethyl 4-(2-trifluoromethylphenyl)-1H-pyrrole-2-carboxylate

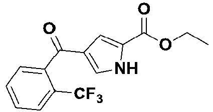

To a Schlenk tube were added copper(I) iodide (0.2 mol) and ethyl 4-(2-iodobenzoyl)-1H-pyrrole-2-carboxylate (2.71 mmol). Under argon protection, anhydrous DMF (20 mL) and methyl fluorosulfonyldifluoroacetate (1.56 g, 8.13 mmol) were added. The reaction mixture was then gradually heated to 80°C and stirred for 12 hours, after which water (40 mL) was added. The mixture was extracted with ethyl acetate (3 × 20 mL), dried over Na₂SO₄, and concentrated to obtain the crude product. Purification by column chromatography afforded ethyl 4-(2-trifluoromethylphenyl)-1H-pyrrole-2-carboxylate (Intermediate 8). ¹H NMR (400 MHz, DMSO-*d*₆) δ 12.77(s,1H),7.87(dd, *J*=7.6,1.4 Hz,1H),7.81-7.71(m,2H),7.58(d, *J*=7.1 Hz,1H),7.39(d, *J=1.7* Hz,1H),6.97(d, *J*=1.7 Hz,1H),4.26(q, *J=7.1* Hz,2H),1.28(t, *J =*7.1 Hz,3H), MS (ESI, positive ion) m/z:311.9[M + H]⁺.

### Intermediate 9: Preparation of 4-(2-trifluoromethylphenyl)-1H-pyrrole-2-carboxylic acid

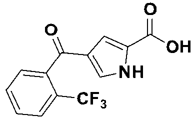

To a solution of 4-(2-(trifluoromethyl)benzoyl)-1H-pyrrole-2-carboxylic acid (Intermediate 8) (200 mg) in ethanol (30 ml) was added 2M aqueous NaOH solution (20 ml). The reaction mixture was stirred at 75°C for 1.5 hours. After confirming the reaction by thin-layer chromatography, the ethanol was concentrated, and the solution was then acidified with hydrochloric acid to a pH of approximately 3. The aqueous layer was extracted with ethyl acetate, and the combined organic layers were concentrated. Intermediate 9 was used in the next step without further purification.

### Compound 2: Preparation of N-(pyridin-3-yl)-4-(2-(trifluoromethyl)phenyl)-1H-pyrrole-2-carboxamide

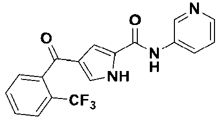

A system containing pyridin-3-amine (5, 0.15 g, 1.58 mmol), 4-(2-(trifluoromethyl)benzoyl)-1H-pyrrole-2-carboxylic acid (9, 0.45 g, 1.58 mmol), 1-propylphosphonic anhydride (50 wt. % solution in ethyl acetate, 1.88 mL, 3.16 mmol) and 4-dimethylaminopyridine (0.18 g, 3.16 mmol) in super-dry tetrahydrofuran (10 mL) was stirred at room temperature for 12 hours. After quenching with aqueous hydrochloric acid solution, the solvent was removed under reduced pressure. The residue was loaded onto silica gel and purified by column chromatography (petroleum ether/ethyl acetate = 1:5) to obtain a white solid, which was Compound 2, with a yield of 85%. ¹H NMR (400 MHz, DMSO-*d*₆) *δ* 12.63(s,1H),10.24(s,1H),8.90(d, *J*=2.5 Hz,1H),8.30(dd, *J*=4.6,1.4 Hz,1H),8.14(dt, *J*=8.5,2.0 Hz,1H),7.89(d, *J*=7.7 Hz,1H),7.79(dt, *J*=15.6,7.5 Hz,2H),7.63(d, *J*=7.4 Hz,1H),7.50(s,1H),7.39(dd, *J*=8.3,4.7 Hz,1H),7.34(s,1H).¹³C NMR (101 MHz, DMSO-*d*₆) *δ* 189.53,159.30,144..83,142.06,139.45,139.43,136.03,132.80,130.54,129.80,128.63,128.23,127 .43,127.11,127.06,126.62,126.31,125.64,124.04,122.93,112.59. HRMS (m/z): calculated for C₁₈H₁₂F₃N₃O₂⁺[M+H]⁺360.0949; found,360.0952.

**Table 1 NMR and HRMS of Compounds 3-153**

| Comp ound | Structure | NMR | HRMS |
|---|---|---|---|
| **3** | 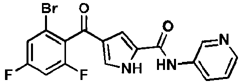 **Exact Mass: 404.9924** | ¹H NMR (400 MHz, DMSO-*d₆*) *δ* 12.73 (s, 1H), 10.24 (s, 1H), 8.90 (s, 1H), 8.31(s, 1H), 8.14 (s, 1H), 7.68-7.40 (m, 5H). ¹³C NMR (101 MHz, DMSO-*d₆*) *δ* 184.64, 161.48, 161.34, 160.63, 160.49, 159.18, 158.16, 158.02, 144.89, 142.11, 135.99, 130.38, 128.72, 127.48, 125.44, 124.07, 120.42, 120.34, 120.29, 120.22, 117.26, 117.23, 117.01, 116.98, 112.04, 105.21, 104.95, 104.69. | 405.99 95 |
| **4** | 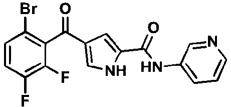 **Exact Mass: 404.9924** | ¹H NMR (400 MHz, DMSO-*d₆*) δ 12.78 (s, 1H), 10.26 (s, 1H), 8.90 (d, *J* = 2.5 Hz, 1H), 8.31 (dd, *J* = 4.7, 1.5 Hz, 1H), 8.14 (dt, *J* = 8.6, 1.9 Hz, 1H), 7.68-7.57 (m, 3H), 7.54 (s, 1H), 7.39 (dd, *J =* 8.3, 4.7 Hz, 1H). ¹³C NMR (101 MHz, DMSO-*d₆*) *δ* 183.88, 159.17, 150.86, 150.76, 148.40, 148.30, 148.26, 145.92, 145.79, 144.90, 142.12, 135.96, 132.19, 132.00, 130.81, 129.93, 129.89, 129.86, 129.83, 128.91, 127.50, 125.01, 124.04, 120.07, 119.89, 113.77, 113.73, 113.70, 112.03. | 405.99 92 |
| **5** | 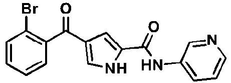 **Exact Mass: 369.0113** | ¹H NMR (400 MHz, DMSO-*d₆*) *δ* 12.61 (s, 1H), 10.24 (s, 1H), 8.91 (d, *J* = 2.6 Hz, 1H), 8.28 (dt, *J* = 4.8, 1.2 Hz, 1H), 8.17-8.14 (m, 1H), 7.74 (d, *J =* 7.9 Hz, 1H), 7.55-7.40 (m, 4H), 7.38 (dd, *J* = 8.3, 4.7 Hz, 1H), 7.30 (d, *J =* 1.4 Hz, 1H). ¹³C NMR (101 MHz, DMSO-*d₆*) *δ* 189.43, 159.96, 144.57, 142.35, 141.93, 136.22, 133.34, 131.47, 128.94, 128.00, 127.16, 125.46, 124.00, 118.81, 112.97. | 370.01 85 |
| **6** | 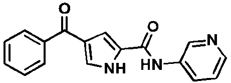 **Exact Mass: 291.1008** | ¹H NMR (400 MHz, DMSO-*d₆*) *δ* 12.57 (s, 1H), 10.25 (s, 1H), 8.95 (s, 1H), 8.32 (s, 1H), 8.17 (d, *J =* 8.3 Hz, 1H), 7.84-7.81 (m, 2H), 7.69-7.60 (m, 2H), 7.61-7.50 (m, 3H), 7.40 (s, 1H). ¹³C NMR (101 MHz, DMSO-*d₆*) *δ* 189.85, 159.46, 144.71, 142.02, 139.54, 132.26, 129.21, 129.03, 128.99, 127.70, 127.30, 124.76, 113.45. | 292.10 80 |
| **7** | 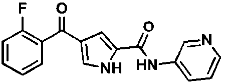 **Exact Mass: 309.0914** | ¹H NMR (400 MHz, DMSO-*d₆*) *δ* 12.62 (s, 1H), 10.25 (s, 1H), 8.91 (d, *J =* 2.5 Hz, 1H), 8.30 (d, *J =* 4.0 Hz, 1H), 8.16 (d, *J =* 8.7 Hz, 1H), 7.65-7.57 (m, 3H), 7.46 (s, 1H), 7.41-7.34 (m, 3H). ¹³ C NMR (101 MHz, DMSO-*d₆*) *δ* 186.79, 160.53, 159.33, 158.06, 144.81, 142.04, 136.07, 133.11, 133.02, 130.23, 130.20, 129.60, 128.68, 128.53, 128.05, 127.40, 125.83, 125.07, 125.04, 124.05, 116.86, 116.65, 112.71. | 310.09 76 |
| **8** | 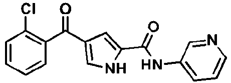 **Exact Mass: 325.0618** | ¹H NMR (400 MHz, DMSO-*d₆*) δ 12.63 (s, 1H), 10.25 (s, 1H), 8.91 (d, *J* = 2.5 Hz, 1H), 8.30 (d, *J* = 4.7 Hz, 1H), 8.15 (d, *J =* 8.3 Hz, 1H), 7.66-7.45 (m, 5H), 7.45-7.31 (m, 2H). ¹³ C NMR (101 MHz, DMSO-*d₆*) *δ* 188.90, 159.30, 144.83, 142.07, 139.89, 136.05, 131.61, 130.37, 129.96, 129.74, 129.06, 128.25, 127.64, 127.42, 125.56, 124.04, 112.61. | 326.06 87 |
| **9** | 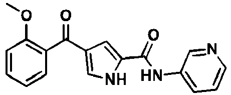 **Exact Mass: 321.1113** | ¹H NMR (400 MHz, DMSO-*d₆*) *δ* 12.45 (s, 1H), 10.21 (s, 1H), 8.92 (d, *J =* 2.5 Hz, 1H), 8.29 (dd, *J =* 4.7, 1.5 Hz, 1H), 8.16 (m, 1H), 7.53-7.45 (m, 2H), 7.38 (dd, *J* = 8.3, 4.7 Hz, 1H), 7.34-7.26 (m, 2H), 7.17 (d, *J =* 8.3 Hz, 1H), 7.05 (t, *J =* 7.4 Hz, 1H), 3.75 (s, 3H). ¹³C NMR (101 MHz, DMSO-*d₆*) δ 190.03, 159.46, 156.69, 144.74, 142.04, 136.13, 131.64, 130.55, 129.07, 128.64, 127.63, 127.36, 126.66, 124.02, 120.69, 112.65, 112.45, 55.96. | 322.11 82 |
| **10** | 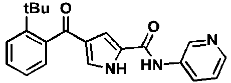 **Exact Mass: 347.1634** | ¹H NMR (400 MHz, DMSO-*d₆*) *δ* 12.52 (s, 1H), 10.23 (s, 1H), 8.92 (d, *J* = 2.6 Hz, 1H), 8.30 (dd, *J* = 4.7, 1.5 Hz, 1H), 8.21-8.10 (m, 1H), 7.56 (d, *J* = 8.0 Hz, 1H), 7.49 (s, 1H), 7.46-7.36 (m, 2H), 7.31-7.26 (m, 2H), 7.17 (dd, *J* = 7.6, 1.5 Hz, 1H), 1.32 (s, 9H). ¹³C NMR (101 MHz, DMSO-*d₆*) *δ* 194.82, 159.42, 147.55, 144.76, 142.08, 140.54, 136.10, 129.34, 129.12, 128.07, 127.85, 127.59, 127.49, 127.40, 125.44, 124.00, 112.75, 36.39, 32.31. | 348.17 01 |
| **11** | 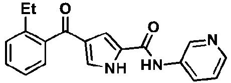 **Exact Mass: 319.1321** | ¹H NMR (400 MHz, DMSO-*d₆*) *δ* 12.54 (s, 1H), 10.24 (s, 1H), 8.92 (s, 1H), 8.30 (d, *J* = 4.7 Hz, 1H), 8.15 (d, *J* = 8.3 Hz, 1H), 7.51 (s, 1H), 7.45 (t, *J* = 7.5 Hz, 1H), 7.40-7.30 (m, 5H), 2.64 (q, *J* = 7.5 Hz, 2H), 1.10 (t, *J* = 7.5 Hz, 3H). ¹³C NMR (101 MHz, DMSO-*d*₆) *δ* 192.45, 159.41, 144.77, 142.06, 142.01, 140.04, 136.10, 130.24, 129.80, 129.27, 127.91, 127.75, 127.38, 126.53, 125.83, 124.02, 112.84, 26.10, 16.55. | 320.13 88 |
| **12** | 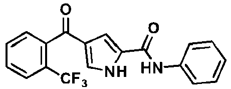 **Exact Mass: 358.0929** | ¹H NMR (400 MHz, DMSO-*d₆*) δ 12.58 (s, 1H), 10.07 (s, 1H), 7.89 (d, *J* = 7.8 Hz, 1H), 7.86-7.69 (m, 4H), 7.64 (d, *J* = 7.4 Hz, 1H), 7.54 (s, 1H), 7.35 (dd, *J* = 15.6, 7.7 Hz, 3H), 7.10 (t, *J* = 7.4 Hz, 1H). ¹³C NMR (101 MHz, DMSO-*d₆*) δ 189.54, 158.99, 139.54, 139.52, 139.33, 132.74, 130.47, 129.54, 129.11, 128.77, 128.64, 127.12, 127.07, 127.03, 126.98, 126.66, 126.35, 125.60, 123.93, 122.94, 120.52, 112.10. | 359.10 07 |
| **13** | 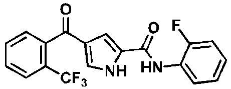 **Exact Mass: 376.0835** | ¹H NMR (400 MHz, DMSO-*d₆*) *δ* 12.58 (s, 1H), 9.98 (s, 1H), 7.89 (d, *J =* 7.7 Hz, 1H), 7.85-7.69 (m, 2H), 7.65-7.54 (m, 2H), 7.45 (s, 1H), 7.41-7.11 (m, 4H). ¹³C NMR (101 MHz, DMSO-*d₆*) *δ* 189.52, 159.05, 157.36, 154.91, 139.50, 132.77, 130.49, 129.44, 129.02, 128.62, 128.13, 127.56, 127.34, 127.26, 127.07, 127.02, 126.58, 126.27, 125.61, 125.50, 124.79, 124.76, 116.41, 116.22, 112.68. | 377.09 09 |
| **14** | 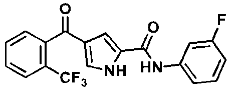 **Exact Mass: 376.0835** | ¹H NMR (400 MHz, DMSO-*d₆*) δ 12.60 (s, 1H), 10.21 (s, 1H), 7.89 (d, *J* = 7.7 Hz, 1H), 7.86-7.68 (m, 3H), 7.62 (d, *J =* 7.4 Hz, 1H), 7.55-7.49 (m, 2H), 7.38 (q, *J* = 7.8 Hz, 1H), 7.32 (s, 1H), 6.91 (td, *J* = 8.4, 2.5 Hz, 1H). ¹³C NMR (101 MHz, DMSO-*d₆*) *δ* 189.52, 163.73, 161.34, 159.16, 141.18, 141.07, 139.47, 139.45, 132.76, 130.77, 130.67, 130.50, 129.83, 128.63, 128.40, 127.09, 127.04, 126.66, 126.35, 125.63, 122.93, 116.10, 116.07, 112.45, 110.43, 110.22, 107.26, 107.00. | 377.09 14 |
| **15** | 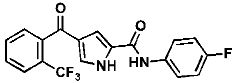 **Exact Mass: 376.0835** | ¹H NMR (400 MHz, DMSO-*d₆*) *δ* 12.55 (s, 1H), 10.10 (s, 1H), 7.89 (d, *J* = 7.1 Hz, 1H), 7.87-7.65 (m, 4H), 7.62 (d, *J* = 7.4 Hz, 1H), 7.47 (d, *J* = 1.6 Hz, 1H), 7.29 (d, *J* = 1.6 Hz, 1H), 7.25 - 7.12 (m, 2H). ¹³C NMR (101 MHz, DMSO-*d₆*) δ 189.54, 159.86, 158.92, 157.47, 139.50, 135.65, 132.73, 130.47, 129.58, 128.62, 127.07, 127.02, 126.66, 126.34,125.60,122.34,122.27,115.80,115.58, 12.09. | 377.09 07 |
| **16** | 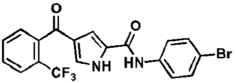 **Exact Mass: 436.0034** | ¹11 NMR (400 MHz, DMSO-*d₆*) *δ* 12.58 (s, 111), 10.15 (s, 1H), 7.88 (d, *J =* 7.7 Hz, 1H), 7.83-7.70 (m, 4H), 7.62 (d, *J =* 7.4 Hz, 1H), 7.57-7.47 (m, 3H), 7.31 (s, 1H). ¹³C NMR (101 MHz, DMSO-*d₆*) *δ* 189.51, 159.03, 139.49, 139.47, 138.75, 132.72, 131.93, 130.46, 129.75, 128.63, 128.52, 127.11, 127.07, 127.02, 126.98, 126.68, 126.37, 125.63, 122.35, 115.61, 112.36. | 437.23 72 |
| **17** | 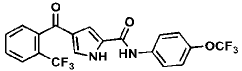 **Exact Mass: 442.0752** | ¹H NMR (400 MHz, DMSO-*d₆*) *δ* 12.59 (s, 1H), 10.22 (s, 1H), 7.99-7.67 (m, 5H), 7.62 (d, *J* = 7.4 Hz, 1H), 7.51 (d, *J* = 1.5 Hz, 1H), 7.36 (d, *J* = 8.7 Hz, 2H), 7.31 (d, *J* = 1.6 Hz, 1H). ¹³C NMR (101 MHz, DMSO-*d₆*) *δ* 189.50, 159.07, 144.20, 144.18, 139.48, 139.45, 138.56, 132.74, 130.49, 129.75, 128.63, 128.43, 127.12, 127.07, 127.03, 126.97, 126.66, 126.34, 125.62, 122.92, 121.98, 121.76, 119.34, 112.37. | 443.08 32 |
| **18** | 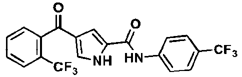 **Exact Mass: 426.0803** | ¹H NMR (400 MHz, DMSO-*d₆*) *δ* 12.63 (s, 1H), 10.35 (s, 1H), 7.97 (d, *J* = 8.5 Hz, 2H), 7.89 (d, *J* = 7.7 Hz, 1H), 7.83-7.69 (m, 4H), 7.63 (d, *J* = 7.4 Hz, 1H), 7.55 (d, *J* = 1.5 Hz, 1H), 7.34 (d, *J* = 1.5 Hz, 1H). ¹³C NMR (101 MHz, DMSO-*d₆*) *δ* 189.50, 159.29, 143.03, 139.43, 139.41, 132.79, 130.55, 129.98, 128.64, 128.27, 127.15, 127.11, 127.06, 127.01, 126.64, 126.48, 126.45, 126.41, 126.37, 126.32, 126.21, 125.65, 124.01, 123.69, 123.51, 122.92, 120.22, 112.77 | 427.08 82 |
| **19** | 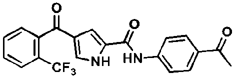 **Exact Mass: 400.1035** | ¹H NMR (400 MHz, DMSO-*d₆*) *δ* 12.62 (s, 1H), 10.33 (s, 1H), 7.97 (d, *J* = 8.8 Hz, 2H), 7.93-7.86 (m, 3H), 7.84-7.73 (m, 2H), 7.62 (d, *J =* 7.3 Hz, 1H), 7.55 (d, *J* = 1.6 Hz, 1H), 7.34 (d, *J* = 1.5 Hz, 1H), 2.54 (s, 3H). ¹³C NMR (101 MHz, DMSO-*d₆*) *δ* 197.06, 189.53, 159.22, 143.80, 139.42, 139.40, 132.81, 132.28, 130.56, 130.00, 129.83, 128.64, 128.35, 127.12, 127.07, 126.62, 126.31, 125.65, 122.92, 119.57, 112.78, 26.91. | 401.11 07 |
| **20** | 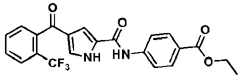 **Exact Mass: 430.1140** | ¹H NMR (400 MHz, DMSO-*d₆*) δ 12.62 (s, 1H), 10.33 (s, 1H), 8.01-7.85 (m, 5H), 7.82-7.73 (m, 2H), 7.62 (d, *J* = 7.4 Hz, 1H), 7.55 (s, 1H), 7.34 (s, 1H), 4.30 (q, *J* = 7.1 Hz, 2H), 1.32 (t, *J =* 7.1 Hz, 3H). ¹³C NMR (101 MHz, DMSO-*d₆*) δ 189.52, 165.82, 159.22, 143.81, 139.43, 139.41, 132.80, 130.58, 130.01, 128.64, 128.36, 127.11, 127.07, 126.61, 126.30, 125.65, 124.82, 122.92, 119.68, 112.80, 60.92, 14.68. | 431.12 14 |
| **21** | 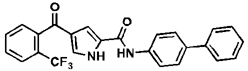 **Exact Mass: 434.1242** | ¹H NMR (400 MHz, DMSO-*d₆*) *δ* 12.58 (s, 1H), 10.14 (s, 1H), 7.97-7.72 (m, 5H), 7.72-7.55 (m, 5H), 7.52 (d, *J* = 1.6 Hz, 1H), 7.46 (t, *J* = 7.7 Hz, 2H), 7.39-7.26 (m, 2H). ¹³C NMR (101 MHz, DMSO-*d₆*) *δ* 189.55, 159.00, 140.17, 139.52, 138.84, 135.61, 132.77, 130.50, 129.64, 129.36, 128.75, 128.65, 127.50, 127.33, 127.09, 127.05, 126.73, 125.63, 120.82, 112.19. | 435.13 10 |
| **22** | 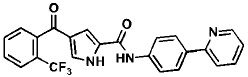 **Exact Mass: 435.1195** | ¹H NMR (400 MHz, DMSO-*d₆*) *δ* 12.59 (s, 1H), 10.19 (s, 1H), 8.65 (dd, *J* = 5.0, 1.7 Hz, 1H), 8.10 (d, *J* = 8.6 Hz, 2H), 7.94 (d, *J* = 8.0 Hz, 1H), 7.92-7.83 (m, 4H), 7.81-7.74(m, 2H), 7.64 (d, *J* = 7.4 Hz, 1H), 7.54 (s, 1H), 7.37-7.25 (m, 2H). ¹³C NMR (101 MHz, DMSO-*d₆*) *δ* 189.53, 159.02, 156.06, 149.90, 140.26, 139.51, 137.59, 134.10, 132.79, 130.53, 129.68, 128.66, 127.36, 127.11, 127.06, 126.63, 126.32, 125.62, 122.59, 120.37, 120.10, 112.32. | 436.12 59 |
| **23** | 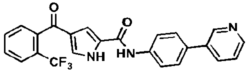 **Exact Mass: 435.1195** | ¹H NMR (400 MHz, DMSO-*d₆*) *δ* 12.60 (s, 1H), 10.18 (s, 1H), 8.91 (d, *J* = 2.5 Hz, 1H), 8.55 (d, *J* = 4.2 Hz, 1H), 8.07 (d, *J* = 8.1 Hz, 1H), 7.89 (d, *J* = 8.2 Hz, 3H), 7.85-7.70 (m, 4H), 7.63 (d, *J =* 7.5 Hz, 1H), 7.53 (s, 1H), 7.47 (dd, *J* = 8.0, 4.7 Hz, 1H), 7.32 (s, 1H). ¹³C NMR (101 MHz, DMSO-*d₆*) δ 189.54, 159.05, 148.53, 147.78, 139.49, 135.56, 134.06, 132.79, 132.37, 130.53, 129.70, 128.65, 127.59, 127.15, 127.10, 127.06, 127.01, 126.63, 126.32, 125.62, 124.30, 122.94, 120.88, 112.27. | 436.12 62 |
| **24** | 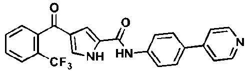 **Exact Mass: 435.1195** | ¹H NMR (400 MHz, DMSO-*d₆*) *δ* 12.60 (s, 1H), 10.23 (s, 1H), 8.61 (d, *J* = 6.1 Hz, 2H), 7.91 (t, *J* = 8.3 Hz, 3H), 7.88-7.74 (m, 4H), 7.71 (d, *J =* 6.2 Hz, 2H), 7.63 (d, *J =* 7.4 Hz, 1H), 7.54 (s, 1H), 7.32 (s, 1H). ¹³C NMR (101 MHz, DMSO-*d₆*) *δ* 189.53, 159.09, 150.64, 146.86, 140.53, 139.47, 132.79, 132.19, 130.53, 129.77, 128.65, 128.60, 127.63, 127.11, 127.06, 126.64, 126.33, 125.63, 121.10, 120.74, 112.41. | 436.12 67 |
| **25** | 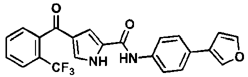 **Exact Mass: 424.1035** | ¹H NMR (400 MHz, DMSO-*d*₆) *δ* 12.58 (s, 1H), 10.11 (s, 1H), 8.16 (s, 1H), 7.91 (d, *J* = 7.7 Hz, 1H), 7.85-7.75 (m, 5H), 7.66-7.61 (m, 3H), 7.52 (s, 1H), 7.33 (s, 1H), 6.97 (d, *J =* 0.9 Hz, 1H). ¹³C NMR (101 MHz, DMSO-*d*₆) *δ* 189.54, 158.89, 144.65, 139.52, 139.49, 139.23, 138.15, 132.79, 130.51, 129.54, 128.72, 128.65, 127.61, 127.10, 127.05, 126.62, 126.31, 126.28, 125.99, 125.66, 125.59, 122.94, 120.73, 112.08, 109.08 | 425.11 10 |
| **26** | 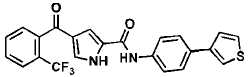 **Exact Mass: 440.0806** | ¹H NMR (400 MHz, DMSO-*d₆*) *δ* 12.58 (s, 1H), 10.11 (s, 1H), 7.89 (d, *J* = 7.7 Hz, 1H), 7.86-7.78 (m, 4H), 7.76 (d, *J* = 7.5 Hz, 1H), 7.71 (d, *J* = 8.6 Hz, 2H), 7.67-7.60 (m, 2H), 7.56 (d, *J* = 5.0 Hz, 1H), 7.52 (s, 1H), 7.31 (s, 1H). ¹³C NMR (101 MHz, DMSO-*d₆*) *δ* 189.53, 158.92, 141.59, 139.53, 139.50, 138.38, 132.78, 130.88, 130.51, 129.58, 128.74, 128.66, 127.43, 127.10, 127.05, 126.80, 126.64, 126.48, 126.32, 125.67, 125.60, 122.94, 120.71, 120.45, 112.12. | 441.08 83 |
| **27** | 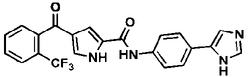 **Exact Mass: 424.1147** | ¹H NMR (400 MHz, DMSO-*d₆*) δ 12.56 (s, 1H), 12.15 (s, 1H), 10.06 (s, 1H), 7.89 (d, *J =* 7.8 Hz, 1H), 7.85-7.67 (m, 7H), 7.63 (d, *J* = 7.5 Hz, 1H), 7.49 (s, 2H), 7.29 (s, 1H). ¹³C NMR (101 MHz, DMSO-*d₆*) *δ* 189.54, 158.80, 139.53, 137.43, 136.28, 132.78, 130.51, 129.46, 128.82, 128.66, 127.14, 127.09, 127.05, 126.99, 126.30, 125.57, 124.96, 122.94, 120.61, 112.01. | 425.12 17 |
| **28** | 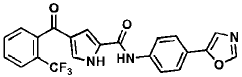 **Exact Mass: 425.0987** | ¹¹H NMR (400 MHz, DMSO-*d₆*) δ 12.56 (s, 1H), 10.18 (s, 1H), 8.41 (s, 1H), 7.94-7.84 (m, 3H), 7.82-7.75 (m, 2H), 7.72 (s, 1H), 7.70 (s, 1H), 7.61 (d, *J* = 12.2 Hz, 2H), 7.50 (s, 1H), 7.31 (s, 1H). ¹³C NMR (101 MHz, DMSO-*d₆*) *δ* 189.53, 159.04, 151.87, 150.97, 139.70, 139.50, 139.48, 132.76, 130.50, 129.74, 128.65, 128.58, 127.14, 127.09, 127.04, 127.00, 126.66, 126.34, 125.64, 125.13, 123.00, 121.51, 120.70, 112.37. | 426.12 30 |
| **29** | 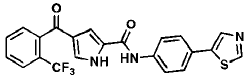 **Exact Mass: 441.0759** | ¹H NMR (400 MHz, DMSO-*d₆*) *δ* 12.59 (s, 1H), 10.19 (s, 1H), 9.05 (s, 1H), 8.26 (s, 1H), 7.89 (d, *J* = 7.7 Hz, 1H), 7.84 (d, *J =* 8.6 Hz, 2H), 7.82-7.72 (m, 2H), 7.68 (d, *J* = 8.4 Hz, 2H), 7.63 (d, *J* = 7.4 Hz, 1H), 7.52 (s, 1II), 7.32 (s, 1H). ¹³C NMR (101 MHz, DMSO-*d₆*) *δ* 189.53, 159.01, 153.37, 139.57, 139.49, 139.47, 139.18, 138.93, 132.78, 130.51, 129.71, 128.65, 128.58, 127.51, 127.15, 127.10, 127.05, 127.00, 126.64, 126.26, 125.66, 125.63, 122.94, 120.86, 112.33. ¹⁹F NMR (376 MHz, DMSO-*d₆*) *δ* -56.77. | 442.08 35 |
| **30** | 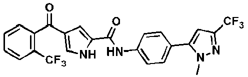 **Exact Mass: 506.1177** | ¹H NMR (400 MHz, DMSO-*d₆*) *δ* 12.62 (s, 1H), 10.24 (s, 1H), 7.91 (t, *J* = 7.9 Hz, 3H), 7.83-7.74 (m, 2H), 7.63 (d, *J* = 7.4 Hz, 1H), 7.59 (d, *J* = 8.7 Hz, 2H), 7.54 (s, 1H), 7.32 (s, 1H), 6.89 (s, 1H), 3.95 (s, 3H). ¹³C NMR (101 MHz, DMSO-*d₆*) δ 189.52, 159.15, 145.08, 140.24, 139.75, 139.48, 132.81, 130.55, 129.83, 129.70, 128.65, 128.53, 127.12, 127.07, 126.32, 125.66, 125.63, 123.90, 120.71, 120.36, 112.41, 104.49, 38.83. | 507.12 55 |
| **31** | 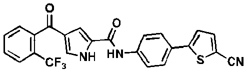 **Exact Mass: 465.0759** | ¹H NMR (400 MHz, DMSO-*d₆*) δ 12.61 (s, 1H), 10.25 (s, 1H), 7.98 (d, *J* = 4.0 Hz, 1H), 7.90-7.87 (m, 3H), 7.83-7.74 (m, 4H), 7.63 (d, *J* = 4.3 Hz, 2H), 7.53 (s, 1H), 7.33 (s, 1H). ¹³C NMR (101 MHz, DMSO-*d₆*) *δ* 189.51, 159.06, 151.62, 140.76, 140.69, 139.46, 132.80, 130.54, 129.81, 128.65, 128.48, 127.23, 127.15, 126.64, 126.32, 125.63, 124.24, 122.93, 120.75, 115.00, 112.47, 106.29. | 466.53 37 |
| **32** | 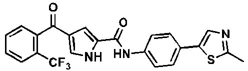 **Exact Mass: 455.0915** | ¹H NMR (400 MHz, DMSO-*d*₆) δ 12.59 (s, 1H), 10.18 (s, 1H), 7.96 (s, 1H), 7.89 (d, *J* = 7.7 Hz, 1H), 7.86-7.71 (m, 4H), 7.64-7.59 (m, 3H), 7.53 (s, 1H), 7.32 (d, *J* = 1.8 Hz, 1H), 2.66 (s, 3H). ¹³C NMR (101 MHz, DMSO-*d₆*) *δ* 189.52, 164.54, 158.99, 139.50, 139.48, 139.24, 138.42, 137.89, 132.76, 130.50, 129.69, 128.64, 128.61, 127.07, 126.65, 126.63, 126.34, 125.66, 125.62, 122.94, 120.85, 112.29, 19.41. | 456.09 87 |
| **33** | 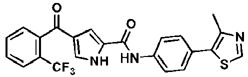 **Exact Mass: 455.0915** | ¹H NMR (400 MHz, DMSO-*d₆*) δ 12.61 (s, 1H), 10.21 (s, 1H), 8.97 (s, 1H), 7.97-7.83 (m, 3H), 7.85-7.69 (m, 2H), 7.63 (d, *J =* 7.4 Hz, 1H), 7.55 (s, 1H), 7.49 (d, *J* = 8.5 Hz, 2H), 7.32 (d, *J* = 1.7 Hz, 1H), 2.48 (s, 3H). ¹³C NMR (101 MHz, DMSO-*d₆*) *δ* 189.52, 159.06, 151.61, 148.05, 139.50, 139.48, 139.15, 132.77, 131.57, 130.51, 129.77, 128.65, 128.61, 127.10, 127.05, 126.83, 126.65, 126.34, 125.66, 125.62, 122.94, 120.61, 112.31, 16.49. | 456.09 88 |
| **34** | 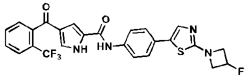 **Exact Mass: 514.1087** | ¹H NMR (400 MHz, DMSO-*d₆*) *δ* 12.55 (s, 1H), 10.11 (s, 1H), 7.89 (d, *J =* 7.7 Hz, 1H), 7.84-.71 (m, 4H), 7.62 (d, *J* = 7.4 Hz, 1H), 7.55 (s, 1H), 7.53-7.42 (m, 3H), 7.30 (s, 1H), 5.62 (tt, *J* = 6.0, 3.1 Hz, 0.5H), 5.48 (tt, *J =* 6.1, 3.1 Hz, 0.5H), 4.43-4.31 (m, 2H), 4.20-4.08 (m, 2H). ¹³C NMR (101 MHz, DMSO-*d₆*) *δ* 189.52, 169.70, 169.68, 158.87, 139.51, 139.48, 138.16, 135.63, 132.78, 130.51, 129.61, 128.83, 128.66, 128.64, 127.46, 127.09, 127.05, 126.63, 126.32, 125.80, 125.60, 122.93, 120.88, 112.16, 85.61, 83.60, 61.41, 61.16. | 515.11 51 |
| **35** | 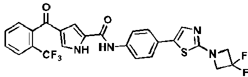 **Exact Mass: 532.0992** | ¹H NMR (400 MHz, DMSO-*d₆*) *δ* 12.56 (s, 1H), 10.12 (s, 1H), 7.89 (d, *J* = 7.7 Hz, 1H), 7.85-7.66 (m, 4H), 7.62 (d, *J* = 9.5 Hz, 2H), 7.49 (d, *J* = 6.9 Hz, 3H), 7.30 (s, 1H), 4.53 (t, *J* = 12.2 Hz, 4H). ¹³C NMR (101 MHz, DMSO-*d₆*) *δ* 189.51, 169.06, 158.90, 139.50, 138.44, 135.47, 132.78, 130.51, 130.21, 129.63, 128.64, 127.13, 127.10, 126.32, 126.01, 125.66, 125.60, 122.94, 120.87, 117.11, 112.19, 65.13, 64.86, 64.60. | 533.10 67 |
| **36** | 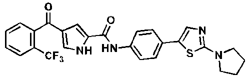 **Exact Mass: 510.1337** | ¹H NMR (400 MHz, DMSO-*d₆*) δ 12.54 (s, 1H), 10.08 (s, 1H), 7.89 (d, *J* = 7.2 Hz, 1H), 7.85-7.70 (m, 4H), 7.62 (d, *J =* 7.4 Hz, 1H), 7.52 (s, 1H), 7.48 (s, 1H), 7.43 (d, *J =* 8.7 Hz, 2H), 7.29 (s, 1H), 3.45-3.39 (m, 4H), 2.04-1.95 (m, 4H). ¹³C NMR (101 MHz, DMSO-*d₆*) *δ* 189.54, 166.25, 158.82, 139.51, 139.49, 137.51, 136.11, 132.76, 130.50, 129.55, 128.72, 128.64, 128.17, 127.09, 127.04, 126.63, 126.32, 125.66, 125.59, 125.36, 125.25, 122.94, 120.92, 112.07, 49.72, 25.61. | 511.14 15 |
| **37** | 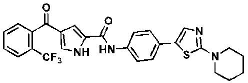 **Exact Mass: 524.1494** | ¹H NMR (400 MHz, DMSO-*d₆*) *δ* 12.55 (s, 1H), 10.09 (s, 1H), 7.89 (d, *J* = 7.7 Hz, 1H), 7.85-7.67 (m, 4H), 7.62 (d, *J* = 7.4 Hz, 1H), 7.50 (d, *J* = 8.5 Hz, 2H), 7.44 (d, *J* = 8.6 Hz, 2H), 7.30 (s, 1H), 3.44 (d, *J =* 4.6 Hz, 4H), 1.61 (s, 6H). ¹³C NMR (101 MHz, DMSO-*d₆*) *δ* 189.52, 170.09, 158.85, 139.52, 139.50, 137.78, 135.64, 132.75, 130.48, 129.57, 128.72, 128.63, 127.89, 127.08, 127.04, 126.65, 126.34, 126.15, 125.61, 125.47, 122.94, 120.90, 112.11, 49.50, 25.07, 24.06. | 525.15 69 |
| **38** | 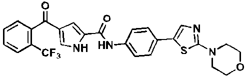 **Exact Mass: 526.1286** | ¹H NMR (400 MHz, DMSO-*d₆*) *δ* 10.13 (s, 1H), 7.88 (d, *J =* 7.8 Hz, 1H), 7.84-7.67 (m, 4H), 7.63 (d, *J* = 7.4 Hz, 1H), 7.57 (s, 1H), 7.53 (s, 1H), 7.47 (d, *J = 8.3* Hz, 2H), 7.32 (s, 1H), 3.72 (t, *J* = 4.7 Hz, 4H), 3.41 (t, *J* = 4.7 Hz, 4H). ¹³ C NMR (101 MHz, DMSO-*d₆*) *δ* 189.53, 170.30, 158.88, 139.53, 139.51, 138.06, 135.52, 132.74, 130.47, 129.61, 128.71, 128.64, 127.59, 127.15, 127.08, 127.03, 126.67, 126.35, 125.67, 125.62, 122.94, 120.91, 112.15, 65.83, 48.62. | 527.13 68 |
| **39** | 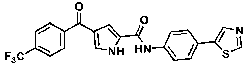 **Exact Mass: 441.08** | ¹H NMR (400 MHz, DMSO-*d₆*) *δ* 12.63 (s, 1H), 10.20 (s, 1H), 9.05 (s, 1H), 8.27 (s, 1H), 8.00 (d, *J =* 8.0 Hz, 2H), 7.93 (d, *J* = 8.1 Hz, 2H), 7.86 (d, *J* = 8.7 Hz, 2H), 7.74-7.64 (m, 3H), 7.61 (s, 1H). ¹³C NMR (101 MHz, DMSO-*d₆*) *δ* 189.10, 188.93, 159.10, 159.05, 153.38, 143.12, 139.62, 139.19, 137.88, 131.96, 131.64, 130.89, 129.70, 129.66, 128.37, 127.52, 126.23, 126.03, 126.00, 125.96, 125.92, 125.75, 124.34, 123.04, 120.82, 113.07. ¹⁹F NMR (376 MHz, DMSO-*d₆*) δ -61.41. | 442.08 35 |
| **40** | 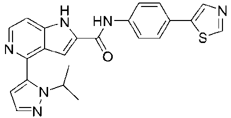 | 1H NMR (400 MHz, DMSO-d6) δ 12.42 (s, 1H), 10.52 (s, 1H), 9.06 (s, 1H), 8.41 (d, J = 5.8 Hz, 1H), 8.29 (s, 1H), 7.91 (d, J = 8.2 Hz, 2H), 7.79 - 7.63 (m, 4H), 7.46 (d, J = 5.8 Hz, 1H), 6.82 (s, 1H), 5.40 (q, J = 6.8 Hz, 1H), 1.42 (d, J = 6.6 Hz, 6H). | 429.13 98 |
| **41** | 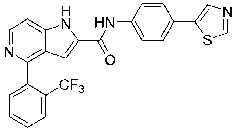 | 1H NMR (400 MHz, DMSO-d6) δ 12.36 (s, 1H), 10.38 (s, 1H), 9.05 (s, 1H), 8.33 (d, J = 5.8 Hz, 1H), 8.27 (d, J = 0.7 Hz, 1H), 7.98 - 7.93 (m, 1H), 7.85 (dd, J = 8.1, 6.1 Hz, 3H), 7.76 (t, J = 7.6 Hz, 1H), 7.70 (d, J = 8.7 Hz, 2H), 7.67 - 7.63 (m, 1H), 7.49 - 7.44 (m, 1H), 7.31 (s, 1H). | 465.09 21 |
| **42** | 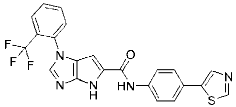 | 1H NMR (400 MHz, DMSO-d6) δ 11.84 (s, 1H), 9.81 (s, 1H), 9.03 (s, 1H), 8.24 (s, 111), 8.03 (d, J = 7.8 Hz, 1H), 7.90 (d, J = 6.4 Hz, 2H), 7.82 (t, J = 8.7 Hz, 3H), 7.74 (d, J = 7.9 Hz, 1H), 7.68 - 7.63 (m, 2H), 6.96 (s, 1H). | 454.09 11 |
| **43** | 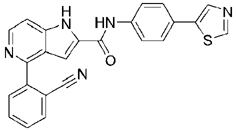 | 1H NMR (400 MHz, DMSO-d6) δ 12.36 (s, 1H), 10.55 (s, 1H), 9.05 (s, 1H), 8.31 - 8.21 (m, 2H), 7.88 (dd, J = 7.8, 4.9 Hz, 3H), 7.73 - 7.61 (m, 4H), 7.57 (td, J = 7.2, 2.0 Hz, 1H), 7.45 (s, 1H), 7.37 (d, J = 5.8 Hz, 1H). | 422.10 11 |
| **44** | 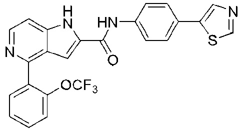 | 1H NMR (400 MHz, DMSO-d6) δ 12.35 (d, J = 2.1 Hz, 1H), 10.45 (s, 1H), 9.06 (s, 1H), 8.39 (d, J = 5.8 Hz, 1H), 8.28 (s, 1H), 7.92 - 7.86 (m, 2H), 7.77 - 7.70 (m, 3H), 7.67 (dd, J = 7.6, 2.1 Hz, 1H), 7.65 - 7.58 (m, 2H), 7.47 (d, J = 5.8 Hz, 1H), 7.44 (d, J = 2.0 Hz, 1H). | 481.10 02 |
| **45** | 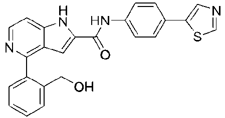 | 1H NMR (400 MHz, DMSO-d6) δ 12.38 (d, J = 2.1 Hz, 1H), 10.46 (s, 1H), 9.06 (s, 1H), 8.35 (d, J = 5.8 Hz, 1H), 8.28 (s, 1H), 7.93 - 7.83 (m, 2H), 7.75 - 7.66 (m, 3H), 7.63 (dd, J = 7.1, 1.8 Hz, 1H), 7.56 7.47 (m, 3H), 7.46 (d, J = 5.9 Hz, 1H), 5.58 (s, 1H), 4.44 (s, 2H). | 427.12 32 |
| **46** | 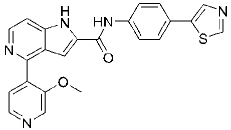 | 1H NMR (400 MHz, DMSO-d6) δ 12.24 (s, 1H), 10.36 (s, 1H), 8.99 (s, 1H), 8.29 (d, J = 5.3 Hz, 2H), 8.21 (s, 1H), 7.89 - 7.74 (m, 3H), 7.64 (d, J = 8.3 Hz, 2H), 7.39 (d, J = 4.2 Hz, 2H), 7.19 - 7.07 (m, 1H), 3.83 (s, 3H). | 428.09 13 |
| **47** | 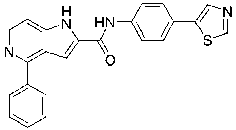 | 1H NMR (400 MHz, DMSO-d6) δ 12.37 (s, 1H), 10.54 (s, 1H), 9.06 (s, 1H), 8.38 (d, J = 5.7 Hz, 1H), 8.29 (s, 1H), 8.10 - 8.00 (m, 2H), 7.96 - 7.85 (m, 3H), 7.73 (d, J = 8.4 Hz, 2H), 7.61 (t, J = 7.5 Hz, 2H), 7.53 (t, J = 7.3 Hz, 1H), 7.42 (d, J = 5.7 Hz, 111). | 397.10 14 |
| **48** | 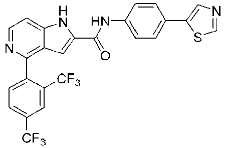 | 1H NMR (400 MHz, DMSO-d6) δ 12.40 (s, 1H), 10.39 (s, 1H), 8.99 (s, 1H), 8.34 (d, J = 5.8 Hz, 1H), 8.26 (s, 1H), 8.23 (d, J = 8.0 Hz, 2H), 7.87 (d, J = 7.9 Hz, 1H), 7.79 (d, J = 8.5 Hz, 2H), 7.67 (d, J = 8.7 IIz, 2II), 7.56 (d, J = 5.8 IIz, 1H), 7.26 (s, 1H). | 533.08 21 |
| **49** | 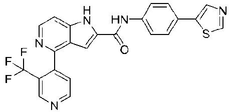 | 1H NMR (400 MHz, DMSO-d6) δ 12.48 (s, 1H), 10.41 (s, 1H), 9.05 (d, J = 9.0 Hz, 2H), 8.97 (s, 1H), 8.39 (d, J = 5.8 Hz, 1H), 8.28 (s, 1H), 8.01 (d, J = 5.2 Hz, 1H), 7.86 (d, J = 8.6 Hz, 2H), 7.71 (d, J = 8.7 Hz, 2H), 7.52 (d, J = 5.7 Hz, 1H), 7.39 (d, J = 2.0 Hz, 1H). | 466.09 01 |
| **50** | 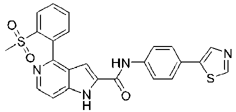 | 1H NMR (400 MHz, DMSO-d6) δ 12.43 - 12.34 (m, 1H), 10.41 (s, 1H), 9.05 (s, 1H), 8.33 (d, J = 5.8 Hz, 1H), 8.27 (s, 1H), 8.19 (dd, J = 8.0, 1.3 Hz, 1H), 7.92 (td, J = 7.6, 1.4 Hz, 1H), 7.90 - 7.85 (m, 2H), 7.82 (td, J = 7.7, 1.4 Hz, 1H), 7.75 - 7.66 (m, 3H), 7.49 - 7.44 (m, 1H), 7.36 (s, 1H), 3.54 (s, 3H). | 475.08 32 |
| **51** | 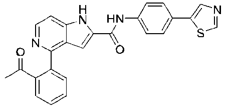 | 1H NMR (400 MHz, DMSO-d6) δ 11.08 (s, 1H), 9.08 (s, 1H), 8.80 (s, 2H), 8.49 (s, 1H), 8.32 (s, 1H), 8.06 (d, J = 8.1 Hz, 2H), 7.96 - 7.81 (m, 4H), 7.76 (d, J = 8.2 Hz, 3H), 1.97 (d, J = 46.4 Hz, 3H). | 439.12 19 |
| **52** | 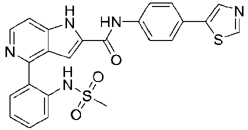 | 1H NMR (400 MHz, DMSO-d6) δ 12.56 (s, 1H), 11.52 (s, 1H), 10.56 (s, 1H), 9.06 (s, 1H), 8.41 (d, J = 5.8 Hz, 1H), 8.29 (s, 1H), 8.06 (dd, J = 7.8, 1.6 Hz, 1H), 7.97 - 7.81 (m, 3H), 7.73 (d, J = 8.6 Hz, 2H), 7.67 (d, J = 8.2 Hz, 1H), 7.61 - 7.49 (m, 2H), 7.44 (t, J = 7.5 Hz, 1H), 2.91 (s, 3H). | 490.09 77 |
| **53** | 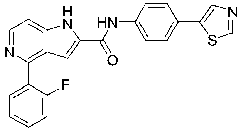 | 1H NMR (400 MHz, DMSO-d6) δ 12.42 (s, 1H), 10.54 (s, 1H), 9.11 (s, 1H), 8.45 (d, J = 5.7 Hz, 1H), 8.34 (s, 1H), 7.95 (d, J = 8.5 Hz, 2H), 7.82 - 7.74 (m, 3H), 7.73 - 7.62 (m, 2H), 7.58 (d, J = 2.6 Hz, 1H), 7.54 (d, J = 6.3 Hz, 1H), 7.50 (q, J = 4.5, 3.4 Hz, 1H). | 415.10 66 |
| **54** | 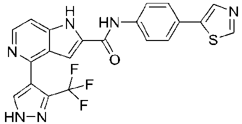 | 1H NMR (400 MHz, 1,4-Dioxane-d8) δ 13.97 (s, 1H), 12.31 (s, 1H), 10.44 (s, 1H), 9.05 (d, J = 0.7 Hz, 1H), 8.50 (s, 1H), 8.33 - 8.26 (m, 2H), 7.88 (d, J = 8.7 Hz, 2H), 7.72 (d, J = 8.6 Hz, 2H), 7.66 (s, 1H), 7.36 (d, J = 5.8 Hz, 1H). | 455.03 31 |
| **55** | 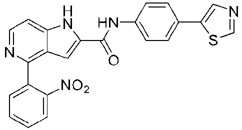 | 1H NMR (400 MHz, DMSO-d6) δ 12.46 (s, 1H), 10.48 (s, 1H), 9.06 (s, 1H), 8.28 (t, J = 2.9 Hz, 2H), 8.10 (d, J = 8.1 Hz, 1H), 7.93 - 7.87 (m, 4H), 7.78 (ddd, J = 8.5, 5.9, 3.0 Hz, 1H), 7.75 - 7.70 (m, 2H), 7.57 (s, 1H), 7.48 - 7.44 (m, 1H). | 442.09 13 |
| **56** | 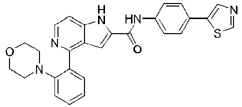 | 1H NMR (400 MHz, DMSO-d6) δ 12.12 (s, 1H), 10.38 (s, 1H), 9.05 (s, 1H), 8.34 (d, J = 5.7 Hz, 1H), 8.27 (s, 1H), 7.92 - 7.85 (m, 2H), 7.74 - 7.67 (m, 2H), 7.54 (d, J = 1.5 Hz, 1H), 7.45 (dd, J = 9.0, 6.6 Hz, 2H), 7.38 (d, J = 5.8 Hz, 1H), 7.23 - 7.12 (m, 2H), 3.16 (d, J = 5.3 Hz, 4H), 2.75 (t, J = 4.5 Hz, 4H). | 482.16 72 |
| **57** | 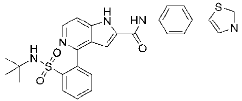 | 1H NMR (400 MHz, DMSO-d6) δ 12.50 (d, J = 2.1 Hz, 1H), 10.46 (s, 1H), 9.06 (s, 1H), 8.34 (d, J = 5.9 Hz, 1H), 8.28 (s, 1H), 8.15 (dd, J = 7.8, 1.3 Hz, 1H), 8.04 (s, 1H), 7.91 - 7.82 (m, 3H), 7.81 - 7.74 (m, 2H), 7.74 - 7.69 (m, 2H), 7.52 (dd, J = 5.8, 0.9 Hz, 1H), 7.47 (t, J = 1.5 Hz, 1H), 1.26 (s, 9H). | 532.14 31 |
| **58** | 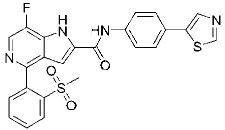 | 1H NMR (400 MHz, DMSO-d6) δ 12.98 (s, 1H), 10.38 (s, 1H), 8.98 (s, 1H), 8.26 (d, J = 2.9 Hz, 1H), 8.20 (s, 1H), 8.11 (dd, J = 7.9, 1.3 Hz, 1H), 7.85 (td, J = 7.5, 1.4 Hz, 1H), 7.82 - 7.72 (m, 3H), 7.64 (td, J = 6.7, 6.2, 1.7 Hz, 3H), 7.32 (d, J = 3.1 Hz, 1H), 3.42 (s, 3H). | 493.07 06 |
| **59** | 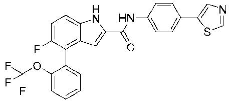 | 1H NMR (400 MHz, DMSO-d6) δ 12.06 (d, J = 2.3 Hz, 1H), 10.34 (s, 1H), 9.05 (s, 1H), 8.27 (s, 1H), 7.91 - 7.84 (m, 2H), 7.72 - 7.65 (m, 4H), 7.64 - 7.58 (m, 2H), 7.56 (dd, J = 8.9, 4.2 Hz, 1H), 7.29 - 7.19 (m, 2H). | 498.08 15 |
| **60** | 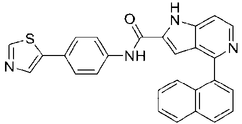 | 1H NMR (400 MHz, DMSO-d6) δ 12.39 (s, 1H), 10.36 (s, 1H), 9.04 (s, 1H), 8.46 (d, J = 5.8 Hz, 1H), 8.26 (s, 1H), 8.10 (dd, J = 6.9, 2.6 Hz, 1H), 8.06 (d, J = 8.3 Hz, 1H), 7.84 (dd, J = 8.5, 3.8 Hz, 3H), 7.75 - 7.71 (m, 2H), 7.68 (dd, J = 9.0, 2.4 Hz, 2H), 7.57 (ddd, J = 8.1, 6.7, 1.3 Hz, 1H), 7.53 (d, J = 5.8 Hz, 1H), 7.47 (ddd, J = 8.2, 6.7, 1.4 Hz, 1H), 7.34 (d, J = 1.8 Hz, 1H). | 465.12 38 |
| **61** | 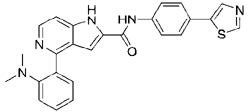 | 1H NMR (400 MHz, DMSO-d6) δ 12.19 - 12.08 (m, 1H), 10.40 (s, 1H), 9.05 (s, 1H), 8.34 (d, J = 5.7 Hz, 1H), 8.27 (s, 1H), 7.92 - 7.85 (m, 2H), 7.73 - 7.66 (m, 2H), 7.42 - 7.35 (m, 3H), 7.33 (dd, J = 7.5, 1.7 Hz, 1H), 7.15 (d, J = 8.1 Hz, 1H), 7.02 (td, J = 7.4, 1.1 Hz, 1H), 2.45 (s, 6H). | 440.15 73 |
| **62** | 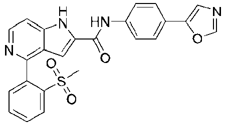 | 1H NMR (400 MHz, DMSO-d6) δ 12.36 (s, 1H), 10.41 (s, 1H), 8.42 (s, 1H), 8.33 (d, J = 5.8 Hz, 1H), 8.18 (dd, J = 8.0, 1.3 Hz, 1H), 7.96 - 7.87 (m, 3H), 7.82 (td, J = 7.7, 1.4 Hz, 1H), 7.73 (dd, J = 8.7, 2.1 Hz, 3H), 7.62 (s, 1H), 7.46 (d, J = 5.8 Hz, 1H), 7.36 (s, 1H), 3.54 (s, 3H). | 459.10 66 |
| **63** | 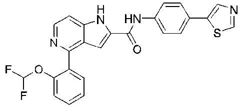 | 1H NMR (400 MHz, DMSO-d6) δ 12.33 (s, 1H), 10.43 (s, 1H), 9.06 (s, 1H), 8.36 (d, J = 5.8 Hz, 1H), 8.28 (s, 1H), 7.91 - 7.85 (m, 2H), 7.75 - 7.68 (m, 2H), 7.68 - 7.58 (m, 2H), 7.51 - 7.38 (m, 4H), 7.14 (t, J = 74.5 Hz, 1H). | 463.10 21 |
| **64** | 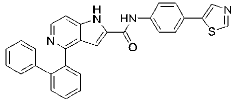 | 1H NMR (400 MHz, DMSO-d6) δ 12.12 (d, J = 2.1 Hz, 1H), 10.32 (s, 1H), 9.05 (s, 1H), 8.27 (s, 1H), 8.18 (d, J = 5.8 Hz, 1H), 7.89 - 7.83 (m, 2H), 7.72 - 7.67 (m, 2H), 7.64 - 7.52 (m, 4H), 7.29 (d, J = 5.8 Hz, 1H), 7.25 (d, J = 2.1 Hz, 1H), 7.09 (d, J = 3.7 Hz, 5H). | 471.14 12 |
| **65** | 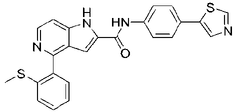 | 1H NMR (400 MHz, DMSO-d6) δ 12.29 (d, J = 1.9 Hz, 1H), 10.43 (s, 1H), 9.05 (s, 1H), 8.33 (d, J = 5.7 Hz, 1H), 8.27 (s, 1H), 7.91 - 7.85 (m, 2H), 7.73 - 7.66 (m, 2H), 7.52 - 7.46 (m, 3H), 7.43 - 7.39 (m, 2H), 7.34 (ddd, J = 8.1, 5.8, 2.5 Hz, 1H), 2.35 (s, 3H). | 443.09 03 |
| **66** | 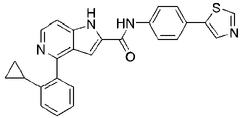 | 1H NMR (400 MHz, DMSO-d6) δ 12.29 (d, J = 2.1 Hz, 1H), 10.43 (s, 1H), 9.06 (s, 1H), 8.37 (d, J = 5.8 Hz, 1H), 8.28 (s, 1H), 7.93 - 7.86 (m, 2H), 7.73 - 7.66 (m, 2H), 7.45 - 7.42 (m, 2H), 7.42 - 7.37 (m, 2H), 7.32 (td, J = 7.2, 1.3 Hz, 1H), 7.09 - 7.02 (m, 1H), 1.94 (tt, J = 8.5, 5.3 Hz, 1H), 0.78 - 0.55 (m, 4H). | 437.14 02 |
| **67** | 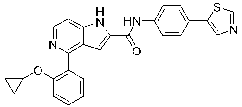 | 1H NMR (400 MHz, DMSO-d6) δ 12.23 - 12.12 (m, 1H), 10.37 (s, 1H), 9.06 (s, 1H), 8.32 (d, J = 5.8 Hz, 1H), 8.28 (s, 1H), 7.92 - 7.87 (m, 2H), 7.74 - 7.68 (m, 2H), 7.54 - 7.47 (m, 2H), 7.47 - 7.42 (m, 1H), 7.39 (d, J = 5.8 Hz, 1H), 7.34 (d, J = 1.9 Hz, 1H), 7.14 (ddd, J = 8.1, 5.8, 2.7 Hz, 1H), 3.83 (dq, J = 6.1, 3.0 Hz, 1H), 0.72 (q, J = 6.5, 5.3 Hz, 2H), 0.62 (d, J = 3.5 Hz, 2H). | 453.13 21 |
| **68** | 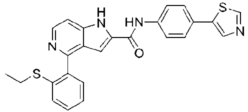 | 1H NMR (400 MHz, DMSO-d6) δ 12.33 (s, 1H), 10.44 (s, 1H), 9.06 (s, 1H), 8.34 (d, J = 5.8 Hz, 1H), 8.28 (s, 1H), 7.92 - 7.86 (m, 2H), 7.73 - 7.68 (m, 2H), 7.56 (dd, J = 8.4, 1.3 Hz, 1H), 7.50 (dtd, J = 6.2, 4.6, 4.0, 1.6 Hz, 2H), 7.46 - 7.40 (m, 2H), 7.36 (td, J = 7.4, 1.3 Hz, 1H), 2.86 (q, J = 7.3 Hz, 2H), 1.11 (t, J = 7.3 Hz, 3H). | 457.11 19 |
| **69** | 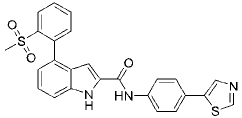 | 1H NMR (400 MHz, DMSO-d6) δ 11.99 (d, J = 2.2 Hz, 1H), 10.27 (s, 1H), 9.05 (s, 1H), 8.27 (s, 1H), 8.20 (dd, J = 8.0, 1.4 Hz, 1H), 7.90 - 7.82 (m, 3H), 7.76 (td, J = 7.7, 1.4 Hz, 1H), 7.71 - 7.64 (m, 2H), 7.55 (td, J = 5.3, 2.8 Hz, 2H), 7.36 - 7.28 (m, 1H), 7.15 - 7.08 (m, 2H), 2.77 (s, 3H). | 474.09 01 |
| **70** | 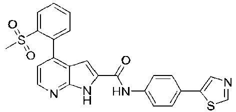 | 1H NMR (400 MHz, DMSO-d6) δ 12.53 (s, 1H), 10.31 (s, 1H), 9.05 (s, 1H), 8.45 (d, J = 4.8 Hz, 1H), 8.28 (s, 1H), 8.21 (dd, J = 7.9, 1.4 Hz, 1H), 7.93 - 7.85 (m, 3H), 7.82 (td, J = 7.7, 1.4 Hz, 1H), 7.72 - 7.65 (m, 2H), 7.56 (dd, J = 7.4, 1.4 Hz, 1H), 7.23 (d, J = 4.8 Hz, 1H), 7.13 (d, J = 2.1 Hz, 1H), 2.98 (s. 3H). | 475.08 90 |
| **71** | 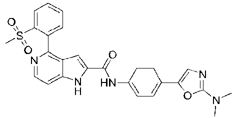 | 1H NMR (400 MHz, DMSO-d6) δ 12.43 - 12.28 (m, 1H), 10.31 (s, 1H), 8.32 (d, J = 5.8 Hz, 1H), 8.18 (dd, J = 8.0, 1.4 Hz, 1H), 7.91 (td, J = 7.5, 1.4 Hz, 1H), 7.81 (dd, J = 8.2, 6.5 Hz, 3H), 7.72 (dd, J = 7.6, 1.4 Hz, 1H), 7.52 (d, J = 8.6 Hz, 2H), 7.45 (d, J = 5.8 Hz, 1H), 7.36 - 7.32 (m, 1H), 7.22 (s, 1H), 3.54 (s, 3H), 3.04 (s, 6H). | 502.15 14 |
| **72** | 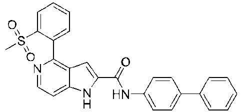 | 1H NMR (400 MHz, DMSO-d6) δ 12.36 (s, 1H), 10.35 (s, 1H), 8.32 (d, J = 5.8 Hz, 1H), 8.18 (dd, J = 7.9, 1.3 Hz, 1H), 7.95 - 7.90 (m, 1H), 7.89 - 7.85 (m, 2H), 7.81 (td, J = 7.7, 1.3 Hz, 1H), 7.76 - 7.71 (m, 1H), 7.70 - 7.63 (m, 4H), 7.49 - 7.42 (m, 3H), 7.38 - 7.30 (m, 2H), 3.54 (s, 3H). | 468.13 39 |
| **73** | 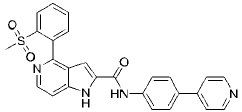 | 1H NMR (400 MHz, DMSO-d6) δ 12.39 (d, J = 2.1 Hz, 1H), 10.44 (s, 1H), 8.62 (s, 2H), 8.33 (d, J = 5.8 Hz, 1H), 8.19 (dd, J = 7.9, 1.3 Hz, 1H), 7.99 - 7.89 (m, 3H), 7.88 - 7.78 (m, 3H), 7.75 - 7.68 (m, 3H), 7.47 (d, J = 5.8 Hz, 1H), 7.38 (s, 1H), 3.55 (s, 3H). | 469.13 02 |
| **74** | 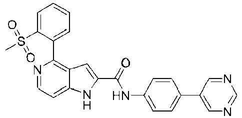 | 1H NMR (400 MHz, DMSO-d6) δ 12.40 (s, 1H), 10.44 (s, 1H), 9.16 (d, J = 2.9 Hz, 3H), 8.33 (d, J = 5.8 Hz, 1H), 8.19 (dd, J = 7.9, 1.3 Hz, 1H), 7.99 - 7.89 (m, 3H), 7.87 - 7.79 (m, 3H), 7.74 (dd, J = 7.6, 1.3 Hz, 1H), 7.47 (d, J = 5.8 Hz, 1H), 7.37 (d, J = 1.7 Hz, 1H), 3.55 (s, 3H). | 470.12 13 |
| **75** | 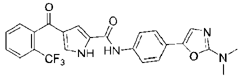 | 1H NMR (400 MHz, DMSO-d6) δ 12.54 (d, J = 3.5 Hz, 1H), 10.10 (s, 1H), 7.92 - 7.86 (m, 1H), 7.83 - 7.72 (m, 4H), 7.65 - 7.58 (m, 1H), 7.53 - 7.47 (m, 3H), 7.30 (dd, J = 3.4, 1.5 Hz, 1H), 3.04 (s, 6H). | 469.14 12 |
| **76** | 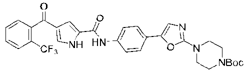 | 1H NMR (400 MHz, DMSO-d6) δ 12.55 (s, 1H), 10.11 (s, 1H), 7.91 7.86 (m, 1H), 7.79 (dq, J = 8.7, 6.9 Hz, 4H), 7.62 (d, J = 7.4 Hz, 1H), 7.57 - 7.51 (m, 2H), 7.49 (d, J = 1.9 Hz, 1H), 7.30 (dd, J = 3.3, 1.6 Hz, 1H), 7.26 (s, 1H), 3.46 (s, 8H), 1.43 (s, 9H). | 610.22 01 |
| **77** | 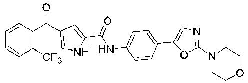 | 1H NMR (400 MHz, DMSO-d6) δ 12.55 (s, 1H), 10.11 (s, 1H), 7.92 - 7.85 (m, 1H), 7.83 - 7.72 (m, 4H), 7.65 - 7.59 (m, 1H), 7.57 - 7.51 (m, 2H), 7.49 (d, J = 1.5 Hz, 1H), 7.30 (d, J = 1.6 Hz, 1H), 7.26 (s, 1H), 3.71 (dd, J = 5.8, 3.8 Hz, 4H), 3.47 (dd, J = 5.7, 3.9 Hz, 4H). | 511.15 31 |
| **78** | 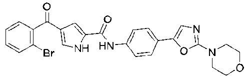 | 1H NMR (400 MHz, DMSO-d6) δ 12.52 (s, 1H), 10.11 (s, 1H), 7.80 - 7.72 (m, 3H), 7.56 - 7.50 (m, 3H), 7.49 - 7.43 (m, 3H), 7.29 (s, 1H), 7.26 (s, 1H), 3.71 (t, J = 4.8 Hz, 4H), 3.46 (dd, J = 5.8, 3.9 Hz, 4H). | 521.07 02 |
| **79** | 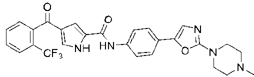 | 1H NMR (400 MHz, DMSO-d6) δ 12.55 (s, 1H), 10.10 (s, 1H), 7.91 - 7.86 (m, 1H), 7.83 - 7.72 (m, 4H), 7.62 (d, J = 7.4 Hz, 1H), 7.55 - 7.47 (m, 3H), 7.30 (d, J = 1.5 Hz, 1H), 7.24 (s, 1H), 3.57 - 3.45 (m, 4H), 2.41 (t, J = 5.1 Hz, 4H), 2.23 (s, 3H). | 524.18 19 |
| **80** | 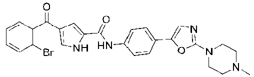 | 1H NMR (400 MHz, DMSO-d6) δ 12.53 (s, 1H), 10.11 (s, 1H), 7.78 (d, J = 2.1 Hz, 1H), 7.77 - 7.72 (m, 2H), 7.56 - 7.50 (m, 3H), 7.47 (dq, J = 7.6, 1.9 Hz, 3H), 7.29 (dd, J = 2.9, 1.5 Hz, 1H), 7.24 (s, 1H), 3.51 3.44 (m, 4H), 2.42 (t, J = 5.1 Hz, 4H), 2.23 (s, 3H). | 534. 1 134 |
| **81** | 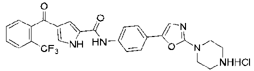 | 1H NMR (400 MHz, DMSO-d6) δ 12.63 (t, J = 2.9 Hz, 1H), 10.26 (s, 1H), 9.56 (s, 2H), 7.92 - 7.87 (m, 1H), 7.86 - 7.78 (m, 3H), 7.75 (t, J = 7.5 Hz, 1H), 7.61 (dd, J = 9.9, 7.7 Hz, 3H), 7.50 (d, J = 3.1 Hz, 2H), 7.31 (dd, J = 3.4, 1.5 Hz, 1H), 3.83 (t, J = 5.2 Hz, 4H), 3.25 (s, 4H), 3.06 (qd, J = 7.3, 4.8 Hz, 1H). | 546.11 21 |
| **82** | 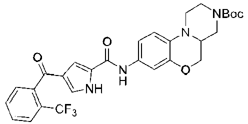 | 1H NMR (400 MHz, DMSO-d6) δ 12.47 (s, 1H), 9.82 (s, 1H), 7.94 - 7.85 (m, 1H), 7.84 - 7.69 (m, 2H), 7.60 (d, J = 7.4 Hz, 1H), 7.41 (d, J = 1.6 Hz, 1H), 7.23 (dd, J = 8.9, 2.0 Hz, 2H), 7.14 (dd, J = 8.8, 2.4 Hz, 1H), 6.87 (d, J = 8.9 Hz, 1H), 4.31 (dd, J = 10.8, 2.7 Hz, 1H), 4.10 - 3.86 (m, 3H), 3.75 (d, J = 11.9 Hz, 1H), 2.92 (ddt, J = 11.4, 8.6, 3.0 Hz, 2H), 2.54 (d, J = 3.4 Hz, 2H), 1.42 (s, 9H). | 571.21 09 |
| **83** | 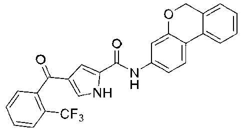 | 1H NMR (400 MHz, DMSO-d6) δ 12.59 (s, 1H), 10.12 (s, 1H), 7.91 - 7.87 (m, 1H), 7.84 (d, J = 8.5 Hz, 1H), 7.81 - 7.73 (m, 3H), 7.63 (d, J = 7.4 Hz, 1H), 7.53 (d, J = 2.2 Hz, 1H), 7.52 (d, J = 1.6 Hz, 1H), 7.43 (dd, J = 8.5, 2.1 Hz, 1H), 7.39 (td, J = 7.4, 1.7 Hz, 1H), 7.30 (d, J = 1.6 Hz, 1H), 7.27 (ddd, J = 10.9, 7.4, 1.5 Hz, 2H), 5.13 (s, 2H). | 463.12 12 |
| **84** | 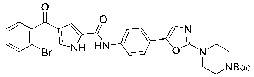 | 1H NMR (400 MHz, DMSO-d6) δ 12.52 (s, 1H), 10.11 (s, 1H), 7.79 (d, J = 1.9 Hz, 1H), 7.77 (d, J = 2.0 Hz, 1H), 7.74 (dd, J = 7.8, 1.3 Hz, 1H), 7.53 (dd, J = 8.0, 6.2 Hz, 3H), 7.50 - 7.43 (m, 3H), 7.29 (d, J = 1.6 Hz, 1H), 7.26 (s, 1H), 3.46 (s, 8H), 1.43 (s, 9H). | 620.14 21 |
| **85** | 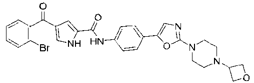 | 1H NMR (400 MHz, DMSO-d6) δ 12.47 (s, 1H), 10.11 (s, 1H), 7.82 - 7.71 (m, 3H), 7.58 - 7.50 (m, 3H), 7.50 - 7.43 (m, 3H), 7.29 (d, J = 1.5 Hz, 1H), 7.24 (s, 1H), 4.56 (t, J = 6.5 Hz, 2H), 4.47 (t, J = 6.0 Hz, 2H), 3.51 (t, J = 5.0 Hz, 4H), 3.49 - 3.44 (m, 1H), 2.37 (t, J = 5.0 Hz, 4H). | 576.12 72 |
| **86** | 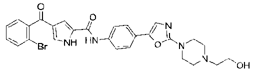 | 1H NMR (400 MHz, DMSO-d6) δ 12.52 (s, 1H), 10.10 (s, 1H), 7.75 (dd, J = 10.5, 8.2 Hz, 3H), 7.51 (dd, J = 8.3, 2.4 Hz, 3H), 7.49 - 7.43 (m, 3H), 7.28 (s, 1H), 7.23 (s, 1H), 4.45 (t, J = 5.4 Hz, 1H), 3.53 (q, J = 6.0 Hz, 2H), 3.47 (t, J = 5.0 Hz, 4H), 2.53 (d, J = 4.9 Hz, 4H), 2.44 (t, J = 6.2 Hz, 2H). | 564.12 01 |
| **87** | 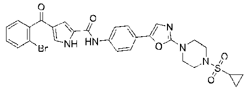 | 1H NMR (400 MHz, DMSO-d6) δ 12.52 (s, 1H), 10.12 (s, 1H), 7.83 - 7.71 (m, 3H), 7.57 - 7.50 (m, 3H), 7.49 - 7.43 (m, 3H), 7.28 (d, J = 6.8 Hz, 2H), 3.61 (t, J = 5.1 Hz, 4H), 3.32 (d, J = 4.2 Hz, 4H), 2.70 - 2.61 (m, 1H), 1.05 - 0.92 (m, 4H). | 624.08 02 |
| **88** | 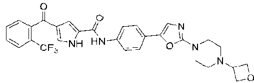 | 1H NMR (400 MHz, DMSO-d6) δ 12.48 (s, 1H), 10.11 (s, 1H), 7.91 - 7.86 (m, 1H), 7.83 - 7.72 (m, 4H), 7.62 (d, J = 7.4 Hz, 1H), 7.55 - 7.51 (m, 2H), 7.49 (d, J = 1.6 Hz, 1H), 7.30 (d, J = 1.5 Hz, 1H), 7.24 (s, 1H), 4.52 (dt, J = 37.0, 6.3 Hz, 4H), 3.51 (t, J = 5.0 Hz, 4H), 3.46 (q, J = 6.3 Hz, 1H), 2.37 (t, J = 5.0 Hz, 4H). | 566.19 11 |
| **89** | 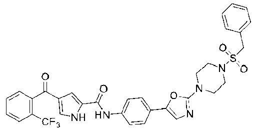 | 1H NMR (400 MHz, DMSO-d6) δ 12.55 (s, 1H), 10.11 (s, 1H), 7.91 - 7.87 (m, 1H), 7.83 - 7.72 (m, 4H), 7.62 (d, J = 7.4 Hz, 1H), 7.57 - 7.52 (m, 2H), 7.49 (d, J = 1.6 Hz, 1H), 7.46 - 7.35 (m, 5H), 7.31 (s, 1H), 7.27 (s, 1H), 4.48 (s, 2H), 3.54 - 3.50 (m, 4H), 3.25 (t, J = 5.1 Hz, 4H). | 664.18 32 |
| **90** | 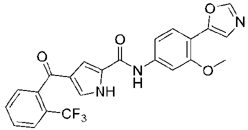 | 1H NMR (400 MHz, DMSO-d6) δ 12.59 (s, 1H), 10.19 (s, 1H), 8.39 (s, 1H), 7.91 - 7.87 (m, 1H), 7.84 - 7.71 (m, 2H), 7.67 (d, J = 8.5 Hz, 1H), 7.63 (q, J = 3.6, 2.8 Hz, 2H), 7.57 (dd, J = 8.5, 1.9 Hz, 1H), 7.52 (d, J = 1.5 Hz, 1H), 7.47 (s, 1H), 7.33 (d, J = 1.6 Hz, 1H), 3.94 (s, 3H). | 456.11 02 |
| **91** | 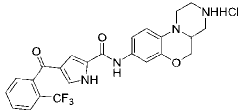 | 1H NMR (400 MHz, DMSO-d6) δ 12.52 (s, 1H), 9.91 (s, 1H), 9.42 (s, 1H), 9.37 (s, 1H), 7.90 - 7.86 (m, 1H), 7.82 - 7.72 (m, 2H), 7.60 (d, J = 7.4 Hz, 1H), 7.42 (t, J = 2.0 Hz, 1H), 7.28 (d, J = 2.4 Hz, 1H), 7.25 (dd, J = 3.4, 1.6 Hz, 1H), 7.20 (dd, J = 8.8, 2.4 Hz, 1H), 6.91 (d, J = 9.0 Hz, 1H), 4.30 (dd, J = 10.8, 2.5 Hz, 1H), 4.03 - 3.90 (m, 2H), 3.35 (d, J = 14.7 Hz, 3H), 3.12 - 2.68 (m, 3H). | 507.13 23 |
| **92** | 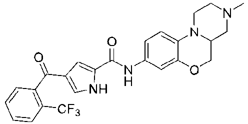 | 1H NMR (400 MHz, DMSO-d6) δ 12.51 (d, J = 3.9 Hz, 1H), 9.87 (s, 1H), 7.88 (d, J = 7.5 Hz, 1H), 7.77 (dq, J = 15.0, 7.4 Hz, 2H), 7.60 (d, J = 7.4 Hz, 1H), 7.42 (t, J = 2.1 Hz, 1H), 7.25 (t, J = 2.4 Hz, 2H), 7.17 (dd, J = 8.8, 2.5 Hz, 1H), 6.87 (d, J = 8.9 Hz, 1H), 4.26 (dd, J = 10.8, 2.7 Hz, 1H), 3.93 (dd, J = 10.8, 8.3 Hz, 1H), 3.81 (d, J = 12.3 Hz, 1H), 3.23 (s, 2H), 3.12 (s, 2H), 2.79 (d, J = 11.6 Hz, 1H), 2.48 (s, 3H), 2.21 (s, 1H). | 485.17 04 |
| **93** | 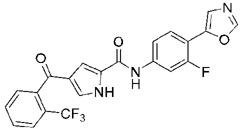 | 1H NMR (400 MHz, DMSO-d6) δ 12.76 - 12.43 (m, 1H), 10.37 (s, 1H), 8.52 (s, 1H), 7.92 (dd, J = 15.4, 4.3 Hz, 2H), 7.79 (dq, J = 14.7, 7.1 Hz, 3H), 7.70 - 7.61 (m, 2H), 7.55 - 7.52 (m, 1H), 7.48 (d, J = 3.4 Hz, 1H), 7.38 - 7.32 (m, 1H). | 444.09 31 |
| **94** | 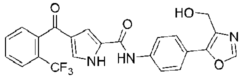 | 1H NMR (400 MHz, DMSO-d6) δ 12.55 (s, 1H), 10.23 (s, 1H), 8.35 (s, 1H), 7.90 (dd, J = 8.2, 3.4 Hz, 3H), 7.84 - 7.69 (m, 4H), 7.63 (d, J = 7.4 Hz, 1H), 7.53 (d, J = 1.6 Hz, 1H), 7.32 (d, J = 1.5 Hz, 1H), 5.31 (t, J = 5.4 Hz, 1H), 4.54 (d, J = 4.4 Hz, 2H). | 456. 11 20 |
| **95** | 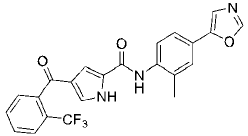 | 1H NMR (400 MHz, DMSO-d6) δ 12.54 (s, 1H), 9.80 (s, 1H), 8.44 (s, 1H), 7.91 - 7.86 (m, 1H), 7.83 - 7.72 (m, 2H), 7.66 (d, J = 2.9 Hz, 2H), 7.64 - 7.56 (m, 2H), 7.49 - 7.42 (m, 2H), 7.26 (d, J = 1.6 Hz, 1H), 2.30 (s, 3H). | 440.11 92 |
| **96** | 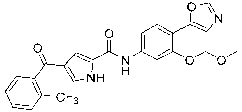 | 1H NMR (400 MHz, DMSO-d6) δ 12.57 (s, 1H), 10.22 (s, 1H), 8.42 (s, 1H), 7.91 - 7.87 (m, 1H), 7.81 (td, J = 7.6, 1.4 Hz, 1H), 7.78 - 7.75 (m, 1H), 7.73 (d, J = 1.9 Hz, 1H), 7.70 (d, J = 8.6 Hz, 1H), 7.63 (dt, J = 8.0, 1.8 Hz, 2H), 7.53 (d, J = 3.8 Hz, 2H), 7.33 (d, J = 1.6 Hz, 1H), 5.38 (s, 2H), 3.44 (s, 3H). | 486.12 65 |
| **97** | 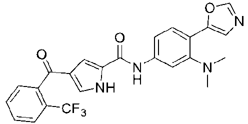 | 1H NMR (400 MHz, DMSO-d6) δ 12.53 (s, 1H), 10.15 (s, 1H), 8.39 (s, 1H), 7.89 (d, J = 7.7 Hz, 1H), 7.83 - 7.73 (m, 2H), 7.67 (s, 1H), 7.65 - 7.61 (m, 3H), 7.53 (s, 1H), 7.52 (d, J = 1.5 Hz, 1H), 7.33 (d, J = 1.6 Hz, 1H), 2.65 (s, 6H). | 468.14 22 |
| **98** | 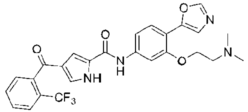 | 1H NMR (400 MHz, DMSO-d6) δ 12.58 (s, 1H), 10.16 (s, 1H), 8.39 (s, 1H), 7.92 - 7.86 (m, 1H), 7.84 - 7.70 (m, 2H), 7.67 (d, J = 8.5 Hz, 1H), 7.63 (q, J = 3.9, 3.0 Hz, 2H), 7.59 (s, 1H), 7.54 (dd, J = 8.5, 1.9 Hz, 1H), 7.51 (d, J = 1.5 Hz, 1H), 7.33 (s, 1H), 4.18 (t, J = 5.7 Hz, 2H), 2.78 (t, J = 5.7 Hz, 2H), 2.27 (s, 6H). | 513.17 79 |
| **99** | 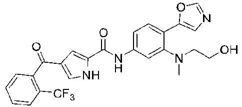 | 1H NMR (400 MHz, DMSO-d6) δ 12.36 (s, 1H), 10.14 (s, 1 H), 8.37 (s, 1H), 7.89 (d, J = 7.7 Hz, 1H), 7.83 7.73 (m, 2H), 7.70 7.61 (m, 5H), 7.50 (d, J = 1.6 Hz, 1H), 7.32 (d, J = 1.7 Hz, 1H), 4.58 (t, J = 5.3 Hz, 1H), 3.55 (q, J = 5.7 Hz, 2H), 3.00 (t, J = 6.3 Hz, 2H), 2.64 (s, 3H). | 499.15 43 |
| **100** | 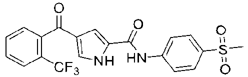 | 1H NMR (400 MHz, DMSO-d6) δ 12.66 (s, 1H), 10.42 (s, 1H), 8.01 (d, J = 8.9 Hz, 2H), 7.90 (dd, J = 8.4, 4.0 Hz, 3H), 7.78 (dq, J = 15.1, 7.5 Hz, 2H), 7.63 (d, J = 7.4 Hz, 1H), 7.57 (s, 1H), 7.36 (s, 1H), 3.19 (s, 3H). | 437.07 01 |
| **101** | 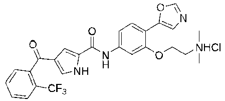 | 1H NMR (400 MHz, DMSO-d6) δ 12.74 (t, J = 2.9 Hz, 1H), 11.27 (d, J = 8.8 Hz, 1H), 10.46 (s, 1H), 8.44 (s, 1H), 7.91 - 7.87 (m, 1H), 7.85 - 7.73 (m, 3H), 7.72 - 7.61 (m, 4H), 7.54 (t, J = 2.0 Hz, 1H), 7.34 (dd, J = 3.4, 1.5 Hz, 1H), 4.56 (t, J = 4.8 Hz, 2H), 3.74 (q, J = 4.9 Hz, 2H), 2.86 (d, J = 4.8 Hz, 6H). | 549.14 13 |
| **102** | 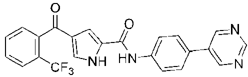 | 1H NMR (400 MHz, DMSO-d6) δ 12.63 (s, 1H), 10.24 (s, 1H), 9.16 (dd, J = 3.1, 0.9 Hz, 3H), 7.94 (d, J = 8.5 Hz, 2H), 7.90 (d, J = 7.8 Hz, 1H), 7.86 - 7.73 (m, 4H), 7.64 (d, J = 7.4 Hz, 1H), 7.55 (s, 1H), 7.33 (s, 1H). | 437.11 02 |
| **103** | 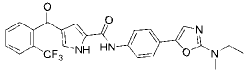 | 1H NMR (400 MHz, DMSO-d6) δ 12.54 (s, 1H), 10.10 (s, 1H), 7.89 (d, J = 7.7 Hz, 1H), 7.84 - 7.72 (m, 4H), 7.62 (d, J = 7.4 Hz, 1H), 7.53 - 7.44 (m, 3H), 7.30 (s, 1H), 7.20 (s, 1H), 3.47 (q, J = 7.1 Hz, 2H), 3.03 (s, 3H), 1.15 (t, J = 7.1 Hz, 3H). | 483.16 19 |
| **104** | 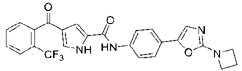 | 1H NMR (400 MHz, DMSO-d6) δ 12.54 (s, 1H), 10.11 (s, 1H), 7.89 (d, J = 7.7 Hz, 1H), 7.84 - 7.71 (m, 4H), 7.62 (d, J = 7.4 Hz, 1H), 7.53 - 7.44 (m, 3H), 7.31 (dd, J = 3.5, 1.5 Hz, 1H), 7.22 (s, 1H), 4.09 (t, J = 7.6 Hz, 4H), 2.38 (p, J = 7.5 Hz, 2H). | 481.14 11 |
| **105** | 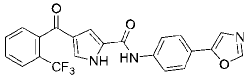 | 1H NMR (400 MHz, DMSO-d6) δ 12.58 (s, 1H), 10.20 (s, 1H), 8.41 (s, 1H), 7.92 - 7.85 (m, 3H), 7.84 - 7.69 (m, 4H), 7.62 (d, J = 11.5 Hz, 2H), 7.52 (t, J = 2.0 Hz, 1H), 7.32 (dd, J = 3.5, 1.5 Hz, 1H). | 426.10 04 |
| **106** | 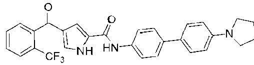 | 1H NMR (400 MHz, DMSO-d6) δ 12.58 (t, J = 3.0 Hz, 1H), 10.12 (s, 1H), 7.93 - 7.87 (m, 1H), 7.83 (d, J = 2.1 Hz, 1H), 7.80 (d, J = 7.7 Hz, 2H), 7.76 (d, J = 7.6 Hz, 1H), 7.66 - 7.59 (m, 3H), 7.52 (t, J = 2.0 Hz, 1H), 7.30 (dd, J = 3.4, 1.6 Hz, 1H), 7.22 (t, J = 7.9 Hz, 1H), 6.89 - 6.82 (m, 1H), 6.74 (t, J = 2.1 Hz, 1H), 6.51 (dd, J = 8.2, 2.3 Hz, 1H), 3.29 (q, J = 6.3, 4.9 Hz, 4H), 2.04 - 1.91 (m, 4H). | 504.53 11 |
| **107** | 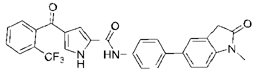 | 1H NMR (400 MHz, DMSO-d6) δ 12.56 (s, 1H), 10.11 (s, 1H), 7.92 - 7.87 (m, 1H), 7.82 (d, J = 2.0 Hz, 1H), 7.82 - 7.72 (m, 3H), 7.66 - 7.55 (m, 5H), 7.52 (d, J = 1.6 Hz, 1H), 7.30 (d, J = 1.6 Hz, 1H), 7.08 - 7.01 (m, 1H), 3.61 (s, 2H), 3.15 (s, 3H). | 504.48 23 |
| **108** | 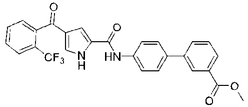 | 1H NMR (400 MHz, DMSO-d6) δ 12.57 (s, 1H), 10. 17 (s, 1H), 8.19 (t, J = 1.9 Hz, 1H), 7.94 (ddt, J = 12.6, 7.8, 1.2 Hz, 2H), 7.88 (dd, J = 8.8, 2.2 Hz, 3H), 7.83 - 7.73 (m, 2H), 7.73 - 7.68 (m, 2H), 7.65 - 7.57 (m, 2H), 7.52 (d, J = 2.1 Hz, 1H), 7.30 (d, J = 2.7 Hz, 1H), 3.89 (s, 3H). | 493.45 12 |
| **109** | 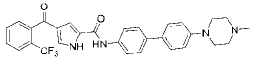 | 1H NMR (400 MHz, DMSO-d6) δ 12.57 (s, 1H), 10.09 (s, 1H), 7.89 (d, J = 7.7 Hz, 1H), 7.84 - 7.71 (m, 4H), 7.61 (dd, J = 15.2, 8.0 Hz, 3H), 7.53 (d, J = 8.8 Hz, 3H), 7.34 - 7.25 (m, 1H), 7.05 - 6.95 (m, 2H), 3.24 - 3.12 (m, 4H), 2.46 (t, J = 5.0 Hz, 4H), 2.22 (s, 3H). | 533.57 42 |
| **110** | 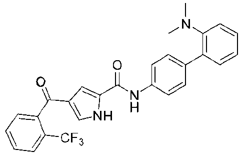 | 1H NMR (400 MHz, DMSO-d6) δ 12.56 (s, 1H), 10.10 (s, 1H), 7.89 (dd, J = 7.8, 1.4 Hz, 1H), 7.84 - 7.71 (m, 4H), 7.63 (d, J = 7.4 Hz, 1H), 7.55 - 7.48 (m, 3H), 7.29 (s, 1H), 7.25 (ddd, J = 8.4, 7.3, 1.7 Hz, 1H), 7.17 (dd, J = 7.6, 1.7 Hz, 1H), 7.06 (dd, J = 8.2, 1.2 Hz, 1H), 7.00 (td, J = 7.4, 1.2 Hz, 1H), 2.49 (s, 6H). | 478.49 01 |
| **111** | 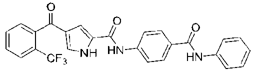 | 1H NMR (400 MHz, DMSO-d6) δ 12.62 (s, 1H), 10.29 (s, 1H), 10.14 (s, 1H), 8.01 - 7.95 (m, 2H), 7.90 (dd, J = 8.8, 1.9 Hz, 3H), 7.84 - 7.72 (m, 4H), 7.63 (d, J = 7.4 Hz, 1H), 7.55 (d, J = 1.6 Hz, 1H), 7.39 - 7.31 (m, 3H), 7.13 - 7.06 (m, 1H). | 478.44 10 |
| **112** | 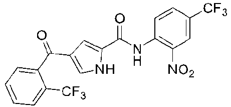 | 1H NMR (400 MHz, DMSO-d6) δ 12.81 (s, 1H), 10.86 (s, 1H), 8.32 (d, J = 2.2 Hz, 1H), 8.13 (dd, J = 8.6, 2.2 Hz, 1H), 7.91 (t, J = 8.7 Hz, 2H), 7.79 (dq, J = 15.2, 7.5 Hz, 2H), 7.64 (d, J = 7.4 Hz, 1H), 7.46 (t, J = 2.0 Hz, 1H), 7.40 (dd, J = 3.5, 1.5 Hz, 1H). | 472.32 33 |
| **113** | 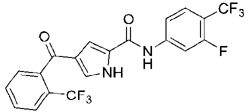 | 1H NMR (400 MHz, DMSO-d6) δ 12.70 (s, 1H), 10.50 (s, 1H), 8.00 - 7.94 (m, 1H), 7.92 - 7.87 (m, 1H), 7.84 - 7.73 (m, 3H), 7.72 - 7.68 (m, 1H), 7.65 - 7.60 (m, 1H), 7.55 (d, J = 1.7 Hz, 1H), 7.37 (dd, J = 2.9, 1.5 Hz, 1H). | 445.31 09 |
| **114** | 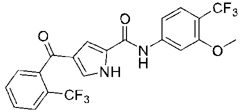 | 1H NMR (400 MHz, DMSO-d6) δ 12.63 (s, 1H), 10.30 (s, 1H), 7.92 - 7.87 (m, 1H), 7.84 - 7.73 (m, 2H), 7.67 (s, 1H), 7.63 (d, J = 7.4 Hz, 1H), 7.60 - 7.51 (m, 3H), 7.36 (s, 1H), 3.88 (s, 3H). | 457. 3 410 |
| **115** | 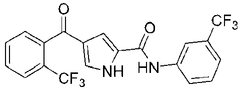 | 1H NMR (400 MHz, DMSO-d6) δ 12.62 (s, 1H), 10.32 (s, 1H), 8.18 (t, J = 2.0 Hz, 1H), 8.07 8.01 (m, 1H), 7.93 - 7.86 (m, 1H), 7.84 - 7.72 (m, 2H), 7.66 - 7.56 (m, 2H), 7.52 (dd, J = 2.5, 1.6 Hz, 1H), 7.46 - 7.41 (m, 1H), 7.35 (dd, J = 3.4, 1.6 Hz, 1H). | 427.32 31 |
| **116** | 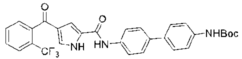 | 1H NMR (400 MHz, DMSO-d6) δ 12.60 - 12.52 (m, 1H), 10.11 (s, 1H), 9.42 (s, 1H), 7.92 - 7.86 (m, 1H), 7.84 - 7.72 (m, 4H), 7.66 - 7.59 (m, 3H), 7.56 (t, J = 8.4 Hz, 3H), 7.53 - 7.49 (m, 2H), 7.30 (dd, J = 3.4, 1.6 Hz, 1H), 1.49 (s, 9H). | 550.55 51 |
| **117** | 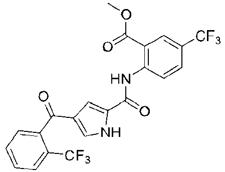 | 1H NMR (400 MHz, DMSO-d6) δ 12.84 (s, 1H), 11.49 (s, 1H), 8.59 (d, J = 8.7 Hz, 1H), 8.20 (d, J = 2.3 Hz, 1H), 8.04 (dd, J = 8.9, 2.3 Hz, 1H), 7.92 - 7.87 (m, 1H), 7.84 - 7.73 (m, 2H), 7.64 (d, J = 7.3 Hz, 1H), 7.37 - 7.27 (m, 2H), 3.93 (s, 3H). | 485.35 18 |
| **118** | 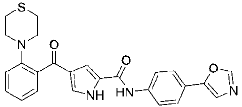 | 1H NMR (400 MHz, DMSO-d6) δ 12.41 (d, J = 3.2 Hz, 1H), 10.21 (s, 1H), 8.47 (s, 1H), 7.97 - 7.91 (m, 2H), 7.81 - 7.73 (m, 2H), 7.66 (s, 1H), 7.59 (t, J = 2.0 Hz, 1H), 7.54 - 7.48 (m, 1H), 7.39 - 7.33 (m, 2H), 7.24 - 7.13 (m, 2H), 3.28 - 3.19 (m, 4H), 2.48 - 2.40 (m, 4H). | 459.45 54 |
| 119 | 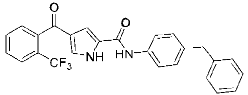 | ¹H NMR (400 MHz, DMSO-*d*₆) δ 12.52 (s, 1H), 9.99 (s, 1H), 7.93 - 7.84 (m, 1H), 7.83 - 7.70 (m, 2H), 7.67 - 7.55 (m, 3H), 7.46 (t, *J* = 2.0 Hz, 1H), 7.34 - 7.26 (m, 3H), 7.26 - 7.16 (m, 5H), 3.90 (s, 2H). | 449.45 02 |
| **120** | 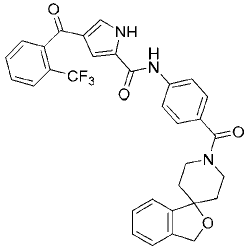 | ¹H NMR (400 MHz, DMSO-*d*₆) δ 12.60 (d, *J* = 3.3 Hz, 1H), 10.20 (s, 1H), 7.93 - 7.85 (m, 1H), 7.85 - 7.78 (m, 3H), 7.75 (t, *J* = 7.6 Hz, 1H), 7.62 (d, *J* = 7.4 Hz, 1H), 7.53 (t, *J* = 2.0 Hz, 1H), 7.51 - 7.45 (m, 2H), 7.36 (dq, *J* = 5.1, 2.1 Hz, 1H), 7.30 (s, 4H), 4.50 (s, 1H), 3.69 (s, 1H), 3.33 - 2.89 (m, 3H), 1.95 (td, *J* = 13.2, 4.8 Hz, 2H), 1.66 (s, 2H). | 574.57 17 |
| **121** | 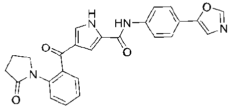 | ¹H NMR (400 MHz, DMSO-*d*₆) δ 11.86 (d, *J* = 9.6 Hz, 1H), 10.85 (s, 1H), 8.30 (dd, *J* = 9.5, 1.5 Hz, 1H), 8.25 (s, 1H), 7.83 - 7.76 (m, 3H), 7.76 - 7.70 (m, 2H), 7.63 (td, *J* = 7.6, 1.6 Hz, 1H), 7.52 - 7.46 (m, 2H), 7.32 (td, *J =* 7.4, 1.5 Hz, 1H), 7.07 (d, *J =* 1.4 Hz, 1H), 3.85 (t, *J* = 7.1 Hz, 2H), 2.53 - 2.46 (m, 2H), 2.07 (q, *J* = 7.1 Hz, 2H). | 441.46 02 |
| **122** | 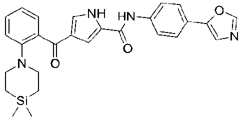 | ¹H NMR (400 MHz, DMSO-*d*₆) δ 11.80 (d, *J* = 9.6 Hz, 1H), 10.85 (s, 1H), 8.30 (dd, *J* = 9.5, 1.5 Hz, 1H), 8.25 (s, 1H), 7.83 - 7.76 (m, 2H), 7.76 - 7.69 (m, 3H), 7.50 (s, 1H), 7.40 (ddd, *J* = 21.3, 7.4, 1.6 Hz, 2H), 7.12 - 7.05 (m, 2H), 3.33 (t, *J* = 7.1 Hz, 4H), 1.08 - 1.01 (m, 4H), -0.15 (s, 6H). | 485.63 91 |
| **123** | 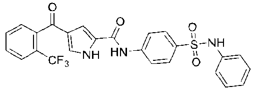 | ¹H NMR (400 MHz, DMSO-*d₆*) δ 12.61 (s, 1H), 10.32 (s, 1H), 10.20 (s, 1H), 7.91 - 7.85 (m, 3H), 7.83 - 7.67 (m, 4H), 7.61 (d, *J =* 7.3 Hz, 1H), 7.51 (d, *J =* 1.6 Hz, 1H), 7.32 (d, *J* = 1.5 Hz, 1H), 7.27 - 7.19 (m, 2H), 7.14 - 7.07 (m, 2H), 7.02 (td, *J* = 7.3, 1.2 Hz, 1H). | 514.49 32 |
| **124** | 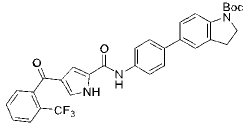 | ¹H NMR (400 MHz, DMSO-*d₆*) δ 12.56 (s, 1H), 10.11 (s, 1H), 7.93 - 7.84 (m, 1H), 7.84 - 7.78 (m, 3H), 7.75 (t, *J* = 7.6 Hz, 1H), 7.66 - 7.57 (m, 4H), 7.55 - 7.40 (m, 3H), 7.29 (d, *J* = 1.6 Hz, 1H), 3.94 (t, *J* = 8.6 Hz, 2H), 3.12 (t, *J* = 8.6 Hz, 2H), 1.52 (s, 9H). | 576.59 02 |
| **125** | 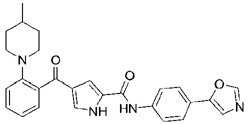 | ¹H NMR (400 MHz, DMSO-*d*₆) δ 12.29 (s, 1H), 10.15 (s, 1H), 8.41 (s, 1H), 7.91 - 7.85 (m, 2H), 7.74 - 7.68 (m, 2H), 7.60 (s, 1H), 7.54 (t, *J* = 1.9 Hz, 1H), 7.41 (ddd, *J* = 8.7, 7.3, 1.7 Hz, 1H), 7.32 (dd, *J* = 3.4, 1.5 Hz, 1H), 7.26 (dd, *J* = 7.5, 1.7 Hz, 1H), 7.10 (d, *J* = 8.1 Hz, 1H), 7.05 - 6.96 (m, 1H), 3.19 (d, *J* = 11.7 Hz, 2H), 2.63 (dd, *J* = 12.9, 10.5 Hz, 2H), 1.47 (d, *J =* 12.4 Hz, 2H), 1.37 - 1.15 (m, 2H), 0.87 - 0.80 (m, 1H), 0.78 (d, *J* = 6.6 Hz, 3H). | 455.53 10 |
| **126** | 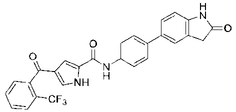 | ¹H NMR (400 MHz, DMSO-*d*₆) δ 12.55 (s, 1H), 10.44 (s, 1H), 10.10 (s, 1H), 7.92 - 7.85 (m, 1H), 7.84 - 7.71 (m, 4H), 7.65 - 7.54 (m, 3H), 7.54 - 7.41 (m, 3H), 7.29 (d, *J* = 1.6 Hz, 1H), 6.89 (d, *J* = 8.1 Hz, 1H), 3.53 (s, 2H). | 490.45 14 |
| **127** | 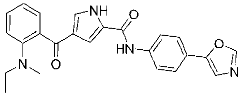 | ¹H NMR (400 MHz, DMSO-*d*₆) δ 12.34 (s, 1H), 10.16 (s, 1H), 8.41 (s, 1H), 7.88 (d, *J* = 8.7 Hz, 2H), 7.71 (d, *J* = 8.7 Hz, 2H), 7.60 (s, 1H), 7.55 (t, *J =* 2.0 Hz, 1H), 7.42 - 7.34 (m, 1H), 7.28 (dd, *J* = 3.4, 1.5 Hz, 1H), 7.23 (dd, *J* = 7.5, 1.7 Hz, 1H), 7.06 (d, *J* = 8.3 Hz, 1H), 6.92 (t, *J* = 7.4 Hz, 1H), 3.03 (q,*J* = 7.0 Hz, 2H), 2.65 (s, 3H), 0.90 (t, J= 7.0 Hz, 3H). | 415.47 30 |
| **128** | 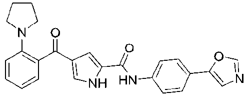 | ¹H NMR (400 MHz, DMSO-*d*₆) δ 12.40 (s, 1H), 10.15 (s, 1H), 8.41 (s, 1H), 7.91 - 7.85 (m, 2H), 7.74 - 7.68 (m, 2H), 7.60 (s, 1H), 7.58 (t, *J =* 2.0 Hz, 1H), 7.39 (dd, *J* = 3.3, 1.5 Hz, 1H), 7.35 - 7.27 (m, 2H), 6.81 (d, *J* = 8.3 Hz, 1H), 6.71 (t, *J* = 7.3 Hz, 1H), 3.16 - 2.94 (m, 4H), 1.95 - 1.69 (m, 4H). | 427.48 43 |
| **129** | 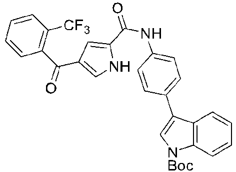 | ¹H NMR (400 MHz, DMSO-*d*₆) δ 12.53 (s, 1H), 10.05 (s, 1H), 7.92 - 7.86 (m, 1H), 7.84 - 7.71 (m, 3H), 7.71 - 7.64 (m, 2H), 7.61 (d, *J* = 7.4 Hz, 1H), 7.48 (d, *J* = 2.1 Hz, 1H), 7.28 (d, *J* = 2.4 Hz, 1H), 7.23 - 7.19 (m, 1H), 7.17 (dd, *J* = 9.0, 2.4 Hz, 3H), 6.93 (d, *J* = 4.2 Hz, 2H), 1.52 (s, 9H). | 576.59 02 |
| **130** | 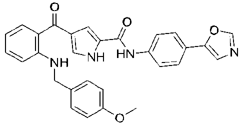 | ¹H NMR (400 MHz, DMSO-*d*₆) δ 12.42 (s, 1H), 10.18 (s, 1H), 8.42 (s, 1H), 8.19 (t, *J* = 5.8 Hz, 1H), 7.95 - 7.85 (m, 2H), 7.77 (dd, *J* = 7.9, 1.6 Hz, 1H), 7.74 - 7.67 (m, 2H), 7.61 (s, 1H), 7.58 (d, *J =* 1.5 Hz, 1H), 7.45 (s, 1H), 7.35 (ddd, *J* = 8.6, 7.1, 1.6 Hz, 1H), 7.32 - 7.26 (m, 2H), 6.96 - 6.88 (m, 2H), 6.77 (d, *J* = 8.5 Hz, 1H), 6.71 - 6.63 (m, 1H), 4.37 (d, *J =* 5.7 Hz, 2H), 3.74 (s, 3H). | 493.54 54 |
| **131** | 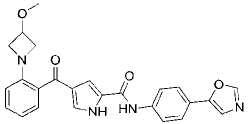 | ¹H NMR (400 MHz, DMSO-*d*₆) δ 11.86 (d, *J* = 9.6 Hz, 1H), 10.85 (s, 1H), 8.33 - 8.23 (m, 2H), 7.83 - 7.70 (m, 4H), 7.60 (d, *J* = 8.9 Hz, 1H), 7.50 (s, 1H), 7.43 - 7.36 (m, 2H), 7.12 - 7.05 (m, 2H), 3.97 - 3.82 (m, 4H), 3.21 (d, *J* = 1.6 Hz, 3H). | 443.48 10 |
| **132** | 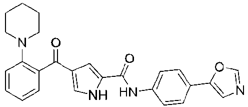 | ¹H NMR (400 MHz, DMSO-*d*₆) δ 12.29 (s, 1H), 10.15 (s, 1H), 8.41 (s, 1H), 7.95 - 7.81 (m, 2H), 7.76 - 7.65 (m, 2H), 7.64 - 7.52 (m, 2H), 7.42 (ddd, *J* = 8.4, 7.4, 1.8 Hz, 1H), 7.36 - 7.19 (m, 2H), 7.14 - 6.85 (m, 2H), 2.90 (t, *J* = 4.9 Hz, 4H), 1.40 - 1.21 (m, 6H). | 441.50 05 |
| **133** | 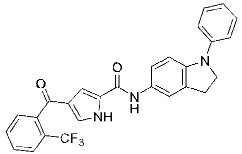 | ¹H NMR (400 MHz, DMSO-*d*₆) δ 12.23 (d, *J =* 9.6 Hz, 1H), 10.19 (s, 1H), 8.31 (dd, *J* = 9.5, 1.5 Hz, 1H), 8.00 (dd, *J* = 7.4, 1.6 Hz, 1H), 7.92 (dd, *J* = 7.6, 1.6 Hz, 1H), 7.71 (td, *J* = 7.4, 1.4 Hz, 1H), 7.50 (td, *J* = 7.6, 1.6 Hz, 1H), 7.42 (dd, *J* = 7.5, 1.5 Hz, 1H), 7.39 - 7.30 (m, 3H), 7.22 - 7.15 (m, 2H), 7.14 - 7.04 (m, 3H), 4.12 - 4.04 (m, 1H), 4.02 (d, *J =* 7.0 Hz, 1H), 3.13 (td, *J* = 7.1, 1.1 Hz, 2H). | 476.47 21 |
| **134** | 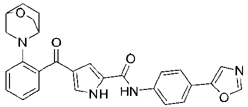 | ¹H NMR (400 MHz, DMSO-*d*₆) δ 12.37 (s, 1H), 10.17 (s, 1H), 8.41 (s, 1H), 7.92 - 7.82 (m, 2H), 7.74 - 7.66 (m, 2H), 7.63 - 7.53 (m, 2H), 7.42 - 7.31 (m, 2H), 7.25 (dd, *J* = 7.6, 1.7 Hz, 1H), 7.03 (d, *J =* 8.3 Hz, 1H), 6.94 (t, *J* = 7.4 Hz, 1H), 3.74 (s, 2H), 3.37 (d, *J* = 10.1 Hz, 2H), 3.27 (d, *J* = 10.3 Hz, 2H), 1.85 - 1.46 (m, 4H). | 469.51 12 |
| **135** | 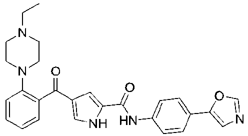 | ¹H NMR (400 MHz, DMSO-*d*₆) δ 12.33 (s, 1H), 10.15 (s, 1H), 8.42 (s, 1H), 7.99 - 7.84 (m, 2H), 7.74 - 7.67 (m, 2H), 7.60 (s, 1H), 7.54 (s, 1H), 7.47 -7.40 (m, 1H), 7.35 - 7.24 (m, 2H), 7.12 (d, *J* = 8.2 Hz, 1H), 7.06 (t, *J* = 7.4 Hz, 1H), 2.95 (t, *J* = 4.8 Hz, 4H), 2.21 (d, *J* = 13.9 Hz, 6H), 0.92 (t, *J* = 7.1 Hz, 3H). | 470.55 10 |
| **136** | 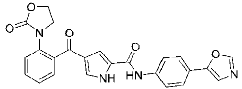 | ¹H NMR (400 MHz, DMSO-*d*₆) δ 12.44 (s, 1H), 10.18 (s, 1H), 8.41 (s, 1H), 7.92 - 7.84 (m, 2H), 7.73 - 7.67 (m, 2H), 7.65 - 7.57 (m, 3H), 7.56 - 7.49 (m, 2H), 7.45 (td, *J* = 7.5, 1.3 Hz, 1H), 7.37 (dd, *J* = 3.3, 1.5 Hz, 1H), 4.32 (dd, *J* = 8.8, 6.9 Hz, 2H), 4.02 (dd, *J* = 8.9, 6.8 Hz, 2H). | 443.43 12 |
| **137** | 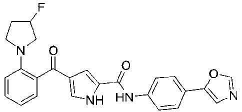 | ¹H NMR (400 MHz, DMSO-*d*₆) δ 12.44 (s, 1H), 10.16 (s, 1H), 8.41 (s, 1H), 7.91 - 7.86 (m, 2H), 7.74 - 7.68 (m, 2H), 7.61 - 7.57 (m, 2H), 7.42 (dd, *J =* 3.3, 1.6 Hz, 1H), 7.39 - 7.26 (m, 2H), 6.85 (d, *J* = 8.3 Hz, 1H), 6.81 - 6.71 (m, 1H), 5.33 (d, *J* = 53.7 Hz, 1H), 3.49 (ddd, *J* = 39.8, 12.5, 3.5 Hz, 1H), 3.40 - 3.35 (m, 1H), 3.20 (t, *J* = 9.0 Hz, 1H), 3.04 (dd, *J* = 24.7, 12.5 Hz, 1H), 2.28 - 1.91 (m, 2H). | 445.47 94 |
| **138** | 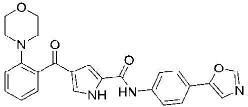 | ¹H NMR (400 MHz, DMSO-*d*₆) δ 12.36 (s, 1H), 10.16 (s, 1H), 8.41 (s, 1H), 7.89 (d, *J* = 8.4 Hz, 2H), 7.71 (d, *J* = 8.4 Hz, 2H), 7.58 (d, *J* = 18.1 Hz, 2H), 7.46 (t, *J =* 7.7 Hz, 1H), 7.37 - 7.26 (m, 2H), 7.18 - 7.05 (m, 2H), 3.40 (t, *J* = 4.5 Hz, 4H), 2.93 (t, *J =* 4.5 Hz, 4H). | 443.48 73 |
| **139** | 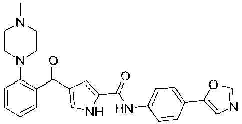 | ¹H NMR (400 MHz, DMSO-*d*₆) δ 12.33 (s, 1H), 10.15 (s, 1H), 8.41 (s, 1H), 7.94 - 7.82 (m, 2H), 7.76 - 7.67 (m, 2H), 7.60 (s, 1H), 7.54 (t, *J* = 2.0 Hz, 1H), 7.44 (td, *J* = 7.7, 1.7 Hz, 1H), 7.33 - 7.21 (m, 2H), 7.12 (d, *J* = 8.2 Hz, 1H), 7.06 (t, *J* = 7.4 Hz, 1H), 2.94 (t, *J* = 4.8 Hz, 4H), 2.19 - 2.12 (m, 4H), 2.07 (s, 3H). | 456.52 39 |
| **140** | 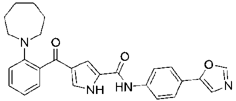 | ¹H NMR (400 MHz, DMSO-*d*₆) δ 12.36 (s, 1H), 10.15 (s, 1H), 8.41 (s, 1H), 7.94-7.84 (m, 2H), 7.74-7.67 (m, 2H), 7.60 (s, 1H), 7.56 (t, *J* = 2.0 Hz, 1H), 7.37-7.30 (m, 2H), 7.23 (dd, *J* = 7.6, 1.8 Hz, 1H), 7.03 (d, *J* = 8.4 Hz, 1H), 6.80 (t, *J* = 7.3 Hz, 1H), 3.19 (t, *J* = 5.8 Hz, 4H), 1.61 (s, 4H), 1.43 (q, *J* = 3.1 Hz, 4H). | 455.53 10 |
| **141** | 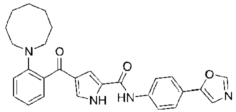 | ¹H NMR (400 MHz, DMSO-(*d*₆) δ 12.38 (s, 1H), 10.19 (s, 1H), 8.41 (s, 1H), 7.96-7.84 (m, 2H), 7.75-7.67 (m, 2H), 7.58 (d, *J* = 18.5 Hz, 2H), 7.39-7.25 (m, 2H), 7.18 (dd, *J* = 7.6, 1.7 Hz, 1H), 7.07 (d, *J* = 8.4 Hz, 1H), 6.81 (t, *J* = 7.3 Hz, 1H), 3.22 (t, *J =* 5.8 Hz, 4H), 1.57 (s, 4H), 1.49-1.34 (m, 6H). | 469.56 04 |
| **142** | 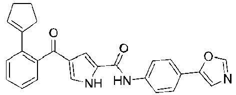 | ¹H NMR (400 MHz, DMSO-*d*₆) δ 12.34 (d, *J* = 9.6 Hz, 1H), 10.84 (s, 1H), 8.31 (dd, *J =* 9.5, 1.5 Hz, 1H), 8.25 (s, 1H), 7.87 - 7.83 (m, 1H), 7.83 - 7.77 (m, 2H), 7.77 - 7.70 (m, 2H), 7.61 (dd, *J =* 7.4, 1.6 Hz, 1H), 7.54 - 7.46 (m, 2H), 7.43 (td, *J* = 7.4, 1.5 Hz, 1H), 7.15 (d, *J* = 1.4 Hz, 1H), 6.01 - 5.94 (m, 1H), 3.02 - 2.94 (m, 2H), 2.83 - 2.74 (m, 1H), 2.29 - 2.20 (m, 1H), 1.70 (p, *J* = 7.1 Hz, 2H). | 424.47 55 |
| **143** | 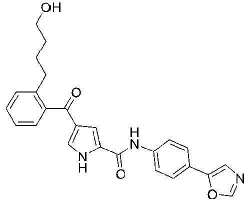 | ¹H NMR (400 MHz, DMSO-*d*₆) δ 12.47 (s, 1H), 10.20 (s, 1H), 8.41 (s, 1H), 7.91-7.85 (m, 2H), 7.74-7.68 (m, 2H), 7.61 (s, 1H), 7.53 (t, *J* = 2.1 Hz, 1H), 7.44 (td, *J* = 7.4, 1.6 Hz, 1H), 7.40-7.23 (m, 4H), 4.31 (t, *J* = 5.2 Hz, 1H), 3.65-3.54 (m, 1H), 3.34-3.27 (m, 2H), 2.64 (t, *J* = 7.7 Hz, 2H), 1.80-1.71 (m, 1H). 1.55-1.46 (m, 2H), 1.40-1.32 (m, 2H). | 430.48 11 |
| **144** | 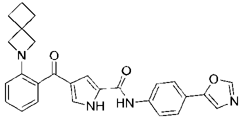 | ¹H NMR (400 MHz, DMSO-*d*₆) δ 12.41 (s, 1H), 10.16 (d, *J* = 3.9 Hz, 1H), 8.42 (s, 1H), 7.89 (d, *J* = 8.8 Hz, 2H), 7.72 (dd, *J* = 8.9, 2.3 Hz, 2H), 7.61 (s, 1H), 7.56 (t, *J* = 2.1 Hz, 1H), 7.40-7.25 (m, 3H), 6.74 (q, *J* = 8.7, 8.1 Hz, 1H), 6.53 (d, *J =* 8.2 Hz, 1H), 3.64 (s, 3H), 2.08 (t, *J* = 7.6 Hz, 4H), 1.74 (p, *J* = 7.6 Hz, 2H). | 453.51 11 |
| **145** | 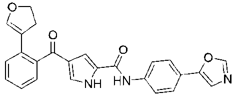 | ¹H NMR (400 MHz, DMSO-*d*₆) δ 12.50 (s, 1H), 10.20 (s, 1H), 8.41 (s, 1H), 7.91-7.84 (m, 2H), 7.76-7.68 (m, 2H), 7.61 (s, 1H), 7.55-7.44 (m, 2H), 7.39-7.25 (m, 4H), 5.94 (dq, *J* = 4.7, 2.2 Hz, 1H), 5.71 (dq, *J* = 6.0, 2.0 Hz, 1H), 4.09-3.89 (m, 1H), 2.49-2.33 (m, 2H). | 426.44 19 |
| **146** | 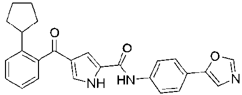 | ¹H NMR (400 MHz, DMSO-*d*₆) δ 12.47 (s, 1H), 10.19 (s, 1H), 8.41 (s, 1H), 7.91-7.84 (m, 2H), 7.75-7.67 (m, 2H), 7.61 (s, 1H), 7.51 (d, *J* = 2.1 Hz, 1H), 7.47 (t, *J* = 3.1 Hz, 2H), 7.33-7.28 (m, 2H), 7.26 (dd, *J* = 3.3, 1.6 Hz, 1H), 1.88 (s, 2H), 1.73 (d, *J =* 12.2 Hz, 2H), 1.61-1.46 (m, 4H). | 426.46 10 |
| **147** | 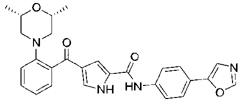 | ¹H NMR (400 MHz, DMSO-*d*₆) δ 12.32 (s, 1H), 10.13 (s, 1H), 8.41 (s, 1H), 7.90-7.85 (m, 2H), 7.74-7.67 (m, 2H), 7.60 (s, 1H), 7.55 (dd, *J* = 2.6, 1.5 Hz, 1H), 7.45 (ddd, *J* = 8.4, 7.3, 1.7 Hz, 1H), 7.34-7.26 (m, 2H), 7.13-7.04 (m, 2H), 3.21 (d, *J* = 8.5 Hz, 2H), 3.08 (d, *J* = 11.4 Hz, 2H), 2.43-2.32 (m, 2H), 0.98 (d, *J* = 6.2 Hz, 6H). | 471.53 32 |
| **148** | 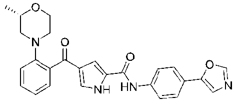 | ¹H NMR (400 MHz, DMSO-*d*₆) δ 10.16 (s, 1H), 8.41 (s, 1H), 7.92-7.85 (m, 2H), 7.75-7.68 (m, 2H), 7.60 (s, 1H), 7.56 (t, *J* = 2.0 Hz, 1H), 7.45 (ddd, *J* = 8.5, 7.4, 1.7 Hz, 1H), 7.37-7.28 (m, 2H), 7.15-7.04 (m, 2H), 3.23-3.06 (m, 3H), 3.00 (dd, *J* = 11.8, 2.2 Hz, 1H), 2.75 (td, *J* = 11.6, 3.0 Hz, 1H), 2.49-2.40 (m, 1H), 0.99 (d, *J* = 6.2 Hz, 3H). | 456.50 55 |
| **149** | 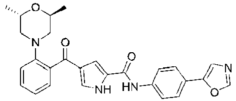 | ¹H NMR (400 MHz, DMSO-*d*₆) δ 12.36 (s, 1H), 10.14 (s, 1H), 8.41 (s, 1H), 7.91-7.85 (m, 2H), 7.74-7.66 (m, 2H), 7.60 (s, 1H), 7.53 (t, *J* = 2.0 Hz, 1H), 7.46 (td, *J* = 7.9, 7.3, 1.7 Hz, 1H), 7.30-7.24 (m, 2H), 7.18-7.08 (m, 2H), 3.78 (td, *J* = 6.2, 3.2 Hz, 2H), 2.94 (dd, *J* = 11.5, 3.1 Hz, 2H), 2.64 (dd, *J* = 11.5, 5.8 Hz, 2H), 0.96 (dd, *J* = 6.6, 2.3 Hz, 6H). | 471.53 28 |
| **150** | 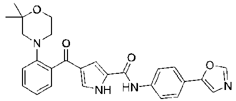 | ¹H NMR (400 MHz, DMSO-*d*₆) δ 12.36 (s, 1H), 10.15 (s, 1H), 7.87 (d, *J* = 8.7 Hz, 2H), 7.71 (d, *J* = 8.7 Hz, 2H), 7.60 (s, 1H), 7.52 (s, 1H), 7.49-7.42 (m, 1H), 7.31-7.23 (m, 2H), 7.20-7.07 (m, 2H), 3.47 (t, *J* = 4.8 Hz, 2H), 2.82 (t, *J* = 4.8 Hz, 2H), 2.73 (s, 2H), 1.00 (s, 6H). | 471.53 33 |
| **151** | 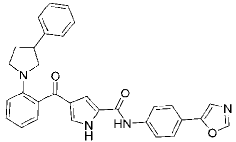 | ¹H NMR (400 MHz, DMSO-*d*₆) δ 12.40 (s, 1H), 10.15 (s, 1H), 8.41 (s, 1H), 7.88 (d, *J* = 8.5 Hz, 2H), 7.71 (d, *J* = 8.4 Hz, 2H), 7.63-7.53 (m, 2H), 7.42-7.17 (m, 8H), 6.87 (d, *J* = 8.3 Hz, 1H), 6.75 (t, *J =* 7.4 Hz, 1H), 3.35-3.29 (m, 0H), 3.22 (q, *J* = 7.2 Hz, 2H), 2.24 (s, 1H), 1.97 (p, *J* = 8.9 Hz, 1H), 1.24 (d, *J* = 11.7 Hz, 1H). | 503.57 61 |
| **152** | 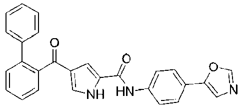 | ¹H NMR (400 MHz, DMSO-*d*₆) δ 12.35 (s, 1H), 10.13 (s, 1H), 8.41 (s, 1H), 7.88-7.82 (m, 2H), 7.73-7.68 (m, 2H), 7.65-7.59 (m, 2H), 7.53-7.49 (m, 3H), 7.44 (t, *J =* 2.0 Hz, 1H), 7.32 (d, *J =* 3.9 Hz, 4H), 7.27 (dd, *J* = 5.1, 3.6 Hz, 1H), 7.19 (dd, *J* = 3.3, 1.5 Hz, 1H). | 434.47 04 |
| **153** | 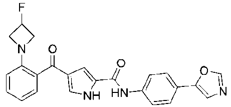 | ¹H NMR (400 MHz, DMSO-*d*₆) δ 11.86 (s, 1H), 10.85 (s, 1H), 8.32-8.23 (m, 4H), 7.83-7.76 (m, 4H), 7.76-7.70 (m, 4H), 7.61 (dd, *J* = 5.6, 3.4 Hz, 2H), 7.50 (s, 2H), 7.37 (dd, *J* = 5.6, 3.4 Hz, 4H), 7.13-7.05 (m, 4H), 4.88-4.77 (m, 1H), 3.91-3.80 (m, 4H). | 431.44 10 |

### Pharmacological Activity Evaluation of Compounds

### 1. In Vitro Enzyme-Level Experiments of 1H-Pyrrole-2-Amide Series Derivatives

The *in vitro* enzyme-level test uses a fluorescence polarization-based method. The experimental method is briefly described as follows: First, 30 nM of GST-YTHDC1(345-509) protein and a series of concentration gradients of the small molecules to be tested are co-incubated for 30 min, and then
a FAM fluorescently labeled m⁶A-modified single-stranded oligonucleotide (5'-AAGAACCGGACUAAGCU-3') (from 5' to 3', the 1st base A is FAM fluorescently labeled, and the 6-position of the 10th base A is methyl-modified) is added and incubated at 18°C for 4 hours. Finally, the fluorescence polarization value of each well is read on a microplate reader with the inhibition rate calculated, and then its IC₅₀ is calculated using Graphpad 8.0.

**Table 2 Inhibitory Activity of the Compounds of the Present Invention against YTHDC1**

| Compound | IC₅₀(µM) | Compound | IC₅₀(µM) | Compound | IC₅₀(µM) |
|---|---|---|---|---|---|
| **1** | 5.50 | **14** | 0.248 | **27** | 0.023 |
| **2** | 0.800 | **15** | 0.105 | **28** | 0.010 |
| **3** | >50 | **16** | 0.041 | **29** | 0.007 |
| **4** | >50 | **17** | 0.042 | **30** | 0.530 |
| **5** | 1.78 | **18** | 0.039 | **31** | 0.015 |
| **6** | >50 | **19** | 0.031 | **32** | 0.011 |
| **7** | >50 | **20** | 0.023 | **33** | 0.012 |
| **8** | 2.84 | **21** | 0.071 | **34** | 0.022 |
| **9** | 10.6 | **22** | 0.023 | **35** | 0.424 |
| **10** | 2.01 | **23** | 0.047 | **36** | 0.013 |
| **11** | 1.57 | **24** | 0.012 | **37** | 0.335 |
| **12** | 0.120 | **25** | 0.036 | **38** | 0.017 |
| **13** | 0.612 | **26** | 0.064 | **39** | >50 |
| **40** | >50 | **73** | 6.27 | **106** | 1.77 |
| **41** | 0.192 | **74** | >50 | **107** | 0.022 |
| **42** | 0.180 | **75** | 0.0085 | **108** | 0.183 |
| **43** | 3.9 | **76** | 5.04 | **109** | 0.044 |
| **44** | 0.138 | **77** | 0.067 | **110** | 0.413 |
| **45** | >50 | **78** | 0.123 | **111** | 0.367 |
| **46** | >50 | **79** | 0.088 | **112** | 50 |
| **47** | >50 | **80** | 0.344 | **113** | 0.054 |
| **48** | >50 | **81** | 0.061 | **114** | 0.016 |
| **49** | >50 | **82** | 0.1249 | 115 | 2.638 |
| **50** | 0.067 | **83** | 0.0987 | **116** | 50 |
| **51** | >50 | **84** | >50 | **117** | 50 |
| **52** | 0.66 | **85** | 0.234 | **118** | 0.012 |
| **53** | >50 | **86** | 0.8473 | **119** | 14.2 |
| **54** | >50 | **87** | 2.317 | **120** | 50 |
| **55** | 0.278 | **88** | 0.0712 | **121** | 0.025 |
| **56** | 0.928 | **89** | >50 | **122** | 0.083 |
| **57** | >50 | **90** | 0.0037 | **123** | 0.118 |
| **58** | 0.08 | **91** | 0.9371 | **124** | 50 |
| **59** | >50 | **92** | 0.4088 | **125** | 0.013 |
| **60** | >50 | **93** | 0.00892 | **126** | 0.013 |
| **61** | 2.509 | **94** | 0.113 | **127** | 0.013 |
| **62** | 0.28 | **95** | 0.03902 | **128** | 0.023 |
| **63** | 0.394 | **96** | 0.0145 | **129** | 31.17 |
| **64** | 1.132 | **97** | 0.0509 | **130** | 50 |
| **65** | 11.24 | **98** | 0.015 | **131** | 0.035 |
| **66** | >50 | **99** | 0.258 | **132** | 0.014 |
| **67** | >50 | **100** | 0.162 | **133** | 0.747 |
| **68** | 1.67 | **101** | 0.043 | **134** | 0.099 |
| **69** | 0.373 | **102** | 0.086 | **135** | 2.823 |
| **70** | 2.28 | **103** | 0.014 | **136** | 0.028 |
| **71** | >50 | **104** | 0.009 | **137** | 0.011 |
| **72** | 36.47 | **105** | 0.0097 | **138** | 0.020 |
| **139** | 1.045 | **144** | 0.048 | **149** | 0.041 |
| **140** | 0.134 | **145** | 0.039 | **150** | 0.084 |
| **141** | 0.189 | **146** | 0.021 | **151** | 1.562 |
| **142** | 0.025 | **147** | 0.19 | **152** | 0.014 |
| **143** | 0.009 | **148** | 0.066 | **153** | 0.014 |

### 2. In Vitro Cell Experiments

### 2.1 Experimental Materials

Main instruments: biosafety cabinet, CO₂ cell culture incubator, flow cytometer, fluorescence inverted microscope, multifunctional microplate reader, precision electronic balance. Main consumables: cell culture dishes, 12-well plates, 96-well plates, 384-well plates. Main reagents: 1640 complete culture medium, fetal bovine serum (Hyclone, South America), penicillin-streptomycin solution.

Cell cycle and apoptosis kits, PE-CD11b and APC-CD14 flow cytometry antibodies, MTT powder, SDS powder. Cell lines: acute myeloid leukaemia cells (MV4-H, HL-60, MOLM-13, THP-1, KG-1, U937, OCI-AML3).

### 2.2 Experimental Methods

(1) Detection of cell proliferation by MTT assay:
   AML cells (MV4-11, HL-60, MOLM-13, THP-1, KG-1, U937, and OCI-AML3) were seeded in 96-well plates at 10,000-15,000 cells per well, and then a series of concentration gradients of **YL-5092** (i.e., Compound **29**) were added for culture for 72 h. 20 µL of MTT solution (5 mg/L) was added to each well and incubated for 2-4 hours, followed by the addition of 50 µL of 20% SDS solution and incubation overnight to dissolve the formazan. The absorbance value was then read at 570 nm on a microplate reader, and the IC₅₀ value of YL-5092 was calculated using GraphPad Prism 8.0. The results are shown in Figure 1a.
(2) Detection of cell cycle, apoptosis, and differentiation by flow cytometry:
   To detect the effect of the small molecule **YL-5092** on the cell cycle of leukaemia cell MOLM-13, MOLM-13 cells were evenly seeded in 12-well plates and treated with different concentration gradients of **YL-5092** (0.5-2 µM) for 48 h. After collection, the cells were washed once with ice-cold PBS and fixed with 70% ice-cold ethanol for 12 h. Before analysis on the flow cytometer, the cells were washed twice with ice-cold PBS and then incubated with PI reagent for 30 min, protected from light. The results are shown in Figure 1b.
   To detect the effect of the small molecule **YL-5092** on the differentiation of leukaemia cell MOLM-13, MOLM-13 cells were evenly seeded in 12-well plates and treated them with different concentration gradients of **YL-5092** (0.5-2 µM) for 5 days. After collection, the cells were washed twice with ice-cold PBS, and 2 µL of PE-CD1 lb antibody and 2 µL of APC-CD14 antibody were added for incubation for 30 min, protected from light, then washed twice with ice-cold PBS, and finally analyzed on the flow cytometer. The results are shown in Figure 1c.
   To detect the effect of the small molecule **YL-5092** on the apoptosis of leukaemia cells MOLM-13, MV4-11, THP-1, and U937, MOLM-13, MV4-11, THP-1, and U937 cells were evenly seeded in 12-well plates and treated them with different concentration gradients of **YL-5092** (0.5-2 µM) for 5 days. After collection, the cells were washed twice with ice-cold PBS, and 5 µL of APC-Annexin V antibody and 5 µL of 7-AAD were added for incubation for 15-30 min, protected from light, and finally analyzed on the flow cytometer. The results are shown in Figure 1d.

### 2.3 Experimental Results

**YL-5092** can significantly inhibit the proliferation of AML cells (MV4-11, HL-60, MOLM-13, THP-1, KG-1, U937, OCI-AML3), with IC₅₀ values as low as 0.28-2.87 µM (Figure 1a). Analysis by flow cytometry found that **YL-5092** can significantly induce AML cell cycle arrest in the G0/G1 phase (Figure 1b), and induce AML cell differentiation (Figure 1c) and apoptosis (Figure 1d).

## Claims

1. A compound represented by Formula I, or a hydrate thereof, or a pharmaceutically acceptable salt thereof, **characterized in that** its structure is as shown below: 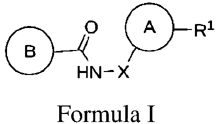
wherein, ring A is selected from a 6- to 10-membered aromatic ring and a 5- to 10-membered heteroaromatic ring;
in ring A, the 5- to 10-membered heteroaromatic ring contains 1 to 3 heteroatoms, wherein the heteroatoms are selected from N, S, and O;
R¹ is selected from H, halogen, cyano, halogen-substituted or unsubstituted C1-C8 alkyl, halogen-substituted or unsubstituted C1-C8 alkoxy, halogen-substituted or unsubstituted C2-C8 alkanoyl, halogen-substituted or unsubstituted C2-C8 alkoxycarbonyl, halogen-substituted or unsubstituted 3- to 8-membered cycloalkyl, substituted or unsubstituted 6- to 10-membered aryl, and substituted or unsubstituted 5- to 10-membered heteroaryl;
in R¹, the 5- to 10-membered heteroaryl contains 1 to 3 heteroatoms, wherein the heteroatoms are selected from N, S, and O;
in R¹, the substituents of the substituted or unsubstituted 6- to 10-membered aryl and the substituted or unsubstituted 5- to 10-membered heteroaryl are selected from at least one of cyano, halogen-substituted or unsubstituted C1-C6 alkyl, and -NR¹³R¹⁴;
R¹³ and R¹⁴ are independently selected from H and halogen-substituted or unsubstituted C1-C4 alkyl, or R¹³ and R¹⁴, together with the N atom in -NR¹³R¹⁴, form a halogen-substituted or unsubstituted 3- to 8-membered heterocycloalkyl, wherein the 3- to 8-membered heterocycloalkyl, in addition to the aforementioned N atom, further contains 0 to 2 heteroatoms selected from N, S, and O;
ring B is selected from 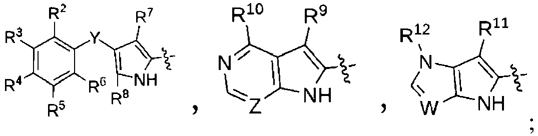
R² to R⁶ are independently selected from H, halogen, halogen-substituted or unsubstituted C1-C8 alkyl, and halogen-substituted or unsubstituted C1-C8 alkoxy;
R⁷ to R⁸ and R¹¹ are independently selected from H, halogen, and halogen-substituted or unsubstituted C1-C6 alkyl;
R¹⁰ and R¹² are independently selected from substituted or unsubstituted 6- to 10-membered aryl and substituted or unsubstituted 5- to 10-membered heteroaryl;
in R¹⁰ and R¹², the 5- to 10-membered heteroaryl contains 1 to 3 heteroatoms, wherein the heteroatoms are selected from N, S, and O;
in R¹⁰ and R¹², the substituents of the substituted 6- to 10-membered aryl and the substituted 5- to 10-membered heteroaryl are selected from cyano, halogen, substituted or unsubstituted C1-C6 alkyl, substituted or unsubstituted C1-C6 alkoxy, -NR¹⁵R¹⁶, and -SO₂R¹⁷;
in R¹⁰ and R¹², the substituents of the substituted C1-C6 alkyl and the substituted C1-C6 alkoxy are selected from hydroxyl and halogen;
R¹⁵, R¹⁶, and R¹⁷ are independently selected from H and halogen-substituted or unsubstituted C1-C4 alkyl;
X is selected from a bond and -CH(R¹⁸)(CH₂)ₙ-; wherein n is an integer from 0 to 3;
R¹⁸ is selected from H and C1-C4 alkyl;
Y is selected from 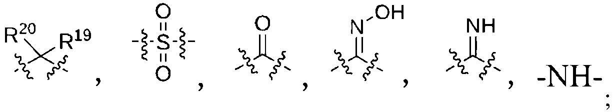
R¹⁹ and R²⁰ are independently selected from H, hydroxyl, cyano, halogen, and halogen-substituted or unsubstituted C1-C4 alkyl;
Z and W are independently selected from N and CR²¹;
R²¹ is selected from H and halogen.

2. The compound according to claim 1, **characterized in that**:
ring A is selected from a 6- to 10-membered aromatic ring and a 5- to 6-membered heteroaromatic ring; in ring A, the 5- to 6-membered heteroaromatic ring contains 1 to 2 heteroatoms, wherein the heteroatoms are selected from N, S, and O;
preferably, ring A is selected from a benzene ring and a pyridine ring;
most preferably, ring A is selected from

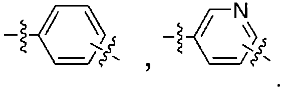

3. The compound according to claim 1, **characterized in that**:
R¹ is selected from H, halogen, cyano, halogen-substituted or unsubstituted C1-C6 alkyl, halogen-substituted or unsubstituted C1-C6 alkoxy, halogen-substituted or unsubstituted C2-C6 alkanoyl, halogen-substituted or unsubstituted C2-C6 alkoxycarbonyl, halogen-substituted or unsubstituted 3- to 6-membered cycloalkyl, substituted or unsubstituted phenyl, and substituted or unsubstituted 5- to 6-membered heteroaryl; in R¹, the 5- to 6-membered heteroaryl contains 1-2 heteroatoms, wherein the heteroatoms are selected from N, S, and O;
preferably, R¹ is selected from H, halogen, cyano, halogen-substituted or unsubstituted C1-C4 alkyl, halogen-substituted or unsubstituted C1-C4 alkoxy, halogen-substituted or unsubstituted C2-C4 alkanoyl, halogen-substituted or unsubstituted C2-C5 alkoxycarbonyl, halogen-substituted or unsubstituted 3- to 6-membered cycloalkyl, substituted or unsubstituted phenyl, and substituted or unsubstituted 5- to 6-membered heteroaryl; in R¹, the 5- to 6-membered heteroaryl contains 1-2 heteroatoms, wherein the heteroatoms are selected from N, S, and O;
further preferably, R¹ is selected from H, F, Cl, Br, cyano, fluorine-substituted or unsubstituted C1-C4 alkyl, fluorine-substituted or unsubstituted C1-C4 alkoxy, fluorine-substituted or unsubstituted C2-C4 alkanoyl, fluorine-substituted or unsubstituted C2-C5 alkoxycarbonyl, fluorine-substituted or unsubstituted 3- to 6-membered cycloalkyl, substituted or unsubstituted phenyl, and substituted or unsubstituted 5- to 6-membered heteroaryl; in R¹, the 5- to 6-membered heteroaryl contains 1-2 heteroatoms, wherein the heteroatoms are selected from N, S, and O;
still further preferably, R¹ is selected from H, F, Cl, Br, cyano, fluorine-substituted or unsubstituted C1-C4 alkyl, fluorine-substituted or unsubstituted C1-C4 alkoxy, fluorine-substituted or unsubstituted C2-C4 alkanoyl, fluorine-substituted or unsubstituted C2-C5 alkoxycarbonyl, fluorine-substituted or unsubstituted 3- to 6-membered cycloalkyl, substituted or unsubstituted phenyl, and substituted or unsubstituted 5- to 6-membered heteroaryl; in R¹, the 5- to 6-membered heteroaryl is selected from pyridyl, thienyl, furyl, pyrrolyl, thiazolyl, isothiazolyl, oxazolyl, isoxazolyl, imidazolyl, and pyrazolyl;
most preferably, R¹ is selected from H, F, Cl, Br, methyl, trifluoromethyl, methoxy, trifluoromethoxy, acetyl, ethoxycarbonyl, substituted or unsubstituted phenyl, and substituted or unsubstituted 5- to 6-membered heteroaryl; in R¹, the 5- to 6-membered heteroaryl is selected from

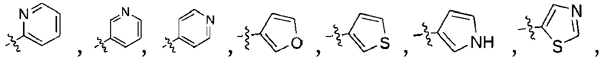

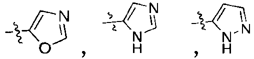

4. The compound according to any one of claims 1-3, **characterized in that**:
in R¹, the substituents of the substituted or unsubstituted aryl and the substituted or unsubstituted heteroaryl are selected from at least one of cyano, halogen-substituted or unsubstituted C1-C4 alkyl, and -NR¹³R¹⁴; R¹³ and R¹⁴ are independently selected from halogen-substituted or unsubstituted C1-C4 alkyl, or R¹³ and R¹⁴, together with the N atom in -NR¹³R¹⁴, form a halogen-substituted or unsubstituted 3- to 6-membered heterocycloalkyl, wherein the 3-to 6-membered heterocycloalkyl, in addition to the aforementioned N atom, further contains 0 to 1 heteroatom selected from N, S, and O;
preferably, in R¹, the substituents of the substituted or unsubstituted aryl and the substituted or unsubstituted heteroaryl are selected from at least one of cyano, fluorine-substituted or unsubstituted C1-C4 alkyl, and -NR¹³R¹⁴; R¹³ and R¹⁴ are independently selected from fluorine-substituted or unsubstituted C1-C4 alkyl, or R¹³ and R¹⁴, together with the N atom in -NR¹³R¹⁴, form a fluorine-substituted or unsubstituted 3- to 6-membered heterocycloalkyl, wherein the 3- to 6-membered heterocycloalkyl, in addition to the aforementioned N atom, further contains 0 to 1 heteroatom selected from S and O;
further preferably, in R¹, the substituents of the substituted or unsubstituted aryl and the substituted or unsubstituted heteroaryl are selected from at least one of cyano, methyl, trifluoromethyl, and -NR¹³R¹⁴; R¹³ and R¹⁴ are independently selected from methyl and trifluoromethyl, or R¹³ and R¹⁴, together with the N atom in -NR¹³R¹⁴, form a fluorine-substituted or unsubstituted 3- to 6-membered heterocycloalkyl, wherein the 3- to 6-membered heterocycloalkyl is selected from 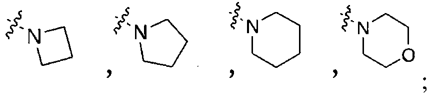
most preferably, in R¹, the substituents of the substituted or unsubstituted aryl and the substituted or unsubstituted heteroaryl are selected from cyano, methyl, trifluoromethyl, and

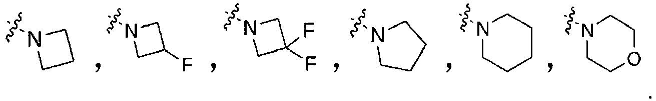

5. The compound according to any one of claims 1-4, **characterized in that**: the structural unit 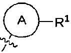 is selected from

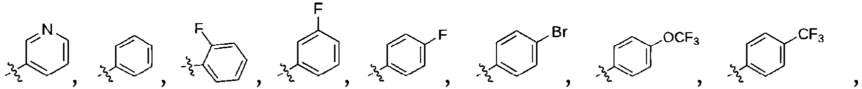

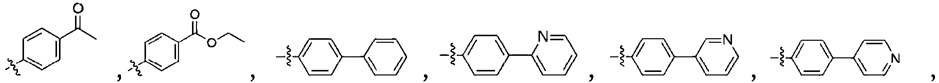

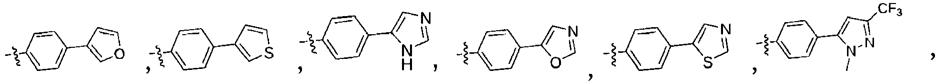

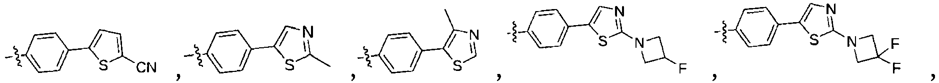

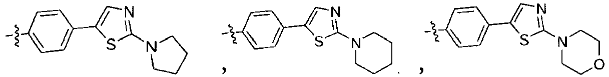

6. The compound according to claim 1, **characterized in that**:
R² to R⁶ are independently selected from H, halogen, halogen-substituted or unsubstituted C1-C6 alkyl, and halogen-substituted or unsubstituted C1-C6 alkoxy;
preferably, R² to R⁶ are independently selected from H, halogen, halogen-substituted or unsubstituted C1-C4 alkyl, and halogen-substituted or unsubstituted C1-C4 alkoxy;
further preferably, R² to R⁶ are independently selected from H, F, Cl, Br, fluorine-substituted or unsubstituted C1-C4 alkyl, and fluorine-substituted or unsubstituted C1-C4 alkoxy;
still further preferably, R² to R⁶ are independently selected from H, F, Cl, Br, fluorine-substituted or unsubstituted C1-C4 alkyl, and fluorine-substituted or unsubstituted C1-C4 alkoxy, and R² and R⁶ are not both H at the same time;
most preferably, R² is selected from H, F, Cl, Br, methyl, trifluoromethyl, ethyl, tert-butyl, methoxy, trifluoromethoxy, and ethoxy, R³ is selected from H, F, Cl, and Br, R⁴ is selected from H, F, Cl, Br, methyl, and trifluoromethyl, R⁵ is selected from H, F, Cl, and Br, R⁶ is selected from H, F, Cl, Br, methyl, trifluoromethyl, ethyl, tert-butyl, methoxy, trifluoromethoxy, and ethoxy, and Rand R⁶ are not both H at the same time.

7. The compound according to claim 1 or 6, **characterized in that**: the structural unit 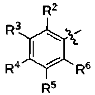 is selected from:

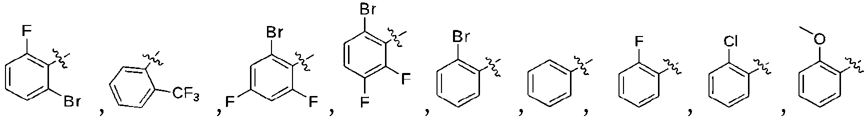

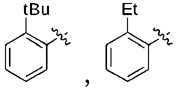

8. The compound according to claim 1, **characterized in that**:
Y is selected from 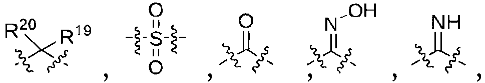
- NH- ; R¹⁹ and R²⁰ are independently selected from H, hydroxyl, cyano, F, and F-substituted or unsubstituted C1-C4 alkyl;
preferably, Y is selected from 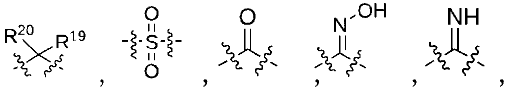 -NH-; R¹⁹ and R²⁰ are independently selected from H, hydroxyl, cyano, F, methyl, and trifluoromethyl;
most preferably, Y is selected from 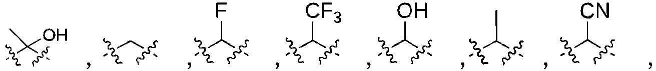 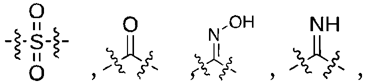 -NH-

9. The compound according to claim 1, **characterized in that**:
R⁷ to R⁹ and R¹¹ are independently selected from H, halogen, and halogen-substituted or unsubstituted C1-C4 alkyl;
preferably, R⁷ to R⁹ and R¹¹ are independently selected from H, F, and F-substituted or unsubstituted C1-C4 alkyl;
most preferably, R⁷ to R⁹ and R¹¹ are independently selected from H, F, methyl, and trifluoromethyl.

10. The compound according to claim 1, **characterized in that**:
R¹⁰ and R¹² are independently selected from substituted or unsubstituted phenyl and substituted or unsubstituted 5- to 6-membered heteroaryl; in R¹⁰ and R¹², the 5- to 6-membered heteroaryl contains 1 to 2 heteroatoms, wherein the heteroatoms are selected from N, S, and O;
preferably, R¹⁰ and R¹² are independently selected from substituted or unsubstituted phenyl and substituted or unsubstituted 5- to 6-membered heteroaryl; in R¹⁰ and R¹², the 5- to 6-membered heteroaryl is selected from pyridyl, thienyl, furyl, pyrrolyl, thiazolyl, isothiazolyl, oxazolyl, isoxazolyl, imidazolyl, and pyrazolyl;
most preferably, R¹⁰ and R¹² are independently selected from substituted or unsubstituted phenyl and substituted or unsubstituted 5- to 6-membered heteroaryl; in R¹⁰ and R¹², the 5- to 6-membered heteroaryl is selected from

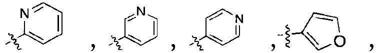

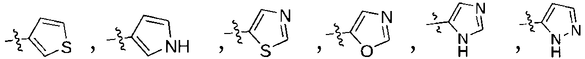

11. The compound according to claim 1 or 10, **characterized in that**:
in R¹⁰ and R¹², the substituents of the substituted aryl and the substituted heteroaryl are selected from cyano, halogen, substituted or unsubstituted C1-C4 alkyl, substituted or unsubstituted C1-C4 alkoxy, -NR¹⁵R¹⁶, and -SO₂R¹⁷; in R¹⁰ and R¹², the substituents of the substituted C1-C4 alkyl and the substituted C1-C4 alkoxy are selected from hydroxyl and halogen; R¹⁵, R¹⁶, and R¹⁷ are independently selected from H and halogen-substituted or unsubstituted C1-C4 alkyl;
preferably, in R¹⁰ and R¹², the substituents of the substituted aryl and the substituted heteroaryl are selected from cyano, F, Cl, Br, substituted or unsubstituted C1-C4 alkyl, substituted or unsubstituted C1-C4 alkoxy, -NR¹⁵R¹⁶,
and -SO₂R ¹⁷; in R¹⁰ and R¹², the substituents of the substituted C1-C4 alkyl and the substituted C1-C4 alkoxy are selected from hydroxyl and F; R¹⁵, R¹⁶, and R¹⁷ are independently selected from H and fluorine-substituted or unsubstituted C1-C4 alkyl;
most preferably, in R¹⁰ and R¹², the substituents of the substituted aryl and the substituted heteroaryl are selected from cyano, F, Cl, Br, methyl, ethyl, n-propyl, isopropyl, hydroxymethyl, trifluoromethyl, methoxy, trifluoromethoxy, amino, -SO₂CF₃, and -SO₂CH₃.

12. The compound according to claim 1, **characterized in that**:
X is selected from a bond and -CH(R¹⁸)(CH₂)ₙ-; n is an integer selected from 0 to 1; R¹⁸ is selected from H and C1-C4 alkyl;
preferably, X is selected from a bond, -CH(CH₃)-, and -CH₂-.

13. The compound according to claim 1, **characterized in that**:
Z and W are independently selected from N and CR²¹; R²¹ is selected from H and F;
preferably, Z is selected from N, CH, and CF, and W is selected from N.

14. The compound according to any one of claims 1-13, **characterized in that** at least one of the following is satisfied: 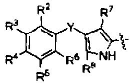 is selected from: 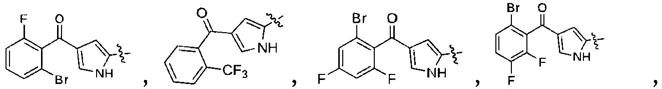 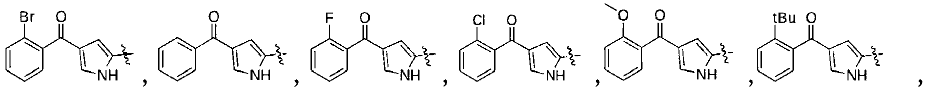 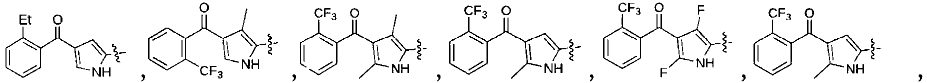 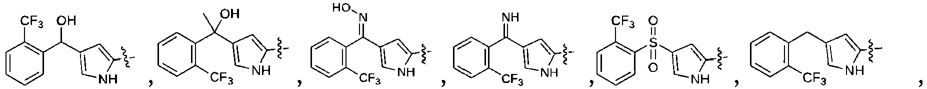 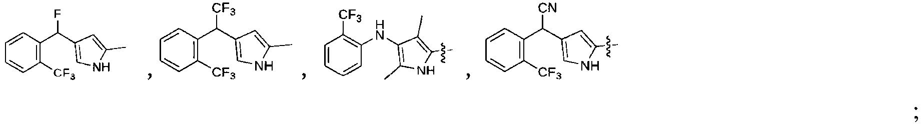 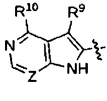 is selected from: 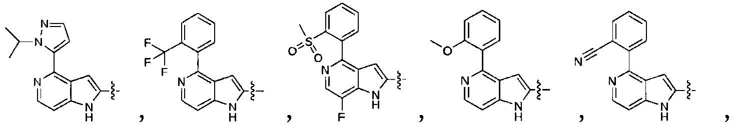 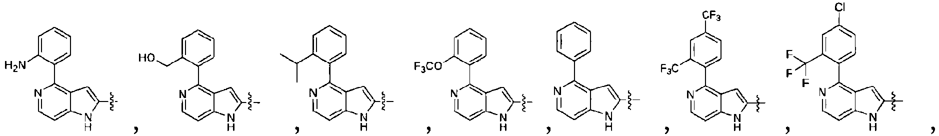 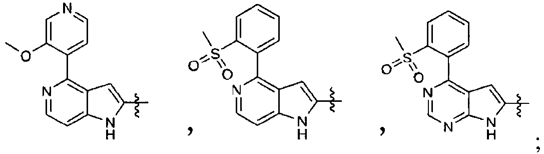 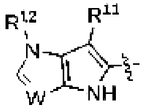 is selected from:

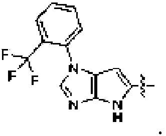

15. The compound according to any one of claims 1-14, **characterized in that** the structural formula is as follows:

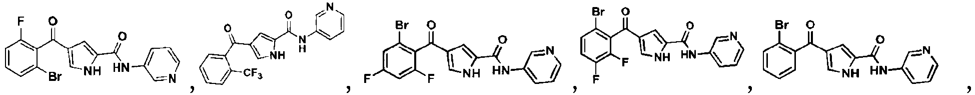

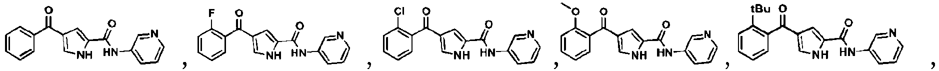

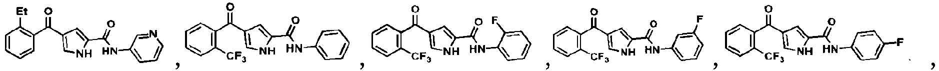

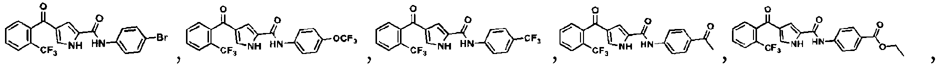

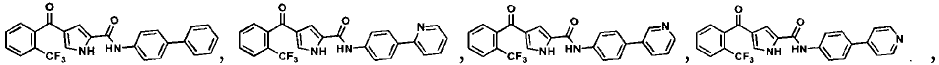

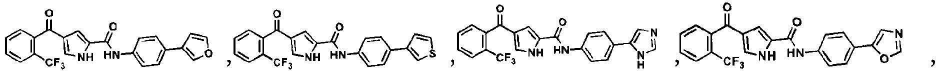

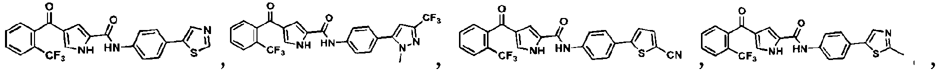

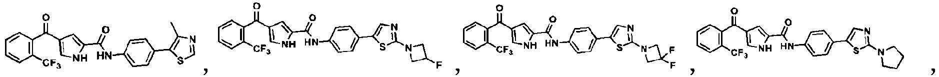

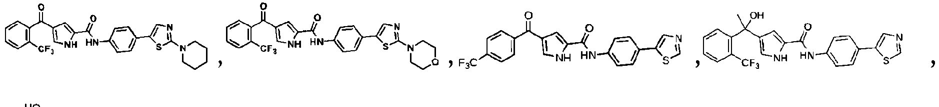

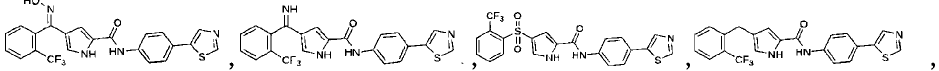

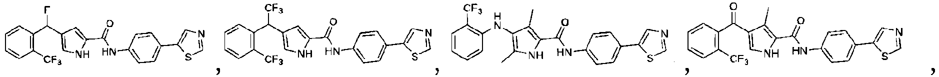

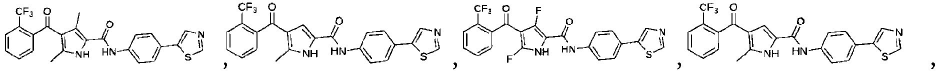

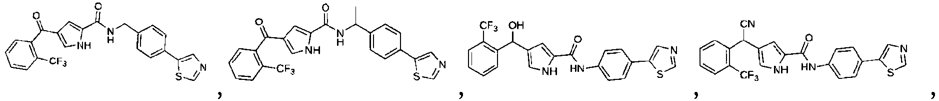

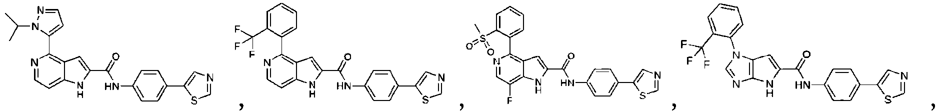

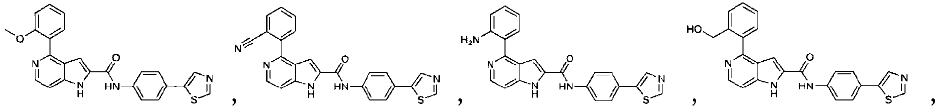

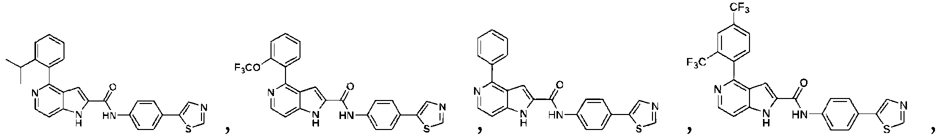

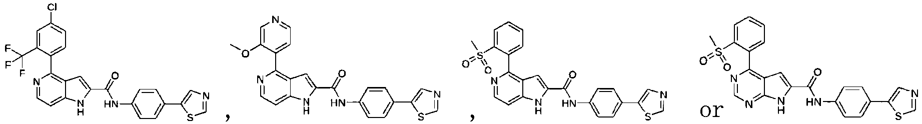

16. The compound according to claim 1 or 2, **characterized in that**: the structural unit 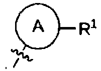 is selected from:

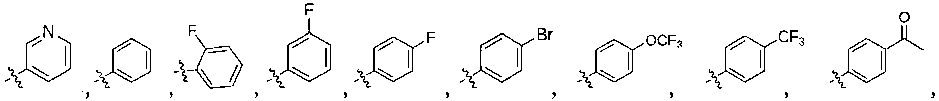

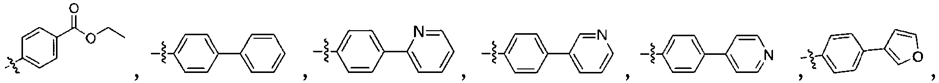

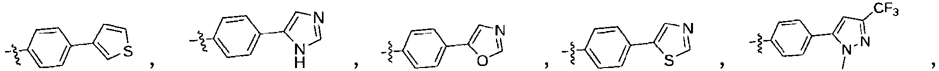

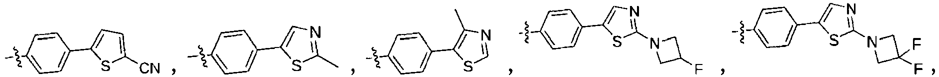

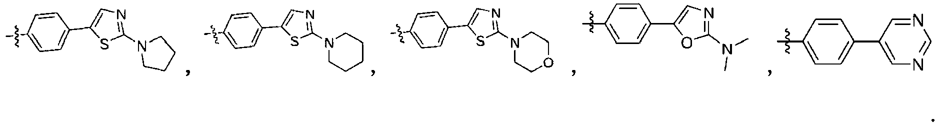

17. The compound according to any one of claims 1, 2, 9, 12-13, or 16, **characterized in that**: 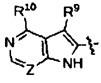 is selected from:

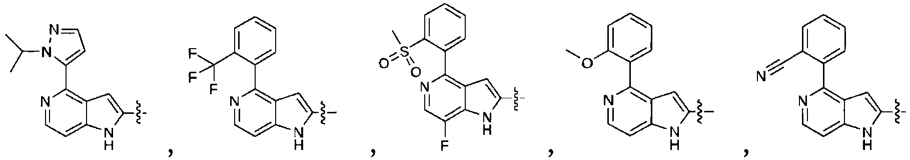

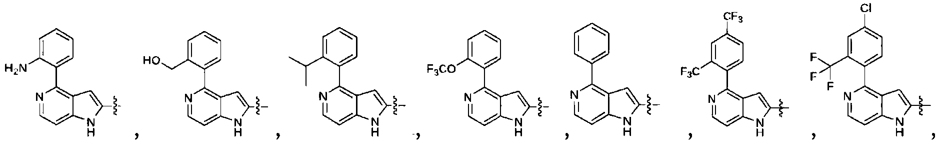

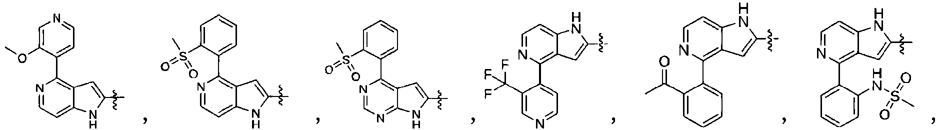

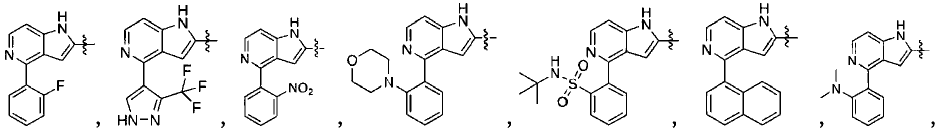

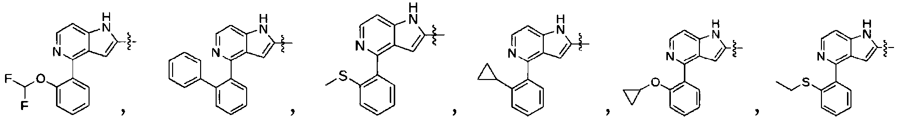

18. The compound according to any one of claims 1, 2, 9, 12-13, or 16-17, **characterized in that** the structural formula is as follows: 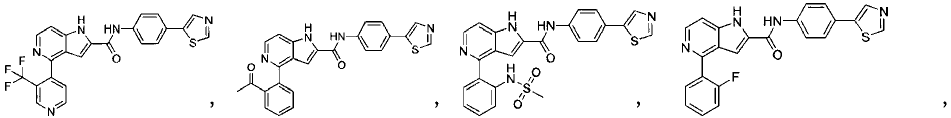 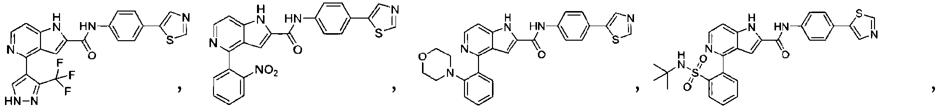 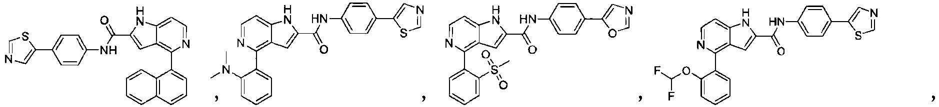 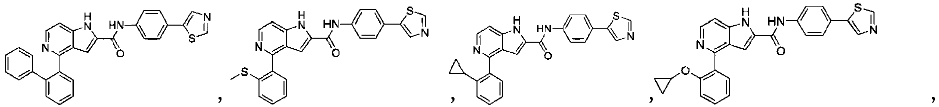 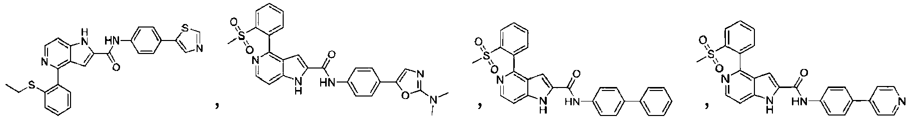 or

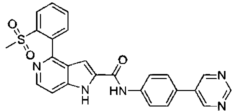

19. The compound according to claim 1, **characterized in that** the structural formula is as follows:

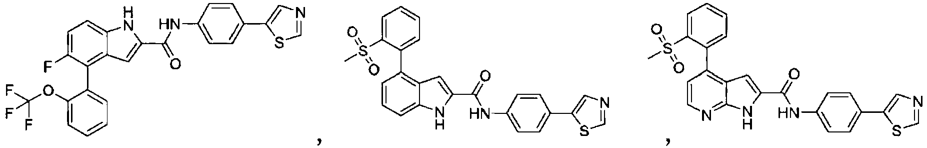

20. The compound according to claim 1 or 2, **characterized in that**: the structural unit 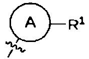 is selected from:

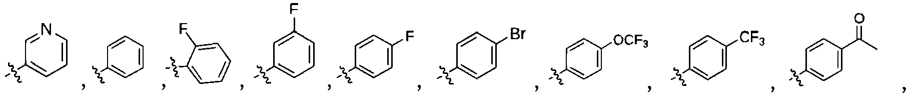

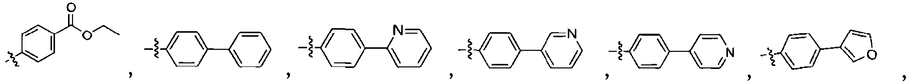

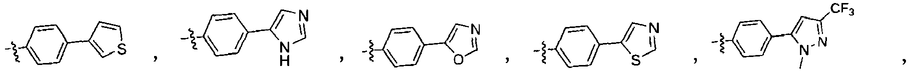

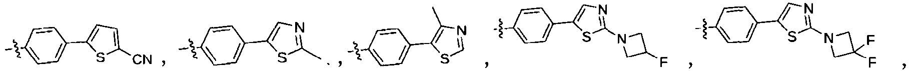

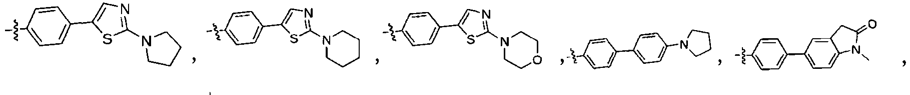

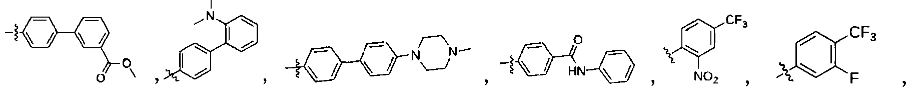

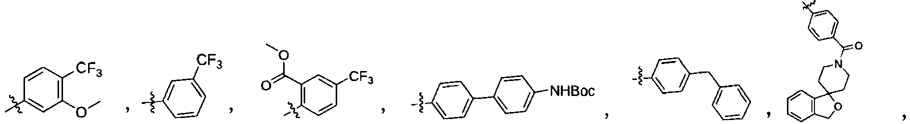

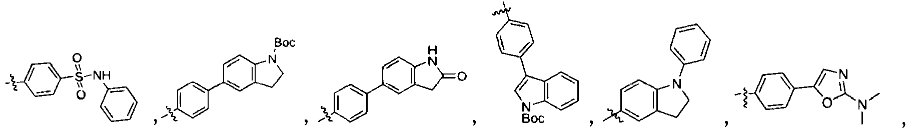

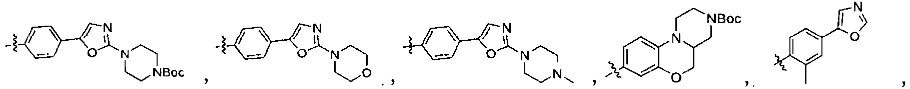

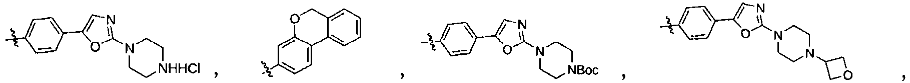

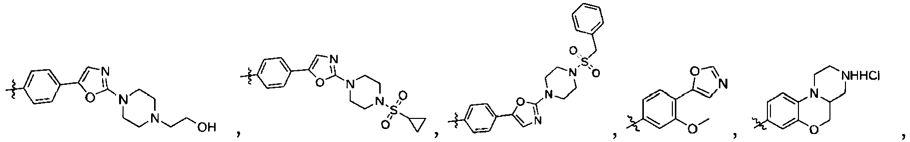

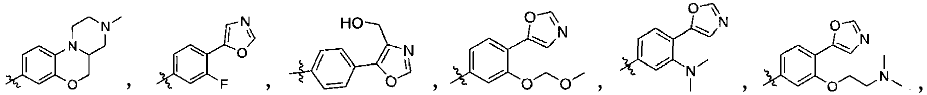

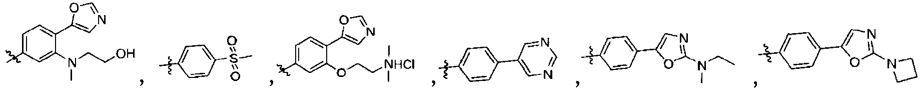

21. The compound according to any one of claims 1, 2, or 20, **characterized in that**: the structural unit 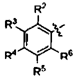 is selected from:

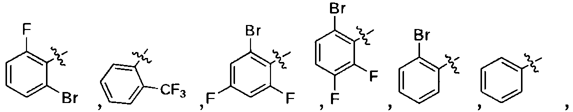

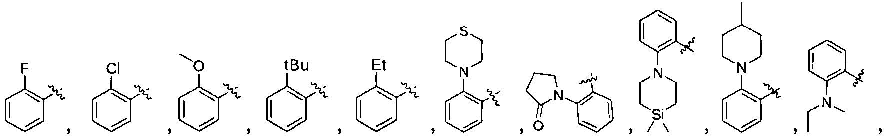

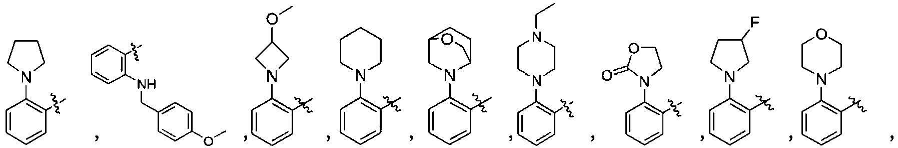

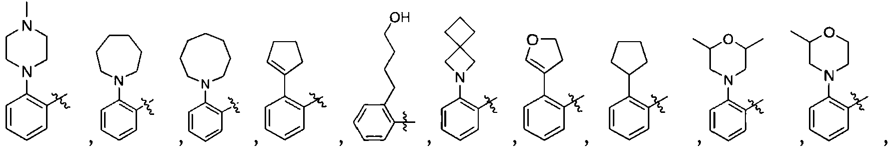

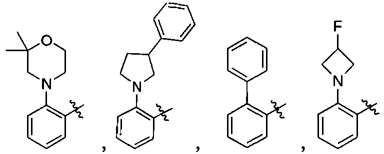

22. The compound according to any one of claims 1, 2, 8, 9, 12, or 20-21, **characterized in that**: 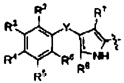 is selected from:

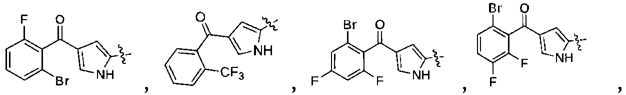

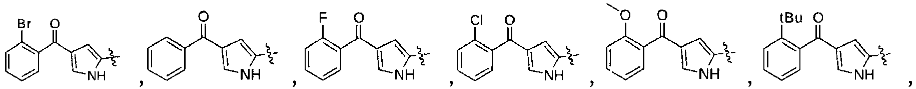

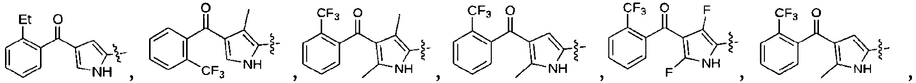

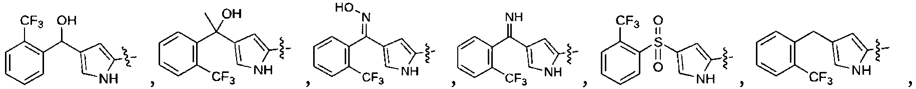

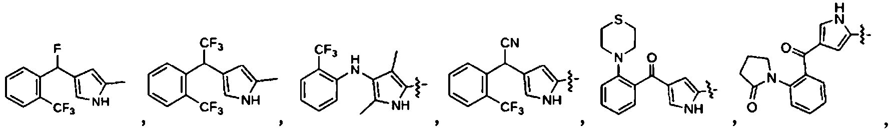

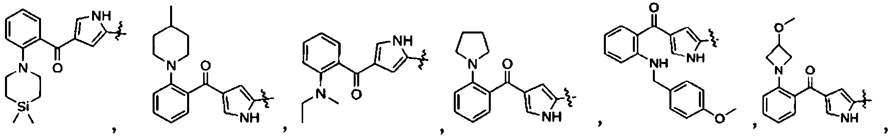

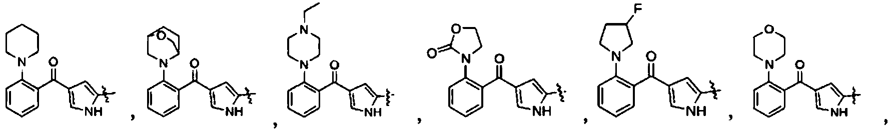

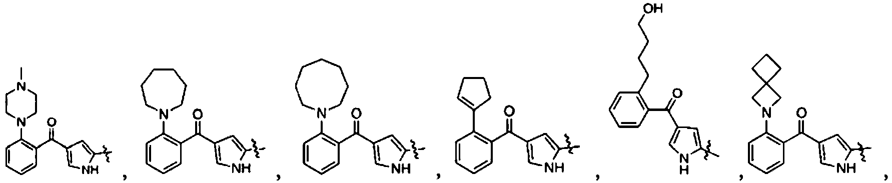

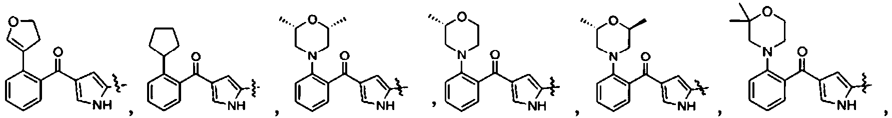

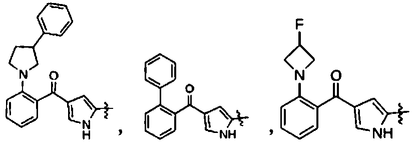

23. The compound according to any one of claims 1, 2, 8, 9, 12, or 20-22, **characterized in that** the structural formula is as follows:

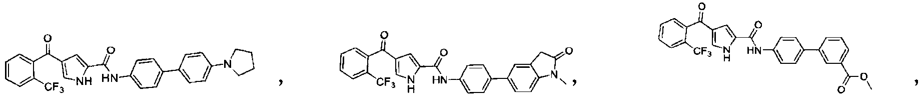

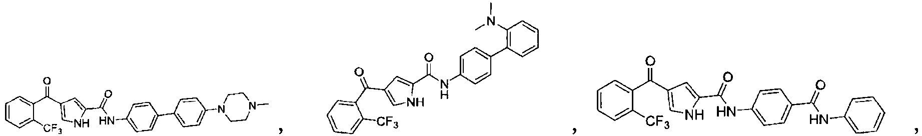

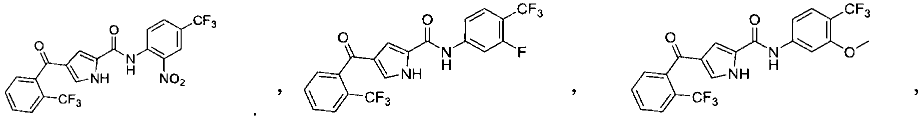

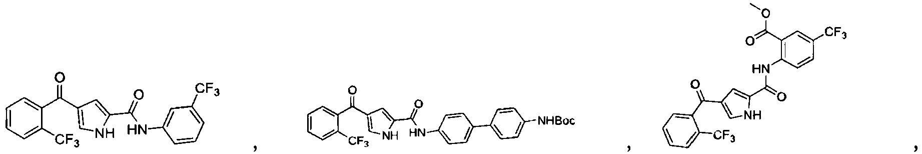

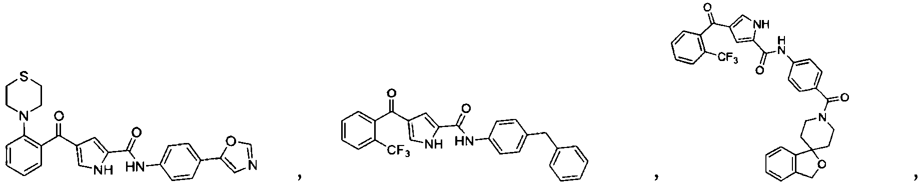

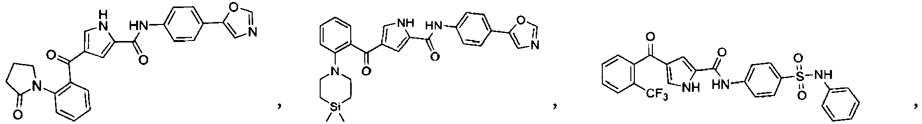

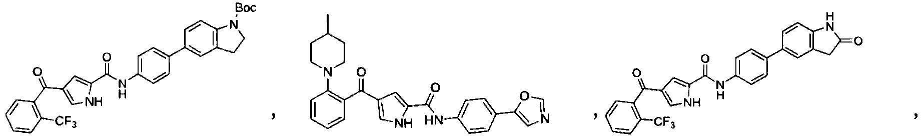

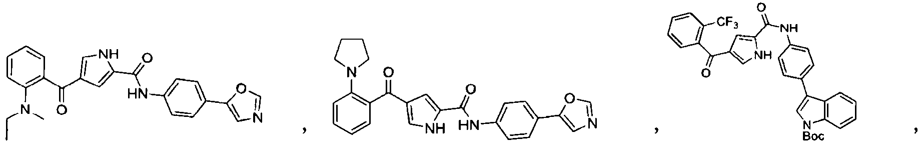

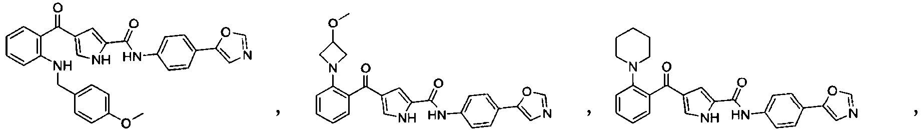

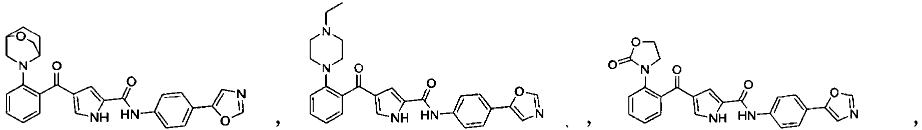

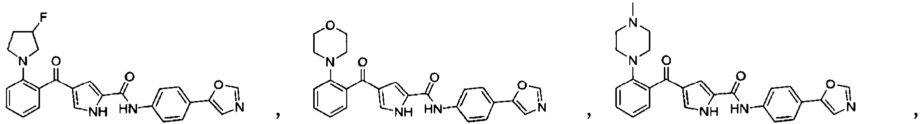

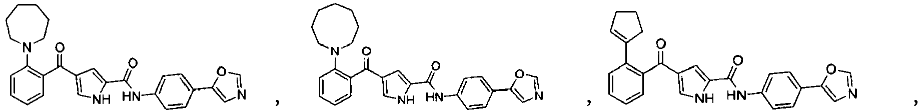

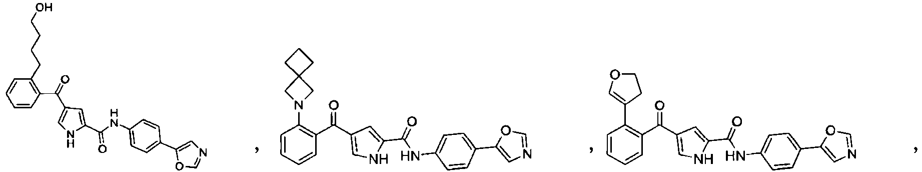

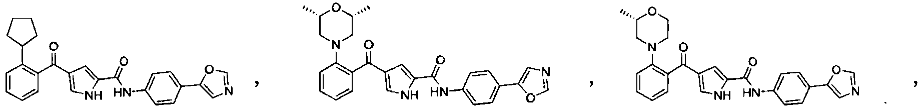

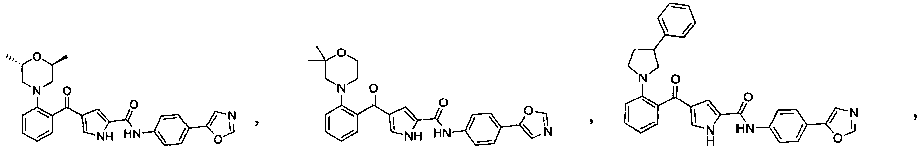

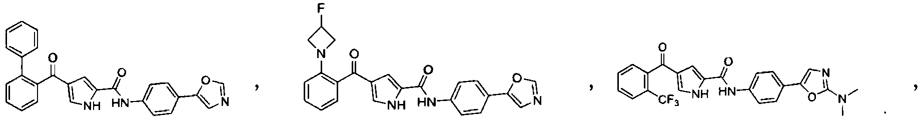

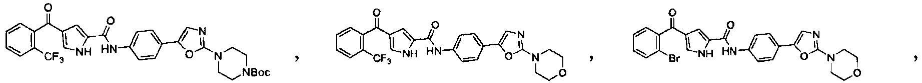

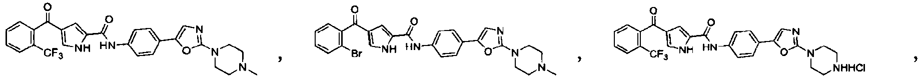

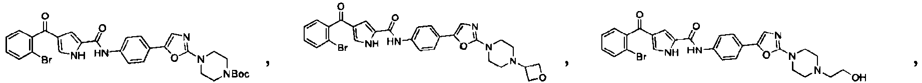

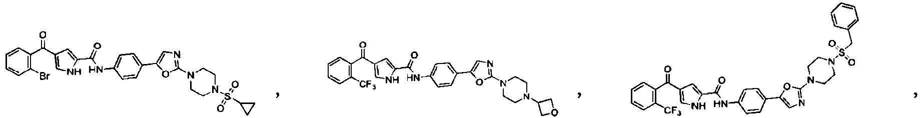

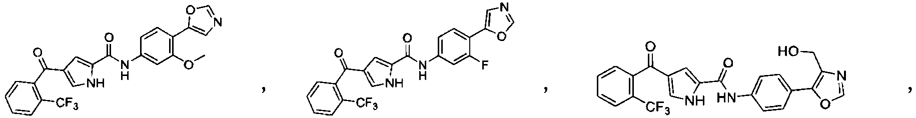

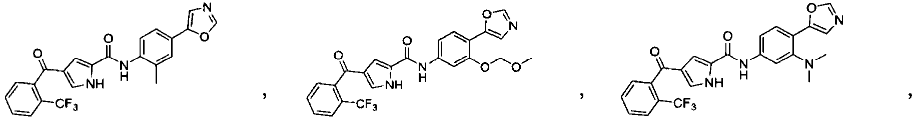

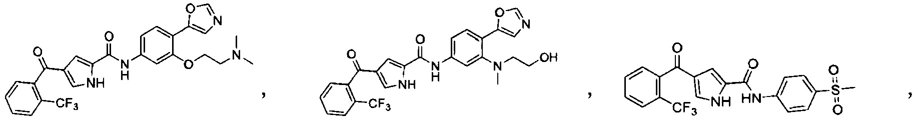

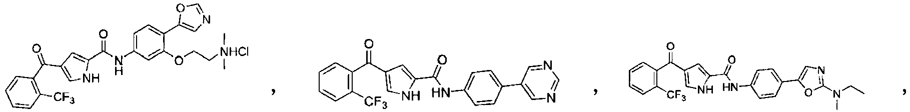

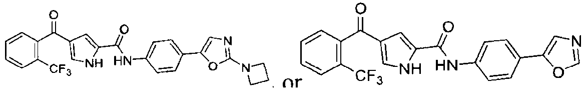

24. The compound according to claim 1, **characterized in that** the structural formula is as follows: 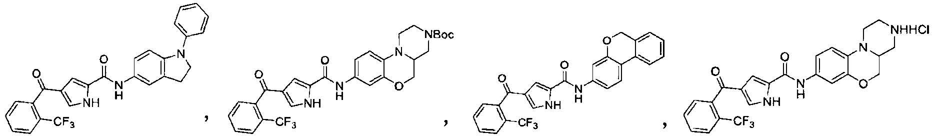 or

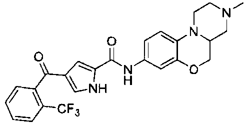

25. A pharmaceutical composition, composed of the compound according to any one of claims 1-24, a hydrate thereof, or a pharmaceutically acceptable salt thereof as an active ingredient, with the addition of pharmaceutically acceptable auxiliary ingredients.

26. Use of the compound according to any one of claims 1-24, a hydrate thereof, a pharmaceutically acceptable salt thereof, or the pharmaceutical composition according to claim 25 in the preparation of a small-molecule inhibitor of YTHDC1.

27. Use of the compound according to any one of claims 1-26, a hydrate thereof, a pharmaceutically acceptable salt thereof, or the pharmaceutical composition according to claim 32 in the preparation of a medicament for treating and/or preventing YTHDC1-related diseases; preferably, the YTHDC1-related diseases include: acute myeloid leukemia, t-cell lymphoma, hepatic cancer, pancreatic carcinoma, breast cancer, glioma, or hepatitis B; more preferably, the YTHDC1-related diseases include acute myeloid leukemia or t-cell lymphoma.
